# EUROPEAN PATENT SPECIFICATION

(11) **EP 1 615 904 B1**
(45) Date of publication and mention of the grant of the patent: **27.02.2008**
(21) Application number: 04750131.7
(22) Date of filing: 15.04.2004
(51) Int. Cl.: C07D 311/16

(54) **SUBSTITUTED BENZOSULPHONAMIDES AS POTENTIATORS OF GLUTAMATE RECEPTORS**
SUBSTITUIRTE BENZOSULFONAMIDE ALS POTENZIERUNGSMITTEL VON GLUTAMATREZEPTOREN
BENZENSULPHONAMIDES SUBSTITUÉES COMME POTENTIATEURS DES RÉCEPTEURS DU GLUTAMATE

(30) Priority: 15.04.2003 US 462697 P
(43) Date of publication of application: 18.01.2006
(73) Proprietor: AstraZeneca AB, 151 85 Södertälje (SE)
(72) Inventor: EGLE, Ian, Toronto, Ontario M5G 1L8 (CA); FREY, Jennifer, Brampton, Ontario L6X 3H2 (CA); ISAAC, Methvin, Toronto, Ontario M5G 1L8 (CA); SLASSI, Abdelmalik, Mississauga, Ontario L5M 7J7 (CA); SUN, Gaung ri, Markham, Ontario L3R 5G8 (CA); FIELD, Johnathan Wesley, Magnetawan, Ontario P0A 1P0 (CA)
(74) Representative: Plougmann & Vingtoft A/S
(86) International application number: PCT/US2004/011544
(87) International publication number: WO 2004/092135

(56) References cited:
- EP-A- 0 860 428
- DATABASE CHEMCATS CHEMICAL ABSTRACTS SERVICE, COLUMBUS, OHIO, US; XP002300638 retrieved from STN & "Interchim intermediates" 2 July 2002 (2002-07-02), INTERCHIM , MONTLUCON, CEDEX 03103 FRANCE

## Description

### FIELD OF THE INVENTION

The present invention relates to novel compounds that function as potentiators of glutamate receptors, methods for their preparation, pharmaceutical compositions containing them and their use in therapy.

### BACKGROUND

The metabotropic glutamate receptors (mGluR) constitute a family of GTP-binding-protein (G-protein) coupled receptors that are activated by glutamate, and have important roles in synaptic activity in the central nervous system, including neural plasticity, neural development and neurodegeneration.

Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase A₂; increases in arachidonic acid release; and increases or decreases in the activity of voltage- and ligand-gated ion channels (Schoepp et al., 1993, Trends Pharmacol. Sci., 14:13 ; Schoepp, 1994, Neurochem. Int., 24:439; Pin et al., 1995, Neuropharmacology 34:1; Bordi & Ugolini, 1999, Prog. Neurobiol. 59:55).

Eight mGluR subtypes have been identified, which are divided into three groups based upon primary sequence similarity, signal transduction linkages, and pharmacological profile. Group-I includes mGluR1 and mGluR5, which activate phospholipase C and the generation of an intracellular calcium signal. The Group-II (mGluR2 and mGluR3) and Group-III (mGluR4, mGluR6, mGluR7, and mGluR8) mGluRs mediate an inhibition of adenylyl cyclase activity and cyclic AMP levels. For a review, *see* Pin et al., 1999, Eur. J. Pharmacol., 375:277-294.

Members of the mGluR family of receptors are implicated in a number of normal processes in the mammalian CNS, and are important targets for compounds for the treatment of a variety of neurological and psychiatric disorders. Activation of mGluRs is required for induction of hippocampal long-term potentiation and cerebellar long-term depression (Bashir et al., 1993, Nature, 363:347 ; Bortolotto et al., 1994, Nature, 368:740 ; Aiba et al., 1994, Cell, 79:365 ; Aiba et al., 1994, Cell, 79:377). A role for mGluR activation in nociception and analgesia also has been demonstrated (Meller et al., 1993, Neuroreport, 4: 879; Bordi & Ugolini, 1999, Brain Res., 871:223). In addition, mGluR activation has been suggested to play a modulatory role in a variety of other normal processes including synaptic transmission, neuronal development, apoptotic neuronal death, synaptic plasticity, spatial learning, olfactory memory, central control of cardiac activity, waking, motor control and control of the vestibulo-ocular reflex (Nakanishi, 1994, Neuron, 13:1031; Pin et al., 1995, Neuropharmacology, *supra;* Knopfel et al., 1995, J. Med. Chem., 38:1417).

Recent advances in the elucidation of the neurophysiological roles of mGluRs have established these receptors as promising drug targets in the therapy of acute and chronic neurological and psychiatric disorders and chronic and acute pain disorders. Because of the physiological and pathophysiological significance of the mGluRs, there is a need for new drugs and compounds that can modulate mGluR function.

### DETAILED DESCRIPTION

The present invention is based upon the discovery of compounds that act as allosteric modulators of mGluR2.

### Definitions

Unless specified otherwise within this specification, the nomenclature used in this specification generally follows the examples and rules stated in Nomenclature of Organic Chemistry, Sections A, B, C, D, E, F, and H, Pergamon Press, Oxford, 1979, referred herein for its exemplary chemical structure names and rules on naming chemical structures. Optionally, a name of a compound may be generated using a chemical naming program: ACD/ChemSketch, Version 5.09/September 2001, Advanced Chemistry Development, Inc., Toronto, Canada.

The term "Cₘ₋ₙ" or "Cₘ₋ₙ group" used alone or as a prefix, refers to any group having m to n carbon atoms, and having 0 to n multivalent heteroatoms selected from O, S and N, wherein m and n are 0 or positive integers, and n>m. For example, "C₁₋₆" would refer to a chemical group having 1 to 6 carbon atoms, and having 0 to 6 multivalent heteroatoms selected from O, S and N.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. Unless otherwise specified, "alkyl" general includes both saturated alkyl and unsaturated alkyl.

The term "alkylene" used alone or as suffix or prefix, refers to divalent straight or branched chain hydrocarbon radicals comprising 1 to about 12 carbon atoms, which serves to links two structures together.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms.

The term "cycloalkenyl" used alone or as suffix or prefix, refers to a monovalent ring-containing hydrocarbon radical having at least one carbon-carbon double bond and comprising at least 3 up to about 12 carbon atoms.

The term "cycloalkynyl" used alone or as suffix or prefix, refers to a monovalent ring-containing hydrocarbon radical having at least one carbon-carbon triple bond and comprising about 7 up to about 12 carbon atoms.

The term "aryl" used alone or as suffix or prefix, refers to a monovalent hydrocarbon radical having one or more polyunsaturated carbon rings having aromatic character, (e.g., 4n + 2 delocalized electrons) and comprising 5 up to about 14 carbon atoms.

The term "arylene" used alone or as suffix or prefix, refers to a divalent hydrocarbon radical having one or more polyunsaturated carbon rings having aromatic character, (e.g., 4n + 2 delocalized electrons) and comprising 5 up to about 14 carbon atoms, which serves to links two structures together.

The term "heterocycle" used alone or as a suffix or prefix, refers to a ring-containing structure or molecule having one or more multivalent heteroatoms, independently selected from N, O and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroalkyl" used alone or as a suffix or prefix, refers to a radical formed as a result of replacing one or more carbon atom of an alkyl with one or more heteroatoms selected from N, O and S.

The term "heteroaromatic" used alone or as a suffix or prefix, refers to a ring-containing structure or molecule having one or more multivalent heteroatoms, independently selected from N, O and S, as a part of the ring structure and including at least 3 and up to about 20 atoms in the ring(s), wherein the ring-containing structure or molecule has an aromatic character (e.g., 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocyclyl" used alone or as a suffix or prefix, refers a monovalent radical derived from a heterocycle by removing one hydrogen therefrom.

The term "heterocyclylene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocyclyl having aromatic character.

The term "heterocylcoalkyl" used alone or as a suffix or prefix, refers to a heterocyclyl that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as prefix refers to a group having a ring that contains six ring atoms.

The term "five-membered" used as prefix refers to a group having a ring that contains five ring atoms.

A five-membered ring heteroaryl is a heteroaryl with a ring having five ring atoms wherein 1,2 or 3 ring atoms are independently selected from N, O and S.

Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4- oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

The term "substituted" used as a prefix refers to a structure, molecule or group, wherein one or more hydrogens are replaced with one or more C₁₋₁₂hydrocarbon groups, or one or more chemical groups containing one or more heteroatoms selected from N, O, S, F, Cl, Br, I, and P. Exemplary chemical groups containing one or more heteroatoms include heterocyclyl, -NO₂, -OR, -R'OR, -Cl, -Br, -I, -F, -CF₃, -C(=O)R, -C(=O)OH, - NH₂, -SH, -NHR, -NR₂, -SR, -SO₃H, -SO₂R, -S(=O)R, -CN, -OH, -C(=O)OR, - C(=O)NR₂, -NRC(=O)R, -NRC(=O)OR, -R'NR₂, oxo (=O), imino (=NR), thio (=S), and oximino (=N-OR), wherein each "R" is hydrogen or a C₁₋₁₂hydrocarbyl and "R"' is a C₁₋₁₂hydrocarbyl. For example, substituted phenyl may refer to nitrophenyl, pyridylphenyl, methoxyphenyl, chlorophenyl, aminophenyl, etc., wherein the nitro, pyridyl, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

The term "optionally substituted" refers to groups, structures, or molecules that are substituted and to those that are not substituted.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole, and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofaran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocyclyl includes, for example, monocyclic heterocyclyls, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydropyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1*H*-azepinyl, homopiperazinyl, 1,3-dioxepanyl, 4,7-dihydro-1,3-dioxepinyl, and hexamethylene oxidyl.

In addition, heterocyclyl includes aromatic heterocyclyls or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocyclyl encompasses polycyclic heterocyclyls (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocyclyls described above, heterocyclyl includes polycyclic heterocyclyls wherein the ring fusion between two or more rings includes more than one bond common to both rings and more than two atoms common to both rings. Examples of such bridged heterocycles include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "amine" or "amino" used alone or as a suffix or prefix, refers to radicals of the general formula-NRR', wherein R and R' are independently selected from hydrogen or a hydrocarbon radical.

"Acyl" used alone, as a prefix or suffix, means -C(=O)-R, wherein R is an optionally substituted hydrocarbyl, hydrogen, amino or alkoxy. Acyl groups include, for example, acetyl, propionyl, benzoyl, phenyl acetyl, carboethoxy, and dimethylcarbamoyl.

"Halogen" includes fluorine, chlorine, bromine and iodine.

"Halogenated," used as a prefix of a group, means one or more hydrogens on the group is replaced with one or more halogens.

"RT" or "rt" means room temperature.

A first ring group being "fused" with a second ring group means the first ring and the second ring share at least two atoms therebetween.

"Link," "linked," or "linking," unless otherwise specified, means covalently linked or bonded.

In one aspect, the present invention provides a compound of formula I, a pharmaceutically acceptable salt thereof, diastereomers, enantiomers, or mixtures thereof: 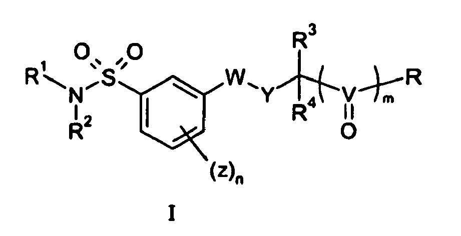 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 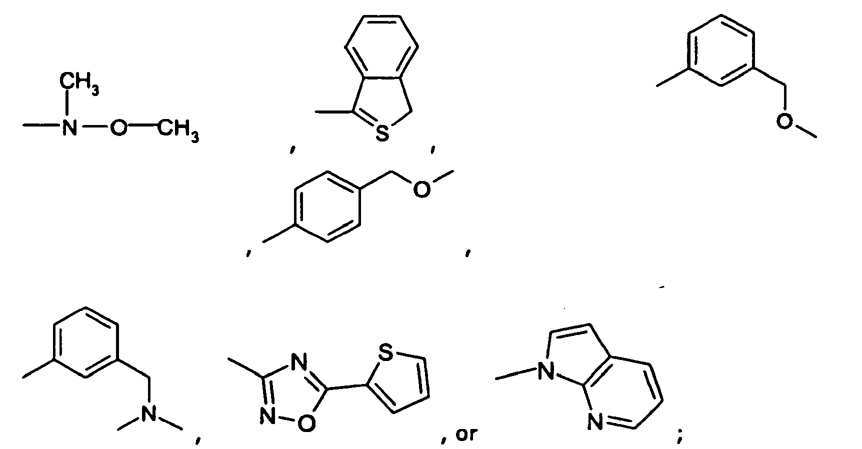
R¹ and R² are each independently C₁₋₆ alkyl, or R¹, R², and the N to which they are attached, in combination, are 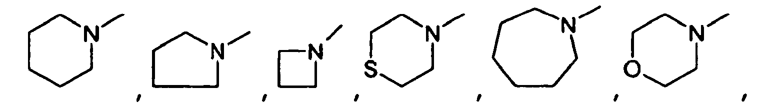 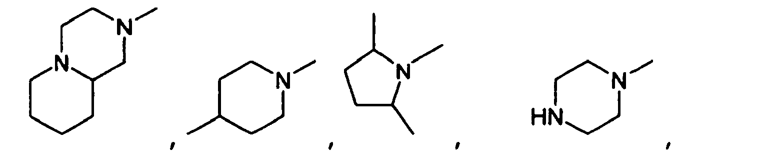 or 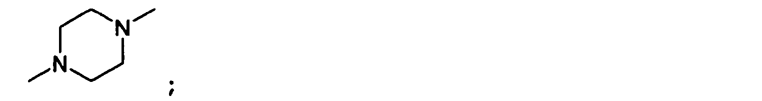
W is CH₂ or C=O;
Y is CH₂, NH, NCH₃, or O;
V is C;
m is 1;
n is 0, 1, 2, 3, or 4;
Z is, at each position, independently, Cl, F, methoxy, or methyl; and
R³and R⁴ are each, independently, H, methyl, or ethyl;
with the proviso that the compound is not
2-methyl-5-(4-morpholinyisulfonyl)-benzoic acid, 2-oxo-2-phenylethyl ester;
4-fluoro-3-(4-morpholinylsulfonyl)-benzoic acid, 2-oxo-2-phenylethyl ester;
4-methoxy-3-(4-morpholinylsulfonyl)-benzoic acid, 2-oxo-2-phenylethyl ester;
4-methyl-3-(4-morpholinylsulfonyl)-benzoic acid, 2-oxo-2-phenylethyl ester;
2-chloro-5-(1-pyrrolidinylsulfonyl)-benzoic acid, 2-oxo-2-phenylethyl ester;
4-chloro-3-(1-piperidinylsulfonyl)-benzoic acid, 2-oxo-2-phenylethyl ester;
4-chloro-3-[(hexahydro-1H-azepin-1-yl)sulfonyl]-benzoic acid, 2-oxo-2-phenylethyl ester;
2,4-dichloro-5-(1-piperidinylsulfonyl)-benzoic acid, 2-oxo-2-phenylethyl ester;
4-methyl-3-(1-piperidinylsulfonyl)-benzoic acid, 2-oxo-2-phenylethyl ester;
2-chloro-5-(1-piperidinylsulfonyl)-benzoic acid, 2-oxo-2-phenylethyl ester;
2,4-dichloro-5-(4-morphoinylsulfonyl)-benzoic acid, 2-oxo-2-phenylethyl ester;
4-chloro-3-(4-morpholinyisulfonyl)-benzoic acid, 2-oxo-2-phenylethyl ester;
2-chloro-5-(4-morpholinylsulfonyl)-benzoic acid, 2-oxo-2-phenylethyl ester;
and
3-(4-morpholinylsulfonyl)-benzoic acid, 2-oxo-2-phenylethyl ester.

In some embodiments, the compounds of the present invention are those of formula I, wherein R¹, R², and the N to which they are attached, in combination, are

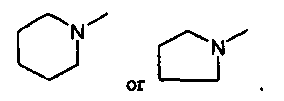

In some embodiments, the compounds of the present invention are those of formula I, wherein Z is 2-chloro.

In one embodiment of the invention there are provided compounds of formula IV 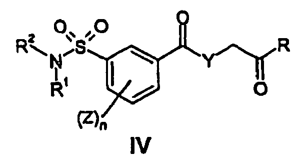 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 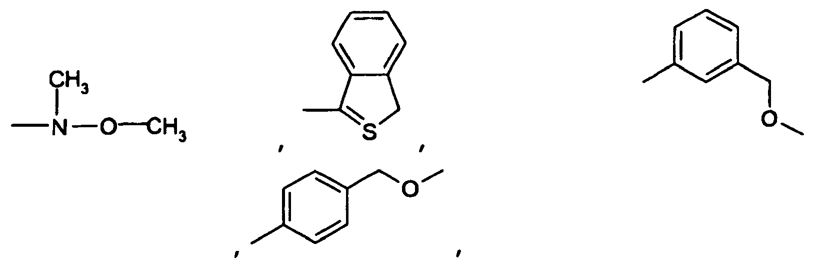 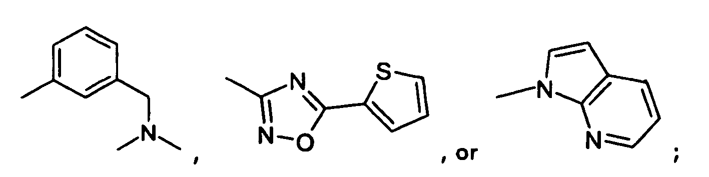
R¹ and R² are each independently C₁₋₆ alkyl, or R¹, R², and the N to which they are attached, in combination, are 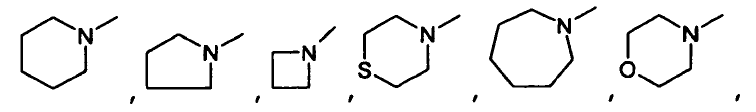 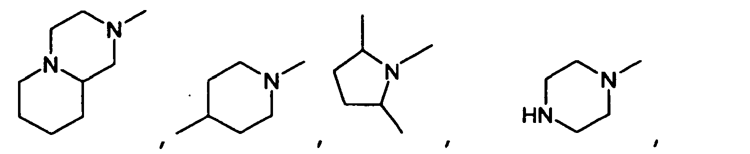 or 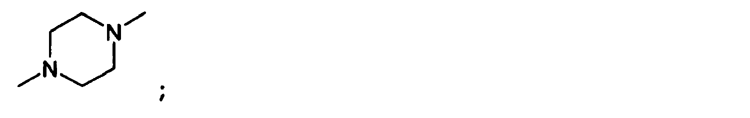
Y is CH₂, NH, NCH₃, or O;
Z is, at each position, independently, Cl, F, methoxy, or methyl; and
n is 0, 1, 2, 3, or 4.

In another embodiment of the invention there are provided compounds of formula VI 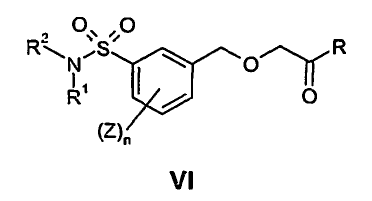 wherein
the variables R, Z, n, R⁵, and R⁶ are as described in relation to formula IV.

In still another embodiment of the invention there are provided compounds of formula IX 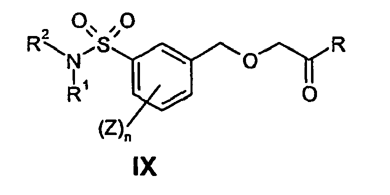 wherein
the variables R, Z, n, R⁵, and R⁶ are as described in relation to formula IV.

In yet another embodiment of the invention there are provided compounds of formula XI 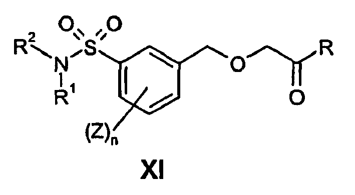 wherein
the variables R, Z, n, R⁵, and R⁶ are as described in relation to formula IV.

In yet another embodiment of the invention there are provided compounds of formula XV 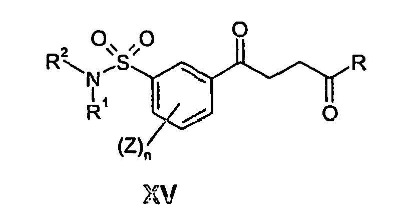 wherein
the variables R, Z, n, R⁵, and R⁶ are as described in relation to formula IV.

In yet another embodiment of the invention there are provided compounds of formula XX 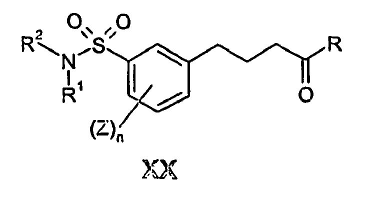 wherein
the variables R, Z, n, R⁵, and R⁶ are as described in relation to formula IV.

In a further aspect, the present invention provides a process for preparing a compound of formula I.

In one embodiment, the invention provides a process for preparing a compound of formula IV, as shown in Scheme 1.

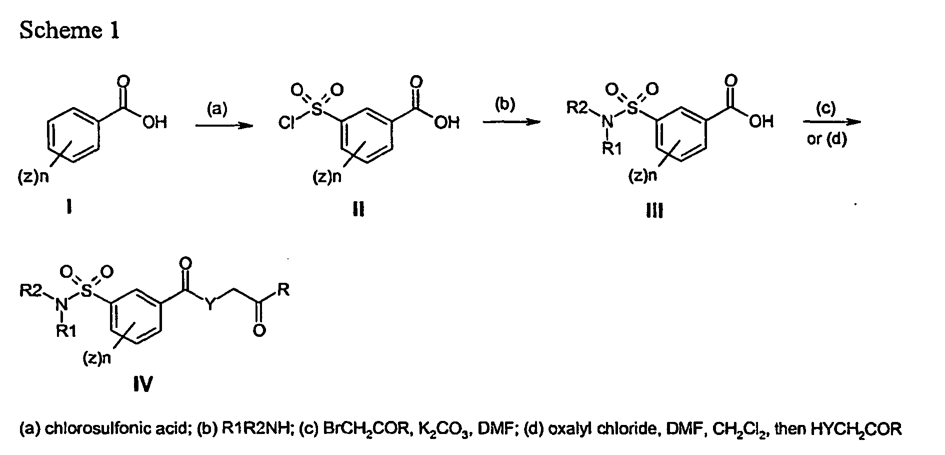

Appropriately substituted benzoic acids I were reacted with chlorosulphonic acid at 140°C to provide sulphonyl chlorides II. These sulphonyl chlorides were reacted with various amines (*i.e.,* R¹R²NH) in a solvent such as THF or benzene to prepare sulphonamides III. The acid function in III can be alkylated with an alpha-bromo ketone using a base such as potassium carbonate. Alternatively, the acid can be converted into its acid chloride using typical methods, and allowed to react with nucleophiles HYCH₂COR, where Y is O or NH, for example.

In another embodiment, the invention provides a process for preparing a compound of formula VI by the route shown in Scheme 2.

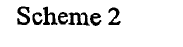

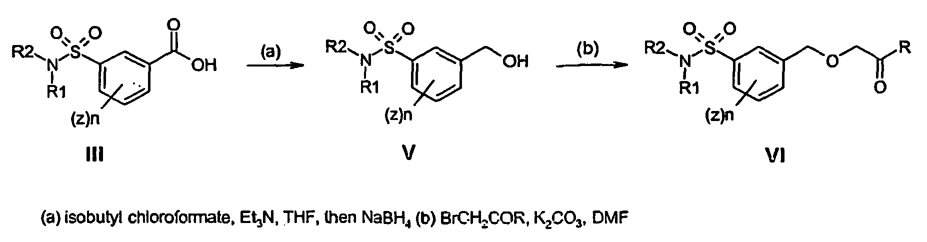

Benzoic acids III were reduced to benzyl alcohols V by first converting them to mixed anhydrides using isobutyl chloroformate, then allowing this intermediate to react with sodium borohydride. The alcohols obtained were alkylated to ethers VI using typical techniques.

In still a further embodiment, the invention provides a process for preparing compounds of formula IX as shown in Scheme 3.

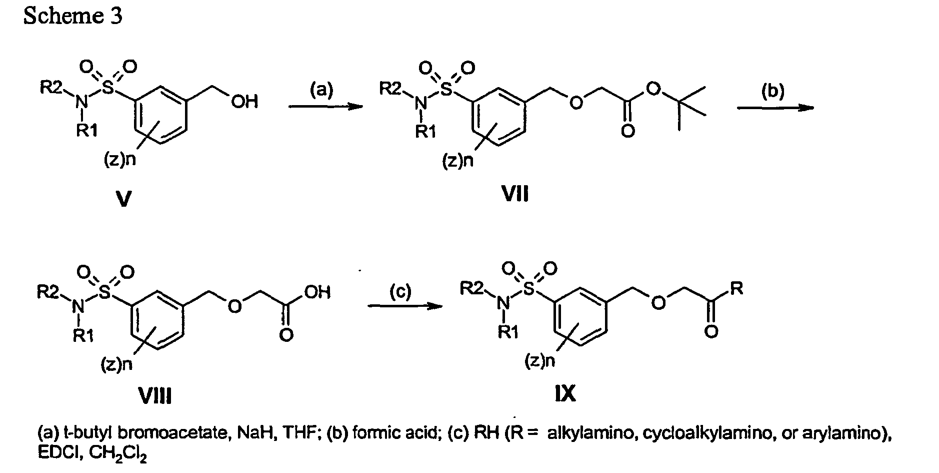

Benzyl alcohol V was alkylated with t-butyl bromoacetate using standard conditions to provide t-butyl ester VII. The t-Butyl ester VII was deprotected with formic acid to yield the acid VIII. The acid VIII was then converted to amide IX using standard amide bond forming conditions.

In yet a further embodiment, the invention provides a process for preparing compounds of formula XI by the route shown in Scheme 4.

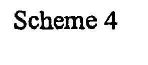

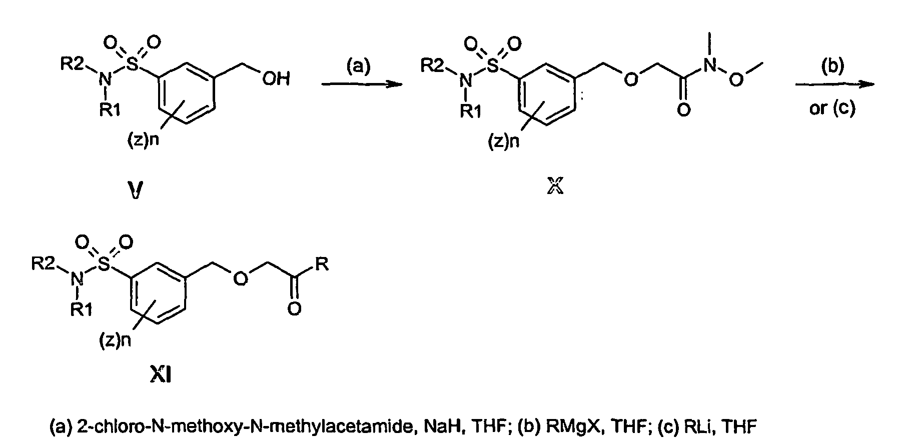

Benzyl alcohol V was alkylated with 2-chloro-N-methoxy-N-methylacetamide using standard conditions to provide amide X. Reacting X with various organometallic reagents (*e.g*., Grignard or organolithium reagents) yielded ketones XI.

In still another embodiment, the invention provides a process for preparing compounds of formula XV as described in Scheme 5.

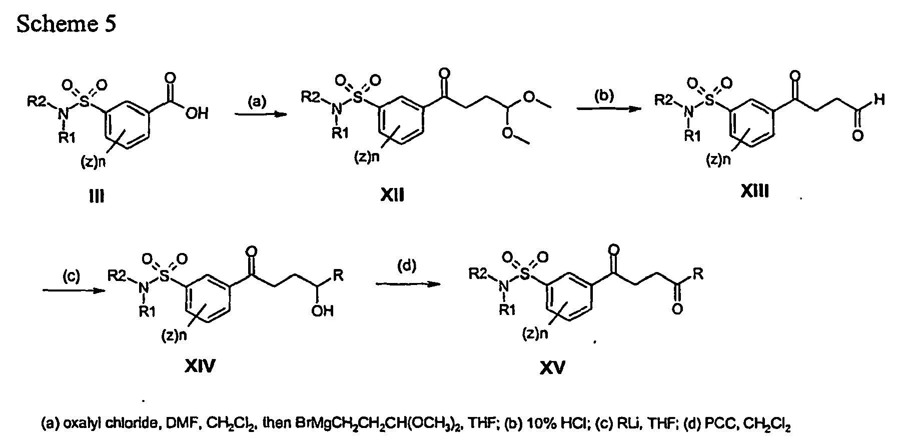

Benzoic acid III was converted to its acid chloride, then allowed to react with the Grignard reagent derived from 1-bromo-3,3-dimethoxypropane to yield the acetal XII. The protecting group was removed under acidic conditions to provide aldehyde XIII. The aldehyde was reacted with various organometallic reagents (*e.g.*, Grignard, organolithium) to provide the secondary alcohol XIV. This alcohol was oxidized to the ketone XV using standard oxidizing reagents *(e.g.,* PCC).

In yet another embodiment the invention describes a process for preparing compounds of formula XX by the route described in Scheme 6.

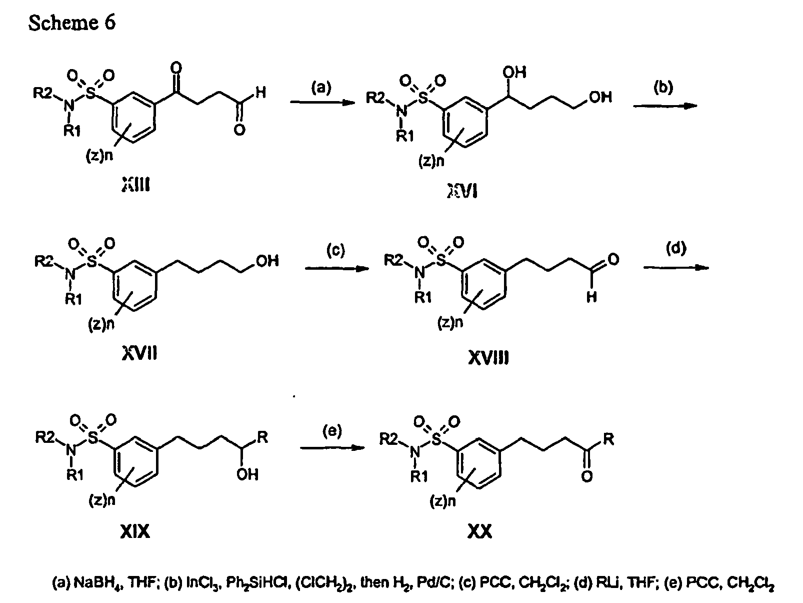

Ketoaldehyde XIII was reduced to the diol XVI using typical reducing agents (*e.g.,* sodium borohydride). The benzylic alcohol in XVI was further reduced with InCl₃ and Ph₂SiHCl. Some alkene was formed via elimination in this reaction; it was hydrogenated to provide the pure saturated alcohol XVII. This alcohol was oxidized to the aldehyde XVIII using standard oxidizing reagents (*e.g*., PCC). The aldehyde was allowed to react with various organometallic reagents (*e.g*., Grignard or organolithium) to provide the secondary alcohol XIX. The final ketone XX was obtained by standard oxidation of XIX with PCC, for example.

Specific embodiments of the present invention include:
2,4-dichloro-5-(piperidine-1-sulfonyl)-benzoic acid 2-oxo-2-phenyl-ethyl ester;
2,4-dichloro-5-(pyrrolidine-1-sulfonyl)-benzoic acid 2-oxo-2-phenyl-ethyl ester,
5-(azetidine-1-sulfonyl)-2,4-dichloro-benzoic acid 2-oxo-2-phenyl-ethyl ester,
2,4-dichloro-5-(thiomorpholine-4-sulfonyl)benzoic acid 2-oxo-2-phenylethyl ester;
2,4-dichloro-5-diethylsulfamoylbenzoic acid 2-oxo-2-phenylethyl ester;
5-(azepane-1-sulfonyl)-2,4-dichlorobenzoic acid 2-oxo-2-phenylethyl ester;
2-methoxy-5-(piperidine-1-sulfonyl)benzoic acid 2-oxo-2-phenylethyl ester;
2-chloro-5-(piperidine-1-sulfonyl)benzoic acid 2-oxo-2-phenylethyl ester;
2-chloro-4-fluoro-5-(piperidine-1-sulphonyl)benzoic acid 2-oxo-phenylethyl ester,
2-chloro-5-(piperidine-1-sulfonyl)benzoic acid 2-(2,5-dimethoxyphenyl)-2-oxoethyl ester;
2-chloro-5-(piperidine-1-sulfonyl)benzoic acid 2-(3-methoxyphenyl)-2-oxoethyl ester;
2-chloro-5-(piperidine-1-sulfonyl)benzoic acid 2-(3-chlorophenyl)-2-oxoethyl ester,
2-methyl-5-(piperidine-1-sulfonyl)benzoic acid 2-oxo-2-phenylethyl ester,
2-fluoro-5-(piperidine-1-sulfonyl)benzoic acid 2-oxo-2-phenylethyl ester,
2-chloro-5-(piperidine-1-sulfonyl)benzoic acid 2-oxo-2-pyridin-2-ylethyl ester;
2-chloro-5-(piperidine-1-sulphonyl)benzoic acid 1-methyl-2-oxo-2-phenylethyl ester;
2-chloro-5-(piperidine-1-sulphonyl)benzoic acid 2-(4-fluorophenyl)-2-oxoethyl ester;
2-chloro-5-(piperidine-1-sulphonyl)benzoic acid 2-oxo-2-p-tolylethyl ester,
2-chloro-5-(piperidine-1-sulphonyl)benzoic acid 2-oxobutyl ester;
2-chloro-5-(piperidine-1-sulphonyl)benzoic acid 2-naphthalen-2-yl-2-oxoethyl ester,
2-chloro-5-(piperidine-1-sulphonyl)benzoic acid 2-benzo[b]thiophen-3-yl-2-oxoethyl ester,
2-chloro-5-(piperidine-1-sulphonyl)benzoic acid 3,3-dimethyl-2-oxobutyl ester,
2-chloro-5-(morpholine-4-sulphonyl)benzoic acid 2-oxo-2-phenylethyl ester,
5-(butylethylsulphamoyl)-2-chlorobenzoic acid 2-oxo-2-phenylethyl ester;
2-chloro-5-dimethylsulphamoylbenzoic acid 2-oxo-2-phenylethyl ester;
2-chloro-5-(octahydropyrido[1,2-a]pyrazine-2-sulphonyl)benzoic acid 2-oxo-2-phenylethyl ester;
2-chloro-5-(4-methylpiperidine-1-sulphonyl)benzoic acid 2-oxo-2-phenylethyl ester;
2-chloro-5-(2,5-dimethylpyrollidine-1-sulphonyl)benzoic acid 2-oxo-2-phenylethyl ester,
3-(piperidine-1-sulphonyl)benzoic acid 2-oxo-2-phenylethyl ester;
N-2-oxo-2-phenethyl 2,4-dichloro-5-[piperidinosulfonyl] benzamide;
2-chloro-4-fluoro-N-(2-oxo-2-phenylethyl)-5-(piperidine-1-sulphonyl) benzamide;
2-oxo-2-phenethyl 2,4-dichloro-5-[piperazinosulfonyl] benzoate hydrochloride;
2-oxo-2-phenethyl 2,4-dichloro-5-[4-methyl-piperazinosulfonyl] benzoate hydrochloride;
2,4-dichloro-5-[piperidiinosulfonyl] benzyloxyacetophenone;
2-chloro-5-(piperidine-1-sulphonyl)benzoic acid 1,1-dimethyl-2-oxo-2-phenylethyl ester;
[2-chloro-5-(piperidine-l-sulphonyl)benzylamino] acetic acid tert-butyl ester;
{[2-chloro-5-(piperidine-1-sulfonyl)-benzyl]-methyl-amino}-acetic acid tert-butyl ester;
(2-chloro-5-(piperidine-1-sulphonyl)benzyloxy)acetic acid tert-butyl ester;
2-[2-chloro-5-(piperidine-1-sulfonyl)benzyloxy]propionic acid tert-butyl ester;
2-[2-chloro-5-(piperidine-1-sulfonyl)benzyloxy]butyric acid tert-butyl ester;
2-(2-chloro-5-(piperidine-1-sulphonyl)benzyloxy)-1-pyrrolidin-1-ylethanone;
2-(2-chloro-5-(piperidine-1-sulphonyl)benzyloxy)-N-methyl-N-phenylacetamide;
N-tert-butyl-2-(2-chloro-5-(piperidine-1-sulphonyl)benzyloxy)acetamide;
(1-(2-(2-chloro-5-piperidine-1-sulphonyl)benzyloxy)acetyl)pyrrolidin-3-yl)carbamic acid tert-butyl ester;
(1-(2-(2-chloro-5-(piperidine-1-sulphonyl)benzyloxy)acetyl)piperidin-4-yl)carbamic acid tert-butyl ester;
2-[2-chloro-5-(piperidine-1-sulfonyl)-benzyloxy]-N-pyridin-2-yl-acetamide;
2-[2-chloro-5-piperidine-1-sulphonyl)benzyloxy]-1-piperidin-1-ylethanone;
2-[2-chloro-5-(piperidine-1-sulfonyl)-benzyloxy]-N-methyl-N-pyridin-2-yl-acetamide;
2-(2,4-dichloro-5-(piperidine-1-sulphonyl)benzyloxy)-N-methoxy-N-methylacetamide;
2-(2-chloro-5-(piperidine-1-sulphonyl)benzyloxy)-N-methoxy-N-methylacetamide;
N-methoxy-2-[2-methoxy-5-(piperidine-1-sulphonyl)-benzyloxy)-N-methylacetamide;
N-methoxy-2-[2-methoxy-5-(piperidine-1-sulphonyl)-benzyloxy)-N-methylacetamide;
2-[2-bromo-5-(piperidine-1-sulphonyl)-benzyloxy)-N-methoxy-N-methyl-acetamide;
N-methoxy-N-methyl-2-[5-(piperidine-1-sulphonyl)-2-trifluoromethoxy-benzyloxy]-acetamide;
2-(1-(2-chloro-5-(piperidine-1-sulfonyl)-phenyl)-ethoxy)-N-methoxy-N-methylacetamide;
2-[2-chloro-5-(thiomorpholine-4-sulfonyl)-benzyloxy]-N-methoxy-N-methylacetamide;
2-[2-chloro-5-(piperidine-1-sulphonyl)benzyloxy]-1-(3-methoxyphenyl)ethanone;
2-[2-chloro-5-(piperidine-1-sulphonyl)benzyloxy]-1-cyclohexylethanone;
2-[2-chloro-5-(piperidine-1-sulfonyl)benzyloxy]-1-(2,5-dimethoxyphenyl)ethanone;
2-[2,4-dichloro-5-(piperidine-1-sulphonyl)benzyloxy]-1-(3-methoxyphenyl)ethanone;
1-(3-chlorophenyl)-2-[2-chloro-5-(piperidine-1-sulphonyl)benzyloxy]-ethanone;
1-(3-chlorophenyl)-2-[2,4-dichloro-5-(piperidine-1-sulphonyl)benzyloxy]-ethanone;
2-[2,4-dichloro-5-(piperidine-1-sulphonyl)benzyloxy]-1-naphthalen-2-ylethanone;
2-[2-chloro-5-(piperidine-1-sulphonyl)benzyloxy]-1-naphthalen-2-ylethanone;
4-chloro-N,N-dimethyl-5-(2-oxo-2-phenylethoxymethyl)benzenesulphonamide;
4-chloro-N,N-diethyl-3-(2-oxo-2-phenylethoxymethyl)benzenesulphonamide;
4-chloro-N-ethyl-N-methyl-3-(2-oxo-2-phenylethoxymethyl)benzenesulphonamide;
4-chloro-N-methyl-3-(2-oxo-2-phenylethoxymethyl)-N-propylbenzenesulphonamide;
2-[2-bromo-5(piperidine-1-sulphonyl)-benzyloxy]-1-phenyl-ethanone;
2-[2-chloro-5-(thiomorpholine-4-sulfonyl)-benzyloxy]-1-phenyl-ethanone;
2-(2-chloro-5-(piperidine-1-sulfonyl)-benzyloxyl)-1-(1H-pyrrolo(2,3-b)-pyridin-3-yl)-ethanone;
2-[2-chloro-5-(4-methylpiperazine-1-sulfonyl)benzyloxy]-1-pyridin-2-yl-ethanone;
2-[2-chloro-5-(piperidine-1-sulphonyl)benzyloxy]-1-pyridin-3-ylethanone;
2-[2-chloro-5-(piperidine-1-sulphonyl)benzyloxy]-1-pyridin-4-ylethanone;
2-[2-chloro-5-(piperidine-1-sulphonyl)benzyloxy]-1-pyridin-2-ylethanone;
4-chloro-N,N-dimethyl-3-(2-oxo-2-pyridin-3-ylethoxymethyl)benzenesulphonamide;
2-[2-chloro-5-(piperidine-1-sulphonyl)benzyloxy]-1-quinolin-8-ylethanone;
4-chloro-N,N-dimethyl-3-(2-oxo-2-pyridin-2-ylethoxymethyl)benzenesulphonamide;
4-chloro-N-ethyl-N-methyl-3-(2-oxo-2-pyridin-2-ylethoxymethyl)benzenesulphonamide;
4-chloro-N,N-diethyl-3-(2-oxo-2-pyridin-2-ylethoxymethyl)benzenesulphonamide;
4-chloro-N-methyl-3-(2-oxo-2-pyridin-2-ylethoxymethyl)-N-propylbenzenesulphonamide;
4-chloro-N-ethyl-N-methyl-3-(2-oxo-2-pyridin-3-ylethoxymethyl)benzenesulphonamide;
4-chloro-N,N-diethyl-3-(2-oxo-2-pyridin-3-ylethoxymethyl)benzenesulphonamide;
2-[2-chloro-5-(piperidine-1-sulpfonyl)-benzyloxy]-1-(4-methyl-pyridin-2-yl)-ethanone;
2-[2-chloro-5-(piperidine-1-sulpfonyl)-benzyloxy]-1-(6-methyl-pyridin-2-yl)-ethanone;
2-[2-chloro-5-(piperidine-1-sulpfonyl)-benzyloxy]-1-(5-methyl-pyridm-2-yl)-ethanone;
4-chloro-N-ethyl-N-methyl-3-[2-(4-methyl-pyridin-2-yl)-2-oxo-ethoxymethyl]-benzenesulfonamide;
4-chloro-N-ethyl-N-methyl-3-[2-(6-methyl-pyridin-2-yl)-2-oxo-ethoxymethyl]-benzenesulfonamide;
4-chloro-N-ethyl-N-methyl-3-[2-(5-methyl-pyridin-2-yl)-2-oxo-ethoxymethyl]-benzenesulfonamide;
2-[2-chloro-5-(piperidine-1-sulpfonyl)-benzyloxy]-1-(3-methyl-pyridin-2-yl)-ethanone;
4-chloro-N-ethyl-N-methyl-3-[2-(3-methyl-pyridin-2-yl)-2-oxo-ethoxymethyl]-benzenesulfonamide;
1-(2-bromo-5-dimethylamino-pyridin-4-yl)-2-[2-chloro-5-(piperidine-1-sulpfonyl)-benzyloxy]-ethanone;
2-[2-methoxy-5-(piperidine-1-sulpfonyl)-benzyloxy]-1-pyridin-2-yl-ethanone;
2-[2-methyl-5-(piperidine-1-sulpfonyl)-benzyloxy]-1-pyridin-2-yl-ethanone;
2-5-(piperidine-1-sulpfonyl)-2-trifluoromethoxy-benzyloxy]-1-pyridin-2-yl-ethanone;
2-(1-(2-chloro-5-(piperidine-1-sulfonyl)-phenyl)-ethoxy)-1-pyridine-2-yl-ethanone;
2-(1-(2-chloro-5-(piperidine-1-sulfonyl)-phenyl)-ethoxy)-1-pyridine-3-yl-ethanone;
2-(2-chloro-5-(piperidine-l-sulfonyl)-benzyloxy)- 1-pyrazine-2-yl-ethanone;
2-(2-chloro-5-(piperidine-1-sulfonyl)-benzyloxy)-1-(1-methyl-1H-imidazol-2-yl)-ethanone;
2-(2-chloro-5-(piperidine-1-sulfonyl)-benzyloxy)-1-(1-pyridin-2-yl-1H-imidazol-2-yl)ethanone;
2-(2-chloro-5-(piperidine-1-sulfonyl)-benzyloxy)- 1-(1-methyl-1H-indol-5-yl)-ethanone;
2-[2-chloro-5-(piperidine-1-sulfonyl)benzyloxy]-1-pyridin-2-yl-propan-1-one;
2-[2,4-dichloro-5-(piperidine-1-sulphonyl)benzyloxy]-1-(3-methoxymethylphenyl)ethanone;
2-[2,4-dichloro-5-(piperidine-1-sulphonyl)benzyloxy]-1-(4-methoxymethylphenyl)ethanone;
2-[2,4-dichloro-5-(piperidine-1-sulphonyl)benzyloxy]-1-(3-dimethylaminomethylphenyl)ethanone;
2-[2-chloro-5-(piperidine-1-sulfonyl)benzyloxy]-1-(3-dimethylaininomethylphenyl) ethanone;
2-(2-Oxo-2-phenyl-ethoxymethyl)-4-(piperidine-1-sulfonyl)-benzonitrile;
4-[2-chloro-5-(piperidine-1-sulfonyl)phenyl]4-oxobutyraldehyde;
4-[2-methyl-5-(piperidine-1-sulfonyl)phenyl]-4-oxobutyraldehyde;
4-[2-chloro-5-(piperidine-1-sulfonyl)phenyl]-butyraldehyde;
1-[2-chloro-5-(piperidine-1-sulfonyl)phenyl]-4-naphthalen-2-ylbutane-1,4-dione;
1-[2-chloro-5-(piperidine-1-sulfonyl)phenyl]-4-naphthalen-1-ylbutane-1,4-dione;
1-[2-chloro-5-(piperidine-1-sulfonyl)phenyl]-4-phenylbutane-1,4-dione;
1-[2-chloro-5-(piperidine-1-sulfonyl)phenyl]-4-pyridin-3-ylbutane-1,4-dione;
1-[2-chloro-5-(piperidine-1-sulfonyl)phenyl]-4-(3-dimethylaminomethylphenyl)butane-1,4-dione;
4-[2-chloro-5-(piperidine-1-sulfonyl)phenyl]-1-phenyl-butan-1-one;
4-[2-chloro-5-(piperidine-1-sulfonyl)phenyl]-1-pyridine-3-yl-butan-1-one;
1-[2-chloro-5-(piperidine-1-sulfonyl)phenyl]-4-pyridin-2-ylbutane-1,4-dione;
1-[2-methyl-5-(piperidine-1-sulfonyl)phenyl]-4-pyridin-2-ylbutane-1,4-dione;
4-[2-chloro-5-(piperidine-1-sulfonyl)phenyl]-1-pyridine-2-yl-butan-1-one;
1-(4-chloro-3-(5-thiophene-2-yl-(1,2,4)oxadiazol-3-yl methoxy methyl)-benzene sulfonyl)-piperidine;
(3-(3-benzenesulfinyl-propenyl)-4-chloro-benzenesulfonyl)-piperidine;
2-[2-chloro-5-(piperidine-1-sulfonyl)benzyloxy]-1-pyrrolo[2,3-b]pyridin-1-ylethanone; and
2-[2-chloro-5-piperidine-1-sulfonyl)benzylamino]-1-piperidin-1-ylethanone.

It will be understood by those of skill in the art that when compounds of the present invention contain one or more chiral centers, the compounds of the invention may exist in, and be isolated as, enantiomeric or diastereomeric forms, or as a racemic mixture. The present invention includes any possible enantiomers, diastereomers, racemates or mixtures thereof, of a compound of formula I, IV, VI, IX, XI, XV, or XX. The optically active forms of the compound of the invention may be prepared, for example, by chiral chromatographic separation of a racemate, by synthesis from optically active starting materials or by asymmetric synthesis based on the procedures described thereafter.

It will also be appreciated by those of skill in the art that certain compounds of the present invention may exist as geometrical isomers, for example E and Z isomers of alkenes. The present invention includes any geometrical isomer of a compound of formula I, IV, VI, IX, XI, XV, or XX. It will further be understood that the present invention encompasses tautomers of the compounds of formula I, IV, VI, IX, XI, XV, or XX.

It will also be understood by those of skill in the art that certain compounds of the present invention may exist in solvated, for example hydrated, as well as unsolvated forms. It will further be understood that the present invention encompasses all such solvated forms of the compounds of formula I, N, VI, IX, XI, XV, or XX.

Within the scope of the invention are also salts of the compounds of formula I, IV, VI, IX, XI, XV, or XX. Generally, pharmaceutically acceptable salts of compounds of the present invention are obtained using standard procedures well known in the art, for example, by reacting a sufficiently basic compound, for example an alkyl amine with a suitable acid, for example, HCl or acetic acid, to afford a physiologically acceptable anion. It is also possible to make a corresponding alkali metal (such as sodium, potassium, or lithium) or an alkaline earth metal (such as a calcium) salt by treating a compound of the present invention having a suitably acidic proton, such as a carboxylic acid or a phenol with one equivalent of an alkali metal or alkaline earth metal hydroxide or alkoxide (such as the ethoxide or methoxide), or a suitably basic organic amine (such as choline or meglumine) in an aqueous medium, followed by conventional purification techniques.

In one embodiment of the present invention, the compound of formula I, IV, VI, IX, XI, XV, or XX may be converted to a pharmaceutically acceptable salt or solvate thereof, particularly, an acid addition salt such as a hydrochloride, hydrobromide, phosphate, acetate, fumarate, maleate, tartrate, citrate, methanesulphonate orp-toluenesulphonate.

We have discovered that the compounds of the present invention have activity as pharmaceuticals, in particular as modulators of metabotropic glutamate receptors. More particularly, the compounds of the present invention exhibit activity as potentiators of the mGluR2 receptor, and are useful in therapy, in particular for the treatment of neurological and psychiatric disorders associated with glutamate dysfunction.

Compounds of the present invention are useful in treating neurological and psychiatric disorders including, but are not limited to, neurological and psychiatric disorders such as cerebral deficit subsequent to cardiac bypass surgery and grafting, stroke, cerebral ischemia, spinal cord trauma, head trauma, perinatal hypoxia, cardiac arrest, hypoglycemic neuronal damage, dementia (including AIDS-induced dementia), Alzheimer's disease, Huntington's Chorea, amyotrophic lateral sclerosis, ocular damage, retinopathy, cognitive disorders, idiopathic and drug-induced Parkinson's disease, muscular spasms and disorders associated with muscular spasticity including tremors, epilepsy, convulsions, migraine (including migraine headache), urinary incontinence, substance tolerance, substance withdrawal (including, substances such as opiates, nicotine, tobacco products, alcohol, benzodiazepines, cocaine, sedatives, hypnotics, etc.), psychosis, schizophrenia, anxiety (including generalized anxiety disorder, panic disorder, and obsessive compulsive disorder, and post-traumatic stress disorder (FTSD)), mood disorders (including depression, mania, bipolar disorders), circadian rhythm disorders (including jet lag and shift work), trigeminal neuralgia, hearing loss, tinnitus, macular degeneration of the eye, emesis, brain edema, pain (including acute and chronic pain states, severe pain, intractable pain, neuropathic pain, inflammatory pain, and post-traumatic pain), tardive dyskinesia, sleep disorders (including narcolepsy), attention deficit/hyperactivity disorder, and conduct disorder.

Also within the scope of the invention is the use of any of the compounds according to formula I, IV, VI, IX, XI, XV, or XX, or a pharmaceutically acceptable-salt or solvate thereof, for the manufacture of a medicament for the treatment of any of the conditions discussed above.

Described is also a method for the treatment of a subject suffering from any of the conditions discussed above, whereby an effective amount of a compound according to formula I, IV, VI, IX, XI, XV, or XX, or a pharmaceutically acceptable salt or solvate thereof, is administered to a patient in need of such treatment.

Thus, the invention provides a compound of formula I, IV, VI, IX, XI, XV, or XX, or pharmaceutically acceptable salt or solvate thereof, as hereinbefore defined for use in therapy.

In a further aspect, the present invention provides the use of a compound of formula I, IV, VI, IX, XI, XV, or XX, or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The term "therapeutic" and "therapeutically" should be construed accordingly. The term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic disorders.

The compounds of the present invention are useful in therapy, especially for the therapy of neurological and psychiatric disorders and conditions associated with glutamate dysfunction.

In use for therapy in a warm-blooded animal such as a human, the compounds of the present invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

In some embodiments of the invention, the route of administration is oral, intravenous, or intramuscular.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of the present invention, inert, pharmaceutically acceptable carriers can be either solid or liquid. Solid form preparations include, but are not limited to, powders, tablets, dispersible granules, capsules, cachets, and suppositories.

A solid carrier can be one or more substances, which may also act as diluents, flavoring agents, solubilizers, lubricants, suspending agents, binders, or table disintegrating agents. A solid carrier can also be an encapsulating material.

In powders, the carrier is a finely divided solid, which is in a mixture with the finely divided compound of the invention, or the active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized moulds and allowed to cool and solidify.

Suitable carriers include, but are not limited to, magnesium carbonate, magnesium stearate, talc, lactose, sugar, pectin, dextrin, starch, tragacanth, methyl cellulose, sodium carboxymethyl cellulose, low-melting wax, cocoa butter, and the like.

The term composition is also intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form compositions include solutions, suspensions, and emulsions. For example, sterile water or water propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will include from about 0.05%w (percent by weight) to about 99%w, more particularly, from about 0.10%w to 50%w, of the compound of the invention, all percentages by weight being based on total composition.

A therapeutically effective amount for the practice of the present invention can be determined, by the use of known criteria including the age, weight and response of the individual patient, and interpreted within the context of the disease which is being treated or which is being prevented, by one of ordinary skill in the art.

Within the scope of the invention is the use of any compound of formula I, IV, VI, IX, XI, XV, or XX as defined above for the manufacture of a medicament.

Also within the scope of the invention is the use of any compound of formula I, IV, VI, IX, XI, XV, or XX for the manufacture of a medicament for the therapy of neurological and psychiatric disorders including, but are not limited to, acute neurological and psychiatric disorders such as cerebral deficit subsequent to cardiac bypass surgery and grafting, stroke, cerebral ischemia, spinal cord trauma, head trauma, perinatal hypoxia, cardiac arrest, hypoglycemic neuronal damage, dementia (including AIDS-induced dementia), Alzheimer's disease, Huntington's Chorea, amyotrophic lateral sclerosis, ocular damage, retinopathy, cognitive disorders, idiopathic and drug-induced Parkinson's disease, muscular spasms and disorders associated with muscular spasticity including tremors, epilepsy, convulsions, migraine (including migraine headache), urinary incontinence, substance tolerance, substance withdrawal (including, substances such as opiates, nicotine, tobacco products, alcohol, benzodiazepines, cocaine, sedatives, hypnotics, etc.), psychosis, schizophrenia, anxiety (including generalized anxiety disorder, panic disorder, and obsessive compulsive disorder), mood disorders (including depression, mania, bipolar disorders), trigeminal neuralgia, hearing loss, tinnitus, macular degeneration of the eye, emesis, brain edema, pain (including acute and chronic pain states, sever pain, intractable pain, neuropathic pain, and post-traumatic pain), tardive dyskinesia, sleep disorders (including narcolepsy), attention deficit/hyperactivity disorder, and conduct disorder.

Additionally provided is the use of any compound according to formula I, IV, VI, IX, XI, XV, or XX for the manufacture of a medicament for the therapy of neurological and psychiatric disorders.

Also described is a method for therapy of a subject suffering from any of the conditions discussed above, whereby an effective amount of a compound according to formula I, IV, VI, IX, XI, XV, or XX, is administered to a patient in need of such therapy.

Additionally, there is provided a pharmaceutical composition comprising a compound of formula I, IV, VI, IX, XI, XV, or XX, or a pharmaceutically acceptable salt or solvate thereof, in association with a pharmaceutically acceptable carrier or excipient.

Particularly, there is provided a pharmaceutical composition comprising a compound of formula I, IV, VI, IX, XI, XV, or XX, or a pharmaceutically acceptable salt or solvate thereof, in association with a pharmaceutically acceptable carrier or excipient for therapy, more particularly for therapy of neurological and psychiatric disorders.

In another aspect of the present invention, there is provided a pharmaceutical composition comprising a compound of formula I, IV, VI, IX, XI, XV, or XX, or a pharmaceutically acceptable salt or solvate thereof, in association with a pharmaceutically acceptable carrier or excipient for use in any of the conditions discussed above.

The compounds described herein may be provided or delivered in a form suitable for oral use, for example, in a tablet, lozenge, hard and soft capsule, aqueous solution, oily solution, emulsion, and suspension. The compounds of the present invention can be provided for topical administration, for example, as a cream, ointment, gel, spray, or aqueous solution, oily solution, emulsion or suspension. The compounds described herein can be provided in a form suitable for nasal administration, for example, as a nasal spray, nasal drops, or dry powder. The compounds can be administered to the vagina or rectum in the form of a suppository. The compounds described herein can be administered parentally, for example, by intravenous, intravesicular, subcutaneous, or intramuscular injection or infusion. The compounds can be administered by insufflation (for example as a finely divided powder). The compounds may also be administered transdermally or sublingually.

The compositions of the present invention can accordingly be obtained by conventional procedures using conventional pharmaceutical excipients, well known in the art. Thus, compositions intended for oral use may contain, for example, one or more coloring, sweetening, flavoring and/or preservative agents.

The amount of active ingredient that is combined with one or more excipients to produce a single dosage form will necessarily vary depending upon the host treated and the particular route of administration. The size of the dose for therapeutic or prophylactic purposes of a compound of formula I, IV, VI, IX, XI, XV, or XX will naturally vary according to the nature and severity of the conditions, the age and sex of the animal or patient and the route of administration, according to well known principles of medicine.

In addition to their use in therapeutic medicine, the compounds of formula I, IV, VI, IX, XI, XV, or XX, or salts thereof, are also useful as pharmacological tools in the development and standardisation of *in vitro* and in vivo test systems for the evaluation of the effects of inhibitors of mGluR-related activity in laboratory animals such as, for example, cats, dogs, rabbits, monkeys, rats and mice, as part of the search for new therapeutics agents.

We have discovered that the compounds of the present invention are active as modulators of mGluR2 function.

Generally the compounds were active in the following assays at concentrations (or with EC₅₀ values) less than 10 µM.

The pharmacological properties of the compounds of the invention can be analyzed using standard assays for functional activity. Examples of glutamate receptor assays are well known in the art as described in, for example, Aramori et al., 1992, Neuron, 8:757; Tanabe et al., 1992, Neuron, 8:169; Miller et al., 1995, J. Neuroscience, 15:6103; Balazs, et al., 1997, J. Neurochemistry, 1997,69:151. Conveniently, the compounds of the invention can be studied by means of an assay that measures the mobilization of intracellular calcium, [Ca²⁺]ᵢ in cells expressing mGluR2.

Fluorometric Imaging Plate Reader (FLIPR) analysis was used to detect allosteric activators of mGluR2 via calcium mobilization. A clonal HEK 293 cell line expressing a chimeric mGluR2/CaR construct comprising the extracellular and transmembrane domains of human mGluR2 and the intracellular domain of the human calcium receptor, fused to the promiscuous chimeric protein G_{αqi5} was used. Activation of this construct by agonists or allosteric activators resulted in stimulation of the PLC pathway and the subsequent mobilization of intracellular Ca²⁺ which was measured via FLIPR analysis. At 24-hours prior to analysis, the cells were trypsinized and plated in DMEM at 100,000 cells/well in black sided, clear-bottom, collagen I coated, 96-well plates. The plates were incubated under 5% CO₂ at 37°C overnight. Cells were loaded with 6µM fluo-3 acetoxymethylester (Molecular Probes, Eugene Oregon) for 60 minutes at room temperature. All assays were performed in a buffer containing 126mM NaCl, 5mM KCl, 1mM MgCl₂, 1mM CaCl₂, 20mM Hepes, 0.06µM DCG-IV (a Group II mGluR selective agonist), supplemented with 1.0mg/ml D-glucose and 1.0mg/ml BSA fraction IV (pH 7.4).

FLIPR experiments were done using a laser setting of 0.8 W and a 0.4 second CCD camera shutter speed. Extracellular fluo-3 was washed off and cells were maintained in 160 µL of buffer and placed in the FLIPR. An addition of test compound (0.01 µM to 30µM in duplicate) was made after 10 seconds of baseline fluorescent readings were recorded on FLIPR. Fluorescent signals were then recorded for an additional 75 seconds at which point a second addition of DCG-IV (0.2µM) was made and fluorescent signals were recorded for an additional 65 seconds. Fluorescent signals were measured as the peak height of the response within the sample period. Data was analyzed using Assay Explorer, and EC₅₀ and Eₘₐₓ values (relative to maximum DCG-IV effect) were calculated using a four parameter logistic equation.

A [³⁵S]-GTPγS binding assay was used to functionally assay mGluR2 receptor activation. The allosteric activator activity of compounds at the human mGluR2 receptor were measured using a [³⁵S]-GTPγS binding assay with membranes prepared from CHO cells which stably express the human mGluR2. The assay is based upon the principle that agonists bind to G-protein coupled receptors to stimulate GDP-GTP exchange at the G-protein. Since [³⁵S]-GTPγS is a non-hydrolyzable GTP analog, it can be used to provide an index of GDP-GTP exchange and, thus, receptor activation. The GTPγS binding assay therefore provides a quantitative measure of receptor activation.

Membranes were prepared from CHO cells stably transfected with human mGluR2. Membranes (30 µg protein) were incubated with test compound (3nM to 300µM) for 15 minutes at room temperature prior to the addition of 1 µM glutamate, and incubated for 30 min at 30°C in 500 µl assay buffer (20 mM HEPES, 100mM NaCl, 10mM MgCl₂), containing 30µM GDP and 0.1nM [³⁵S]-GTPγS (1250 Ci/mmol). Reactions were carried out in triplicate in 2 ml polypropylene 96-well plates. Reactions were terminated by vacuum filtration using a Packard 96-well harvester and Unifilter-96, GF/B filter microplates. The filter plates were washed 4 x 1.5 ml with ice-cold wash buffer (10mM sodium phosphate buffer, pH 7.4). The filter plates were dried and 35 µl of scintillation fluid (Microscint 20) was added to each well. The amount of radioactivity bound was determined by counting plates on the Packard TopCount. Data was analyzed using GraphPad Prism, and EC₅₀ and Eₘₐₓ values (relative to the maximum glutamate effect) were calculated using non-linear regression.

The invention is further illustrated by way of the following examples, which are intended to elaborate several embodiments of the invention. These examples are not intended to, nor are they to be construed to, limit the scope of the invention. It will be clear that the invention may be practiced otherwise than as particularly described herein.

### EXAMPLES

### General Synthesis of Chlorosulfonylbenzoic Acids

To chlorosulfonic acid (as solvent) cooled in an ice-bath was slowly added an appropriate benzoic acid. After the addition of the acid, the resulting mixture was heated to 140°C for 3 h. The mixture was then cooled to room temperature and added dropwise to stirred ice-water (150 mL). The resulting precipitate was filtered and the solid residue obtained was used directly in the next step.

The following chlorosulfonylbenzoic acids were synthesized using the above procedure.
2,4-Dichloro-5-chlorosulfonyl benzoic acid (4.3 g, beige solid, 57% yield) from 2,4-dichlorobenzoic acid (5.0 g, 26.2 mmol).
2-Chloro-5-chlorosulfonyl benzoic acid (4.3 g, beige solid, quantitative yield) from 2,-chlorobenzoic acid (5.0 g, 31.9 mmol).
2-Methyl-5-chlorosulfonyl benzoic acid (3.2 g, white solid, 73% yield) from 2,-methylbenzoic acid (2.5 g, 17.8 mmol).
2-Fluoro-5-chlorosulfonyl benzoic acid (3.1 g, white solid, 74% yield) from 2,-methylbenzoic acid (2.5 g, 18.4 mmol).

### General Synthesis of Alkylaminosulfonylbenzoic Acids

To a mixture of an appropriate chlorosulfonylbenzoic acid in benzene or THF was added the alkylamine. The mixture was then stirred at room temperature for 2 h after which the solvent was removed *in vacuo* and the residue was diluted with 1N HCl. The reaction mixture was then extracted with ethyl acetate or dichloromethane, and the organic extract was washed with brine, dried over MgSO₄ (anhydrous). The organic solvent was removed *in vacuo* and the product was triturated with 10% ethyl acetate/hexane.

The following alkylaminosulfonylbenzoic acids were synthesized using the above procedure.
2,4-Dichloro-5-[piperidinosulfonyl]benzoic acid (850 mg, white solid, 73% yield) from piperidine (1.16g, 0.6 mmol) and 2,4-dichloro-5-chlorosulfonyl benzoic acid (1.0g, 3.45 mmol) in benzene (10 mL).
2,4-Dichloro-5-[pyrrolindinosulfonyl]benzoic acid (41 mg, white solid, 41 % yield) from pyrrolidine (168 mg, 2.36 mmol) and 2,4-dichloro-5-chlorosulfonyl benzoic acid (200 mg, 0.6 mmol) in benzene (5 mL).
2,4-Dichloro-5-[azetidinosulfonyl]benzoic acid (92 mg, white solid, 49%yield) from azetidine (135 mg, 2.36 mmol) and 2,4-dichloro-5-chlorosulfonyl benzoic acid (200 mg, 0.6 mmol) in benzene (5 mL).
2,4-Dichloro-5-[N,N-diethylaminosulfonyl]benzoic acid (off-white, quantitative yield) from N,N-diethylamine (125 mg, 1.71 mmol) and 2,4-dichloro-5-chlorosulfonyl benzoic acid (100 mg, 0.35 mmol) in benzene (5 mL).
2,4-Dichloro-5-[thiomorpholinosulfonyl]benzoic acid (off-white solid, quantitative yield) from thiomorpholine (176 mg, 1.71 mmol) and 2,4-dichloro-5-chlorosulfonyl benzoic acid (100 mg, 0.35 mmol) in benzene (5 mL).
2,4-Dichloro-5-[hexamethyleneiminosulfonyl]benzoic acid (white solid, quantitative yield) from hexamethyleneimine (170 mg, 1.71 mmol) and 2,4-dichloro-5-chlorosulfonyl benzoic acid (100 mg, 0.35 mmol) in benzene (5 mL).
2-Chloro-5-[piperidinosulfonyl]benzoic acid (1.8 g, beige solid, 51% yield) from piperidine (3.0 g, 35.3 mmol) and 2,4-dichloro-5-chlorosulfonyl benzoic acid (3.0g, 11.76 mmol) in THF (15 mL).
2-Hydroxy-5-[piperidinosulfonyl]benzoic acid (1.02 g, off-white solid, 84% yield) from piperidine (1.44 g, 16.9 mmol) and 2-hydroxy-5-chlorosulfonyl benzoic acid (1.0g, 4.23 mmol) in THF (15 mL).
2,4-Dichloro-5-[4-tertbutyloxycarbonylpiperazinosulfonyl]benzoic acid (1.93g, white solid, 64% yield) from 4-tertbutyloxycarbonylpiperazine (1.93 g, 0.6 mmol) and 2,4-dichloro-5-chlorosulfonyl benzoic acid (2.0g, 3.45 mmol) in benzene (10 mL).
2-Chloro-5-(morpholine-4-sulphonyl)benzoic acid (42 mg, colourless oil, 70% yield) from morpholine (68 mg, 0.784 mmol) and 2-chloro-5-chlorosulphonyl benzoic acid (50 mg, 0.196 mmol) in THF (2 mL). ¹H-NMR (CDCl₃) δ: 8.34 (s, 1H), 7.81 (dd, 1H), 7.73 (d, 1H), 3.76 (t, 4H), 3.05 (t, 4H).
5-(Butylethylsulphamoyl)-2-chlorobenzoic acid (21 mg, 34% yield) from butylethylamine (79 mg, 0.784 mmol) and 2-chloro-5-chlorosulphonylbenzoic acid (50 mg, 0.196 mmol) in THF (2 mL). ¹H-NMR (CDCl₃) δ: 8.42 (d, 1H), 7.89 (dd, 1H), 7.63 (d, 1H), 3.26 (q, 2H), 3.16 (t, 2H), 1.49-1.59 (m, 2H), 1.25-1.38 (m, 2H), 1.14 (t, 3H), 0.89 (t, 3H).
2-Chloro-5-dimethylsulphamoylbenzoic acid (27.5 mg, 53% yield) from dimethylamine (0.4 mL of 2.0M solution in THF, 0.784 mmol) and 2-chloro-5-chlorosulphonylbenzoic acid (50 mg, 0.196 mmol) in THF (1 mL). ¹H-NMR. (CDCl₃) δ: 8.39 (d, 1H), 7.87 (dd, 1H), 7.68 (d, 1H), 2.76 (s, 6H).
2-Chloro-5-(octahydropyrido[1,2-a]pyrazine-2-sulphonyl)benzoic acid (71.9 mg, yellow oil, 102% yield) from octahydropyrido[1,2-a]pyrazine (110 mg, 0.784 mmol) and 2-chloro-5-chlorosulphonylbenzoic acid (50 mg, 0.196 mmol) in THF (1.5 mL).
2-Chloro-5-(4-methylpiperidine-1-sulphonyl)benzoic acid (28.4 mg, white solid, 46% yield) from 4-methylpiperidine (78 mg, 0.784 mmol) and 2-chloro-5-chlorosulphonylbenzoic acid (50 mg, 0.196 mmol) in THF (1.5 mL). ¹H-NMR (CDCl₃) δ: 8.35 (d, 1H), 7.84 (dd, 1H), 7.65 (d, 1H), 3.77 (d, 2H), 2.30 (t, 2H), 1.69 (d, 2H), 1.25-1.30 (m, 3H), 0.91-0.96 (m, 3H).
2-Chloro-5-(2,5-dimethylpyrollidine-1-sulphonyl)benzoic acid (37 mg, yellow gum, 60% yield) from 2,5-dimethylpyrollidine (78 mg, 0.784 mmol) and 2-chloro-5-chlorosulphonylbenzoic acid (50 mg, 0.196 mmol) in THF (1.5 mL). ¹H-NMR (CDCl₃) δ: 8.27 (s, 1H), 7.79 (d, 1H), 7.55 (d, 1H), 3.62 (s, 3H), 1.52-1.57 (m, 3H), 1.29 (d, 6H).
3-(Piperidine-1-sulphonyl)benzoic acid (562 mg, beige solid, 46%) from 2-chlorosulphonylbenzoic acid (1.0 g, 4.53 mmol) and piperidine (1.54 g, 18.13 mmol) in benzene (20 mL).
2-Chloro-4-fluoro-5-(piperidine-1-sulphonyl)benzoic acid (3.2 g, light yellow solid, 90%) from 2-chloro-5-sulphonyl-4-fluorobenzoic acid (3.0 g, 11.0 mmol) and piperidine (3.75 g, 44.0 mmol) in THF (40 mL).
2-Methyl-5-(piperidine-1-sulfonyl)benzoic acid (400 mg, a white solid, 67%) from 2-methyl-5-chlorosulphonylbenzoic acid (500 mg, 2.1 mmol) and piperidine (360 mg, 4.22 mmol).
2-Fluoro-5-(piperidine-1-sulfonyl)benzoic acid (495 mg, a white solid, 82%) from 2-fluoro-5-chlorosulphonylbenzoic acid (500 mg, 2.1 mmol) and piperidine (358 mg, 4.2 mmol).
2-Chloro-5-(thiomorpholine-4-sulfonyl)-benzoic acid (3.1 g, 50%) from 2-chlorosulphonylbenzoic acid (5.0 g, 19.6 mmol) and thiomorpholine (2.23 g, 21.6 mmol).

### Synthesis of 2-Methoxy-5-[piperidinosulfonyl]benzoic Acid

To a stirred solution of 2-hydroxy-5-[piperidinosulfonyl]benzoic acid (1.0 g, 3.5 mmol) in DMF (10 mL) was added NaOH (500 mg, 12.5 mmol) and the mixture was heated to 60°C for 0.5 h. To the resulting white solid suspension was added MeI (1.5 g, 10.5 mmol) and stirring was continued overnight. The mixture was then diluted with water and extracted with ethyl acetate. The organic layer was washed with brine and dried over MgSO₄ (anhydrous). The organic solvent was then removed *in vacuo* to afford the product as a yellow oil (1.0g, 91 % yield). The ester was then dissolved in a THF/MeOH/ H₂O (10mL / 5mL / 2 mL) mixture, LiOH (270 mg, 6.4 mmol) was added and the mixture was heated to 90°C for 0.5 h. Upon cooling, the mixture was concentrated *in vacuo* and the pH of the resulting solution was adjusted to pH 3. The off-white precipitate obtained was filtered, collected and dried giving 842 mg or 88% yield.

### General Synthesis of (Alkylaminosulphonyl)phenylmethanols

To a solution of a benzoic acid in anhydrous CH₂Cl₂ was added triethylamine (1.5 eq.) and isobutyl chloroformate (1.5 eq.) at 0°C. After 2 hours the reaction mixture was concentrated. The residue was triturated with anhydrous THF and filtered. The filtrate was chilled in an ice bath and a solution of NaBH₄ (5 eq.) in water was added dropwise. The reaction was slowly allowed to warm to room temperature. After 16 hours the reaction was quenched with 6M HCl and basified with saturated NaHCO₃. This was partitioned between ether and saturated NaHCO₃. The organic phase was washed with brine, dried (NaₛSO₄), filtered, and concentrated. The (alkylaminosulphonyl)phenylmethanols were isolated by column chromatography.

The following (alkylaminosulfonyl)phenylmethanols were synthesized using the above procedure.
(2-chloro-5-(piperidine-1-sulphonyl)phenyl)methanol (0.66 g, 69% yield) from 2-chloro-5-(piperidine-1-sulphonyl)benzoic acid (1.00 g, 3.29 mmol).
[2-Chloro-5-(thiomorpholine-4-sulfonyl)-phenyl]-methanol (900 mg, 29% yield) from 2-Chloro-5-(thiomorpholine-4-sulfonyl)-benzoic acid (3.14 g, 9.77 mmol).

### Synthesis of 1-(3-bromomethyl-4-chloro-benzenesulphonyl)-piperidine

To a solution of (2-chloro-5-(piperidine-1-sulphonyl)phenyl)methanol (103 mg, 0.355 mmol) dissolved in anhydrous dichloromethane (3 mL) was added carbontetrabromide (130 mg, 0.391 mmol). The reaction mixture was cooled to 0°C and triphenylphosphine (103 mg, 0.391 mmol) was added. The reaction was warmed to room temperature over 2 hours. It was poured into brine and diluted with dichloromethane. The organic was dried over sodium sulphate and the solvent was removed *in vacuo.* Eluting through a solid phase extraction tube (5g) with 20% ethyl acetate/hexanes afforded 1-(3-bromomethyl-4-chloro-benzenesulphonyl)-piperidine (135 mg, 108%) as a white solid. ¹H-NMR (CDCl₃) δ: 7.84 (dd, 1H) 7.62 (dd, 1H), 7.53-7.57 (m, 1H) 4.73 (s, 1H), 4.60 (s, 1H), 3.00 (s, 4H), 1.2-1.69 (m, 4H), 1.44-1.48 (m, 2H).

### General Synthesis of 2-Oxo-2-phenethyl Alkylaminosulfonylbenzoates

### Method A

To a solution of an appropriate alkylaminosulfonylbenzoic acid (1 eqv.) in DMF was added K₂CO₃ (2 eqv) and the mixture was heated with a heat gun for 1 minute. Bromoacetophenone (1eqv) was then added and the mixture was heated for a further minute. The reaction was allowed to cool to room temperature and diluted with ethyl acetate and washed with brine, dried over MgSO4 (anhydrous). The organic solvent was removed in *vacuo* and the product was triturated with hexane to afford the product after filtration.

### Example 1

### 2,4-Dichloro-5-(piperidine-1-sulfonyl)-benzoic acid 2-oxo-2-phenyl-ethyl ester (170 mg, white solid, 85 % yield)

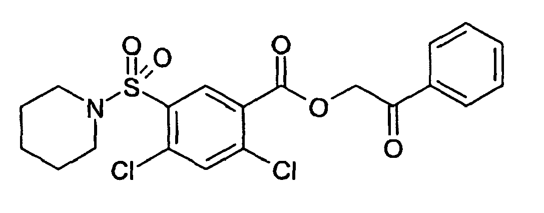

¹H-NMR (CDCl₃) δ: 8.70 (s, 1H), 7.95 (d, 2H), 7.67 (s, 1H), 7.63 (t, 1H), 7.52 (t, 2H), 5.60 (s, 2H), 3.30 (t, 4H), 1.60 (m, 6H).

### Example 2

2,4-Dichloro-5-(pyrrolidine-1-sulfonyl)-benzoic acid 2-oxo-2-phenyl-ethyl ester (12 mg, white solid, 11 % yield)

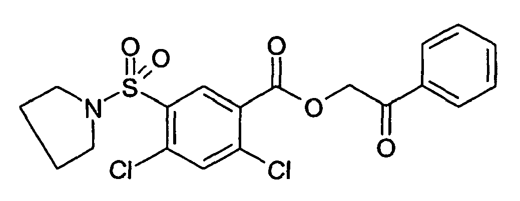

¹H-NMR (CDCl₃) δ: 8.73 (s, 1H), 7.95 (d, 2H), 7.68 (s, 1H), 7.63 (t, 1H), 7.52 (t, 2H), 5.61 (s, 2H), 3.45 (t, 4H), 1.94 (t, 4H).

### Example 3

### 5-(Azetidine-1-sulfonyl)-2,4-dichloro-benzoic acid 2-oxo-2-phenyl-ethyl ester (34 mg, white solid, 32 % yield)

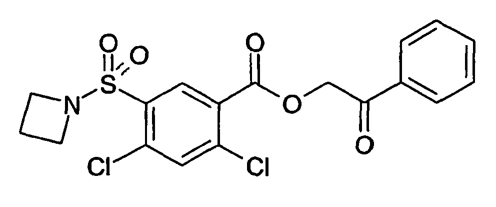

¹H-NMR (CDCl₃) δ: 8.69 (s, 1H), 7.95 (d, 2H), 7.69 (s, 1H), 7.64 (t, 1H), 7.52 (t, 2H), 5.61 (s, 2H), 4.10 (t, 4H), 2.26 (m, 2H).

### Example 4

### 2,4-Dichloro-5-(thiomorpholine-4-sulfonyl)benzoic acid 2-oxo-2-phenylethyl ester (82 mg, white solid, 49 % yield)

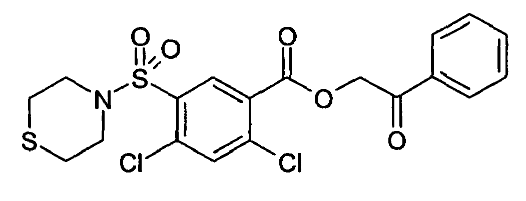

¹H-NMR (CDCl₃) δ: 8.71 (s, 1H), 7.95 (d, 2H), 7.67 (s, 1H), 7.63 (t, 1H), 7.52 (t, 2H), 5.60 (s, 2H), 3.60 (t, 4H), 2.70 (t, 4H).

### Example 5

### 2,4-Dichloro-5-diethylsulfamoylbenzoic acid 2-oxo-2-phenylethyl ester (19.5 mg, white solid, 13 % yield)

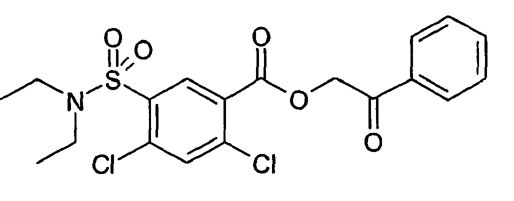

¹H-NMR (CDCl₃) δ: 8.72 (s, 1H), 7.95 (d, 2H), 7.67 (s, 1H), 7.65 (t, 1H), 7.52 (t, 2H), 5.61 (s, 2H), 3.40 (q, 4H), 1.10 (t, 6H).

### Example 6

### 5-(Azepane-1-sulfonyl)-2,4-dichlorobenzoic acid 2-oxo-2-phenylethyl ester (44 mg, white solid, 27 % yield)

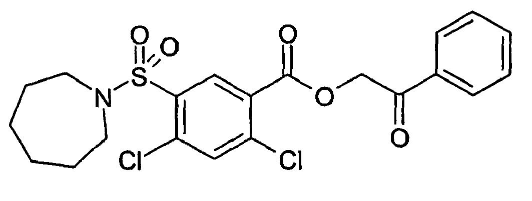

¹H-NMR (CDCl₃) δ: 8.70 (s, 1H), 7.95 (d, 2H), 7.67 (s, 1H), 7.64 (t, 1H), 7.52 (t, 2H), 5.62 (s, 2H), 3.42 (t, 4H), 1.80 (m, 4H), 1.68 (m, 4H).

### Example 7

### 2-Methoxy-5-(piperidine-1-sulfonyl)benzoic acid 2-oxo-2-phenylethyl ester (41 mg, off- white solid, 59 % yield)

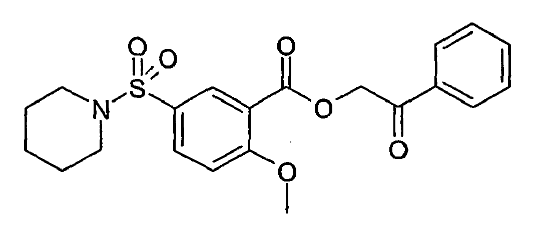

¹H-NMR (CDCl₃) δ: 8.36 (s, 1H), 7.96 (d, 2H), 7.89 (dd, 1H), 7.63 (t, 1H), 7.52 (t, 2H), 7.10 (d, 1H), 5.60 (s, 2H), 3.99 (s, 3H), 3.10 (t, 4H), 1.65 (m, 4H), 1.43 (m, 2H).

### Example 8

### 2-Chloro-5-(piperidine-1-sulfonyl)benzoic acid 2-oxo-2-phenylethyl ester (60 mg, white solid, 60 % yield)

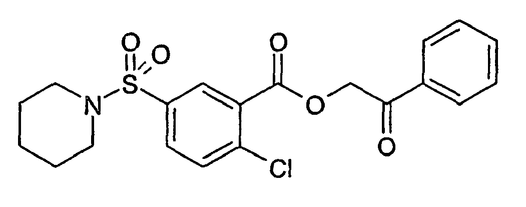

¹H-NMR (CDCl₃) δ: 8.40 (s, 1H), 7.96 (d, 2H), 7.82 (dd, 1H), 7.65 (m, 1H), 7.53 (t, 2H), 7.10 (d, 1H), 5.62 (s, 2H), 3.04 (t, 4H), 1.65 (m, 4H), 1.46 (m, 2H).

### Example 9

### 2-Chloro-4-fluoro-5-(piperidine-1-sulphonyl)benzoic acid 2-oxo-phenylethyl ester (19 mg, yellow oil, 56% yield)

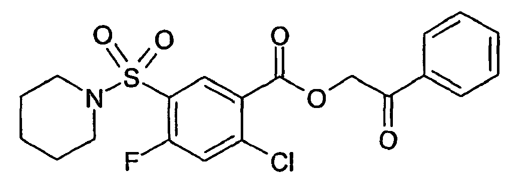

¹H-NMR (CDCl₃) δ: 8.56 (d, 1H), 8.45 (d, 2H), 7.64 (t, 1H), 7.52 (t, 2H), 7.38 (d, 1H), 5.60 (s, 2H), 3.21 (t, 4H), 1.54-1.66 (m, 4H), 1.51-1.53 (m, 2H)..

### Example 10

### 2-Chloro-5-(piperidine-1-sulfonyl)benzoic acid 2-(2,5-dimethoxyphenyl)-2-oxoethyl ester (49 mg, white solid, 100 % yield)

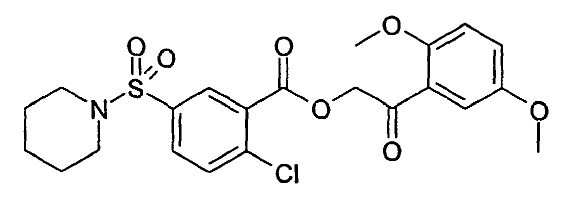

¹H-NMR (CDCl₃) δ: 8.38 (s, 1H), 7.80 (dd, 1H), 7.64 (d, 1H), 7.49 (d, 1H), 7.13 (dd, 1H), 6.97 (d, 1H), 5.51 (s, 2H), 3.95 (s, 3H), 3.80 (s, 3H), 3.03 (t, 4H), 1.64 (m, 4H), 1.45 (m, 2H).

### Example 11

### 2-Chloro-5-(piperidine-1-sulfonyl)benzoic acid 2-(3-methoxyphenyl)-2-oxoethyl ester (28 mg, colourless oil, 62 % yield)

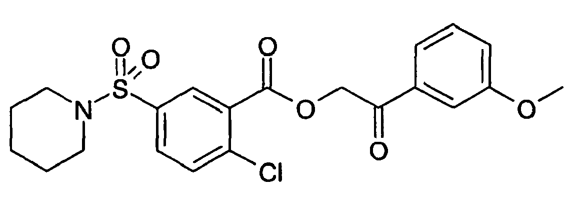

¹H-NMR (CDCl₃) δ: 8.38 (s, 1H), 7.82 (dd, 1H), 7.64 (d, 1H), 7.50 (m, 2H), 7.42 (t, 1H) 7.17 (dd, 1H), 5.60 (s, 2H), 3.86 (s, 3H), 3.03 (t, 4H), 1.64 (m, 4H), 1.45 (m, 2H).

### Example 12

### 2-Chloro-5-(piperidine-1-sulfonyl)benzoic acid 2-(3-chlorophenyl)-2-oxoethyl ester (28 mg, colourless oil, 62 % yield)

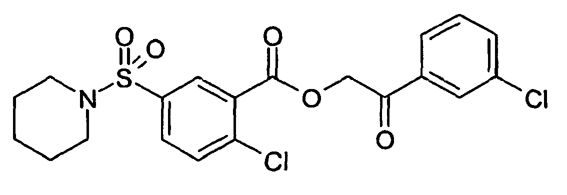

¹H-NMR (CDCl₃) δ: 8.38 (s, 1H), 7.92 (d, 1H), 7.82 (m, 2H), 7.60 (m, 2H), 7.45 (m, 1H), 5.57 (s, 2H), 3.03 (t, 4H), 1.64 (m, 4H), 1.45 (m, 2H).

### Example 13

### 2-Methyl-5-(piperidine-1-sulfonyl)benzoic acid 2-oxo-2-phenylethyl ester (48 mg, a white solid, 68 % yield)

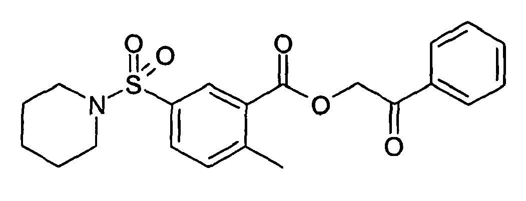

¹H-NMR (CDCl₃) δ: 8.41 (s, 1H), 7.95 (d, 2H), 7.80 (m, 1H), 7.64 (m, 1H), 7.52 (m, 2H), 7.44 (d, 1H), 5.60 (s, 2H), 3.01 (t, 4H), 2.72 (s, 3H), 1.64 (m, 4H), 1.45 (m, 2H).

### Example 14

### 2-Fluoro-5-(piperidine-1-sulfonyl)benzoic acid 2-oxo-2-phenylethyl ester (45 mg, a white solid, 62 % yield)

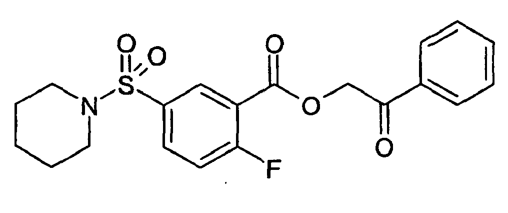

¹H-NMR (CDCl₃) δ: 8.45 (dd, 1H), 7.96 (m, 3H), 7.62 (m, 1H), 7.52 (m, 2H), 7.34 (t, 1H), 5.63 (s, 2H), 3.02 (t, 4H), 1.66 (m, 4H), 1.45 (m, 2H).

### Example 15

### 2-Chloro-5-(piperidine-1-sulfonyl)benzoic acid 2-oxo-2-pyridin-2-ylethyl ester (28 mg, colourless oil, 62 % yield)

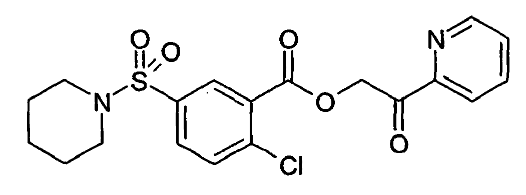

¹H-NMR (CDCl₃) δ: 8.70 (d, 1H), 8.40 (s, 1H), 7.96 (d, 1H), 7.88 (dd, 1H), 7.82 (dd, 1H), 7.65 (d, 1H), 7.55 (m, 1H), 5.90 (s, 2H), 3.03 (t, 4H), 1.64 (m, 4H), 1.45 (m, 2H).

### Example 16

### 2-Chloro-5-(piperidine-1-sulphonyl)benzoic acid 1-methyl-2-oxo-2-phenylethyl ester (58 mg, colourless oil, 83% yield)

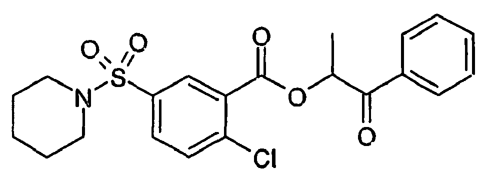

¹H-NMR (CDCl₃) δ: 8.28 (d, 1H), 7.98 (d, 2H), 7.79 (dd, 1H), 7.60 (dd, 2H), 7.50 (collapsed dd, 2H), 6.22 (q, 1H), 3.00 (t, 4H), 1.61-1.69 (m, 7H), 1.40-1.47 (m, 2H).

### Example 17

### 2-Chloro-5-(piperidine-1-sulphonyl)benzoic acid 2-(4-fluorophenyl)-2-oxoethyl ester (23 mg, yellow oil, 64% yield)

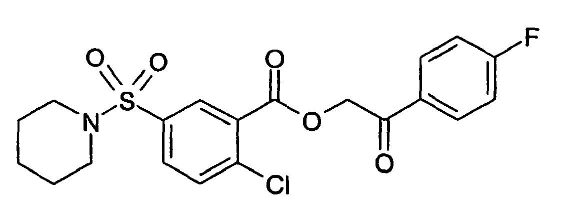

¹H-NMR (CDCl₃) δ: 8.39 (d, 1H), 7.93-8.03 (m, 2H), 7.64 (d, 1H), 7.31 (d, 2H), 5.59 (s, 2H), 3.03 (t, 4H), 2.43 (s, 1H), 1.62-1.69 (m, 4H), 1.38-1.48 (m, 2H).

### Example 18

### 2-Chloro-5-(piperidine-1-sulphonyl)benzoic acid 2-oxo-2-p-tolylethyl ester (25.4 mg, pale yellow solid, 71% yield)

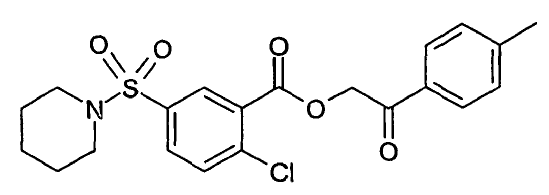

¹H-NMR (CDCl₃) δ: 8.39 (s, 1H), 7.80-7.86 (m, 2H), 7.82 (d, 1H), 7.65 (d, 2H), 7.15-7.23 (m, 2H), 5.58 (s, 2H), 3.03 (t, 4H), 1.61-1.70 (m, 4H), 1.45-1.49 (m, 2H).

### Example 19

### 2-Chloro-5-(piperidine-1-sulphonyl)benzoic acid 2-oxobutyl ester (18 mg, colourless oil, 58% yield)

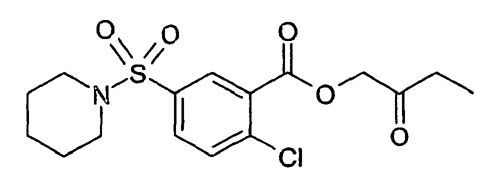

¹H-NMR (CDCl₃) δ: 8.31 (d, 1H), 7.81 (dd, 1H), 7.63 (d, 2H), 4.94 (s, 2H), 3.01 (t, 4H), 2.53 (q, 2H), 1.61-1.69(m, 4H), 1.44-1.48 (m, 2H), 1.14 (t, 3H).

### Example 20

### 2-Chloro-5-(piperidine-1-sulphonyl)benzoic acid 2-naphthalen-2-yl-2-oxoethyl ester (26 mg, yellow oil, 67% yield)

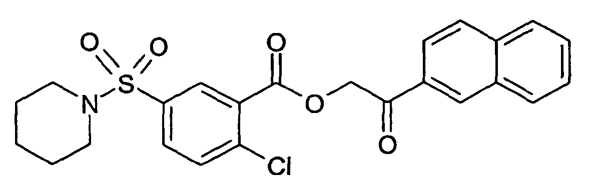

¹H-NMR (CDCl₃) δ: 8.48 (s, 1H), 8.42 (d, 1H), 7.89-8.02 (m, 4H), 7.82 (dd, 1H), 7.57-7.66 (m, 3H), 5.75 (s, 2H), 3.04 (t, 4H), 1.62-1.70 (m, 4H), 1.39-1.49 (m, 2H).

### Example 21

### 2-Chloro-5-(piperidine-1-sulphonyl)benzoic acid 2-benzo[b]thiophen-3-yl-2-oxoethyl ester (23 mg, yellow oil, 59% yield)

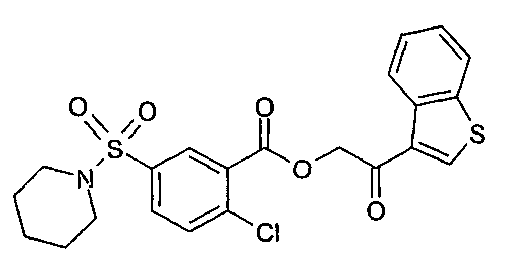

¹H-NMR (CDCl₃) δ: 8.70 (d, 1H), 8.43 (s, 1H), 8.38 (s, 1H), 7.89 (d, 1H), 7.83 (dd, 1H), 7.65 (d, 1H), 7.43-7.54 (m, 2H), 5.59 (s, 2H), 3.05 (t, 4H), 1.61-1.71 (m, 4H), 1.39-1.50 (m, 2H).

### Example 22

### 2-Chloro-5-(piperidine-1-sulphonyl)benzoic acid 3,3-dimethyl-2-oxobutyl ester (25 mg, colourless oil, 83% yield)

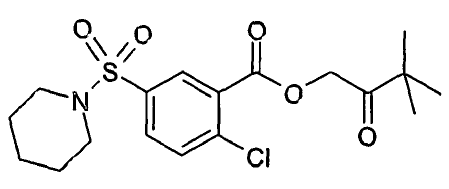

¹H-NMR (CDCl₃) δ: 8.33 (s, 1H), 7.79 (dd, 1H), 7.62 (d, 1H), 5.15 (s, 2H), 3.02 (t, 4H), 1.61-1.69 (m, 4H), 1.44-1.48 (m, 2H), 1.26 (s, 9H).

### Example 23

### 2-Chloro-5-(morpholine-4-sulphonyl)benzoic acid 2-oxo-2-phenylethyl ester (44 mg, white solid, 76% yield)

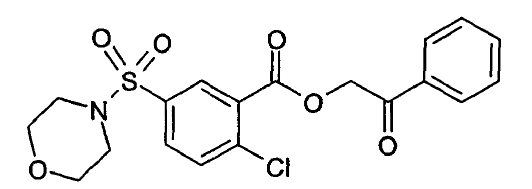

¹H-NMR (CDCl₃) δ: 8.41 (d, 1H), 7.96 (d, 2H), 7.83 (dd, 1H), 7.62-7.70 (m, 2H), 7.52 (t, 2H), 5.63 (s, 2H), 3.76 (t, 4H), 3,06 (t, 4H).

### Example 24

### 5-(Butylethylsulphamoyl)-2-chlorobenzoic acid 2-oxo-2-phenylethyl ester (26 mg, yellow oil, 90% yield)

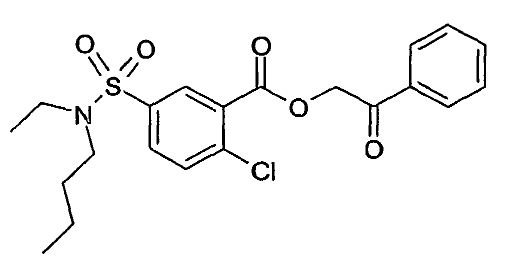

¹H-NMR (CDCl₃) δ: 8.46 (d, 1H), 7.94 (dd, 2H), 7.88 (dd, 1H), 7.60-7.67 (m, 2H), 7.52 (t, 2H), 5.61 (s, 2H), 3.26 (q, 2H), 3.18 (t, 2H), 1.50-1.60 (m, 2H), 1.25-1.39 (m, 2H), 1.15 (t, 3H), 0.92 (t, 3H).

### Example 25

### 2-Chloro-5-dimethylsulphamoylbenzoic acid 2-oxo-2-phenylethyl ester (27 mg, light yellow solid, 70% yield)

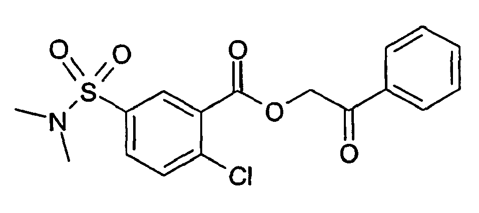

¹H-NMR (CDCl₃) δ: 8.43 (d, 1H), 7.94 (d, 2H), 7.85 (dd, 1H), 7.62-7.65 (m, 2H), 7.52 (t, 2H), 5.63 (s, 2H), 2.77 (s, 6H).

### Example 26

### 2-Chloro-5-(octahydropyrido[1,2-a]pyrazine-2-sulphonyl)benzoic acid 2-oxo-2-phenylethyl ester (45 mg, yellow oil, 48% yield)

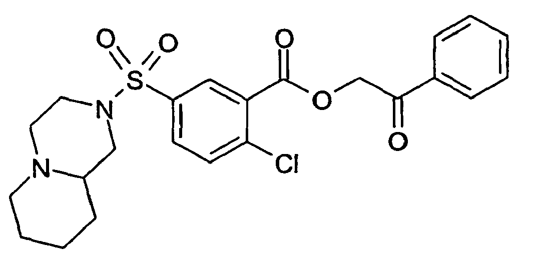

¹H-NMR (CDCl₃) δ: 8.39 (d, 1H), 7.94 (d, 2H), 7.81 (dd, 1H), 7.61 (t, 2H), 7.52 (t, 3H), 5.61 (s, 2H), 3.69 (d, 1H), 3.55 (d, 1H), 2.74-2.80 (m, 2H), 2.56 (dt, 1H), 2.34 (dt, 1H), 2.00-2.15 (m, 3H), 1.52-1.76 (m, 4H), 1.10-1.43 (m, 3H).

### Example 27

### 2-Chloro-5-(4-methylpiperidine-1-sulphonyl)benzoic acid 2-oxo-2-phenylethyl ester (38 mg, pale yellow solid, 100% yield)

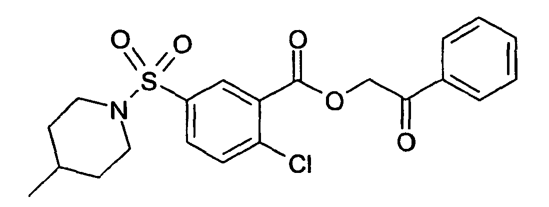

¹H-NMR (CDCl₃) δ: 8.39 (d, 1H), 7.95 (dd, 2H), 7.82 (dd, 1H), 7.61-7.67 (m, 2H), 7.50 (t, 2H), 5.62 (s, 2H), 3.77 (d, 2H), 2.31 (t, 2H), 1.69 (d, 2H), 1.22-1.32 (m, 3H), 0.88-0.93 (m, 3H).

### Example 28

### 2-Chloro-5-(2,5-dimethylpyrollidine-1-sulphonyl)benzoic acid 2-oxo-2-phenylethyl ester (37 mg, pale yellow solid, 99% yield)

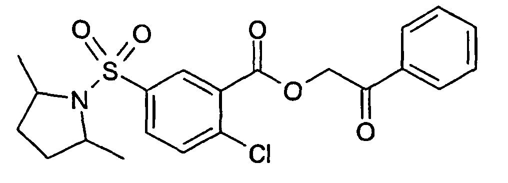

¹H-NMR (CDCl₃) δ: 8.48 (s, 1H), 7.89-7.97 (m, 3H), 7.57-7.67 (m, 2H), 7.52 (t, 2H), 5.62 (s, 2H), 3.69-3.74 (m, 2H), 1.54-1.72 (m, 5H), 1.36 (d, 7H).

### Example 29

### 3-(Piperidine-1-sulphonyl)benzoic acid 2-oxo-2-phenylethyl ester (82 mg, yellow oil, 86% yield)

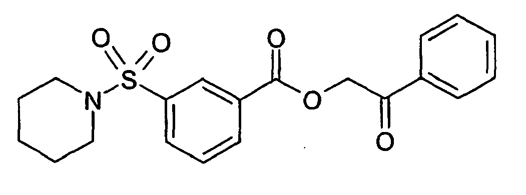

¹H-NMR (CDCl₃) δ: 8.48 (s, 1H), 8.33 (d, 1H), 7.94-7.99 (m, 3H), 7.61-7.68 (m, 2H), 7.51 (t, 2H), 5.62 (s, 2H), 3.01 (t, 4H), 1.61-1.68 (m, 4H), 1.38-1.46 (m, 2H).

### Method B

To a mixture of the alkylaminosulfonylbenzoic acids in CH₂Cl₂ was added oxalyl chloride followed by DMF (one drop). The mixture was stirred at room temperature for 5 h. The solvent was then removed *in vacuo* and the residue was taken up into CH₂Cl₂ followed by the addition of amines or alcohol. The mixture was stirred at room temperature for 1 h. The reaction mixture was concentrated onto silica and purified by flash column chromatography to afford the amides and esters.

### Example 30

### N-2-Oxo-2-phenethyl 2,4-dichloro-5-[piperidinosulfonyl] benzamide (40 mg, white solid, 49 % yield)

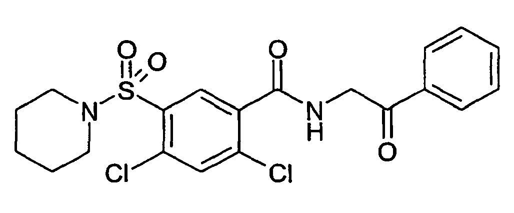

¹H-NMR (CDCl₃) δ: 8.36 (s, 1H), 7.99 (d, 2H), 7.66 (m, 1H), 7.61 (s, 1H), 7.53 (t, 2H), 7.45 (br, t, 1H, -NH), 4.97 (d, 2H), 3.27 (t, 4H), 1.58 (m, 6H).

### Example 31

### 2-Chloro-4-fluoro-N-(2-oxo-2-phenylethyl)-5-(piperidine-1-sulphonyl) benzamide (30.4 mg, white solid, 41% yield)

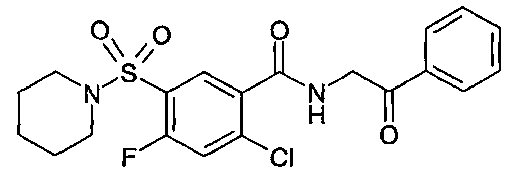

¹H-NMR (CDCl₃) δ: S.19 (d, 1H), 8.01 (d, 2H), 7.66 (t, 1H), 7.53 (t, 2H), 7.42 (s, 1H), 7.29 (d, 1H), 4.97 (d, 2H), 3.18 (t, 4H), 1.61-1.68 (m, 4H), 1.27-1.42 (m, 2H).

### Preparation of Piperazine HCl Salts

### Example 32

### 2-Oxo-2-phenethyl 2,4-dichloro-5-[piperazinosulfonyl] benzoate hydrochloride

A mixture of the 2-oxo-2-phenethyl 2,4-dichloro-5-[4-tertbutyloxycarbonyl piperazinosulfonyl] benzoate in TFA/CH₂Cl₂ (1:1) was stirred at room temperature for 1 h after which NaHCO₃ was added. The neutralized mixture was extracted with CH₂Cl₂ and concentrated *in vacuo.* The result was then dissolved in CH₂Cl₂ (2mL) followed by HCl/ether (1M solution in ether) giving 51 mg of the HCl salt (74% yield).

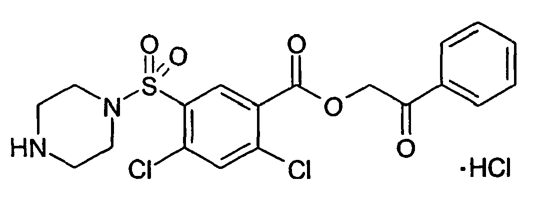

¹H-NMR (CDCl₃/CD₃OD) δ: 8.70 (s, 1H), 7.94 (d, 2H), 7.69 (s, 1H), 7.64 (t, 1H), 7.52 (t, 2H), 5.62 (s, 2H), 3.75 (br, m, 4H), 3.32 (br, m, 4H).

### Example 33

### 2-Oxo-2-phenethyl 2,4-dichloro-5-[4-methyl-piperazinosulfonyl] benzoate hydrochloride

To a mixture of the 2,4-dichloro-5-[piperazinosulfonyl] benzoate hydrochloride in formic acid was added formaldehyde. The mixture was then stirred at room temperature for 1 h followed by heating at 120°C for 4 h. The reaction mixture was then cooled and neutralized with saturated NaHCO₃. The neutralized mixture was then extracted with CH₂Cl₂, wash with brine and dried over Na₂SO₄ (anhydrous). The organic layer was treated with HCl/ether (1M solution in ether) and concentrated *in vacuo* to afford the product as a white solid (25 mg, 11% overall yield from benzoic acid derivative).

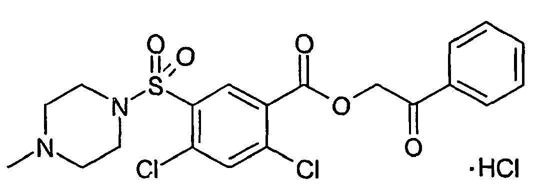

¹H-NMR (CDCl₃/CD₃OD) δ: 8.70 (s, 1H), 7.97 (d, 2H), 7.70 (s, 1H), 7.64 (t, 1H), 7.52 (t, 2H), 5.61 (s, 2H), 3.95 (br, m, 4H), 2.93 (s, 3H), 1.70 (br, m, 4H).

### Example 34

### Synthesis of 2,4-dichloro-5-[piperidiinosulfonyl] benzyloxyacetophenone

To a vigorously stirred solution of the 2,4-dichloro-5-[piperidinosulfonyl]benzoic acid (200 mg, 0.6 mmol) in THF (10 mL) was added isobutylchloroformate (121 mg, 0.89 mmol) followed by Et₃N (1 mL). The reaction mixture was stirred at room temperature for 1h after which the solvent was removed *in vacuo.* THF (5 mL) was then added followed by the addition of NaBH₄ (114 mg, 3.0 mmol, dissolved in 2 mL of H₂O). The reaction was stirred for a further 1 h and then quenched by the addition of methanol. The mixture was extracted with ethyl acetate, and the organic layer was washed with brine, and dried over MgSO₄. The solvent was removed *in vacuo* and the residue was purified by flash chromatography giving 83 mg of a colourless oil (43% yield). The alcohol obtained was alkylated according to method A below to afford the corresponding ether (see below).

### 2,4-dichloro-5-[piperidiinosulfonyl] benzyloxyacetophenone (6 mg, off-white solid, 5% yield)

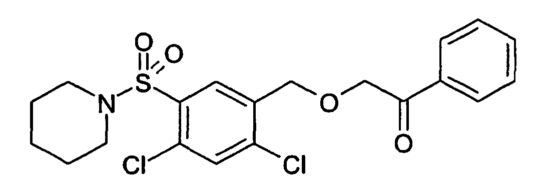

¹H-NMR (CDCl₃) δ: 8.25 (s, 1H), 7.93 (d, 2H), 7.64 (t, 1H), 7.55 (s, 1H), 7.52 (m, 2H) 4.90 (s, 2H), 4.75 (s, 2H), 3.25 (t, 4H), 1.60 (m, 6H). ,

### Example 35

### Synthesis of 2-Chloro-5-(piperidine-l-sulphonyl)benzoic Acid 1,1-Dimethyl-2-oxo-2-phenylethyl Ester

To a solution of 2-chloro-5-(piperidine-1-sulphonyl)benzoyl chloride (80 mg, 0.248 mmol) in anhydrous toluene (2 mL) was added triethylamine (38 µL, 28 mg, 0.273 mmol), polystyrene-supported DMAP (17 mg of 1.46 mmol/g, 0.0248 mmol), and 2-hydroxy-2-methyl-1-phenylpropan-1-one (38 µL, 41 mg, 0.248 mmol). The mixture was heated to reflux. After 16 hours the cooled reaction mixture was filtered. The resin was washed with CH₂Cl₂. The filtrate was partitioned between saturated NaHCO₃ and CH₂Cl₂. The organic phase was dried (MgSO₄), filtered, and concentrated. Column chromatography (20% EtOAc/hexanes) provided 2-chloro-5-(piperidine-1-sulphonyl)benzoic acid 1,1-dimethyl-2-oxo-2-phenylethyl ester (20 mg, 18%) as a colourless oil.

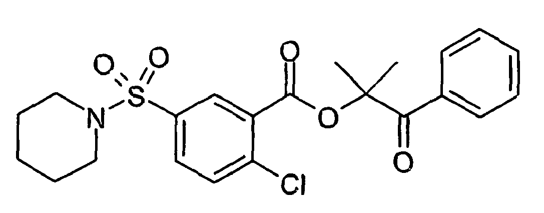

¹H NMR (CDCl₃) δ: 8.02 (d, 2H), 7.85 (d, 1H), 7.74 (dd, 1H), 7.56 (d, 1H), 7.50 (collapsed dd, 1H), 7.39 (collapsed dd, 2H), 2.92-2.96 (t, 4H), 1.89 (s, 6H), 1.58-1.64 (m, 4H), 1.43-1.45 (m, 2H).

### Example 36

### Synthesis of [2-Chloro-5-(piperidine-1-sulphonyl)-benzylamino]-acetic acid tert-butyl ester

### Method A

To a solution of alkylaminosulphonylphenylmethylbromide (50 mg, 0.142 mmol) in acentonitrile (3 mL) was added aminoacetic acid tert-butyl ester (36 mg, 0.213 mmol) and cesium carbonate (231 mg, 0.710 mmol). The reaction was allowed to stir for 1 hour before it was poured into water and extracted with ethyl acetate. The organic layer was washed with brine, dried over sodium sulphate and the solvent was removed *in vacuo.* Eluting through a solid phase extraction tube (5g) with 5% ethyl acetate/hexanes afforded [2-chloro-5-(piperidine-1-sulphonyl)benzylamino]acetic acid tert-butyl ester (17.5 mg, 31%).

### Method B

To a solution of 2-chloro-5-(piperidine-1-sulfonyl)benzaldehyde (80 mg, 0.28 mmol) and NaOAc (23 mg, 0.28 mmol) in MeOH (5 mL) was added glycine tert-butyl ester hydrochloride (113 mg, 0.67 mmol). The mixture was stirred at room temperature for 15 minutes after which NaBH₃CN (1 1 mg, 0.17 mmol) was added, and the resulting mixture was stirred overnight. The mixture was diluted with ethyl acetate and then washed with saturated NaHCO3. The organic layer was washed with brine, dried over sodium sulphate and the solvent was removed *in vacuo.* Eluting through a solid phase extraction tube (5g) with 5% ethyl acetate/hexanes afforded [2-chloro-5-(piperidine-1-sulphonyl)benzylamino]acetic acid tert-butyl ester (63 mg, 56%).

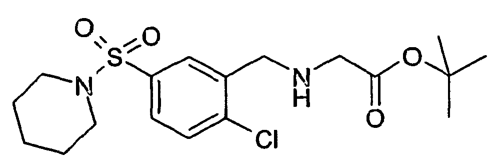

¹H-NMR (CDCl₃) δ: 7.95 (s, 1H) 7.63 (d, 1H), 7.53 (d, 1H) 4.17 (s, 2H), 3.52 (s, 2H), 2.99 (t, 4H), 1.53-1.61 (m, 4H), 1.45 (s, 9H), 1.39-1.42 (m, 2H).

### Example 37

### Synthesis of {[2-chloro-5-(piperidine-1-sulfonyl)-benzyl]-methyl-amino)-acetic acid tert-butyl ester

To a solution of alkylaminosulphonylphenylmethyl bromide (50 mg, 0.142 mmol) in DMF (5 mL) was added tert-butyl sarcosine (75 mg, 0.4 mmol) and cesium carbonate (100 mg, 0.28 mmol). The reaction was allowed to stir at room temperature overnight before it was poured into water and extracted with CH₂Cl₂. The organic layer was washed with brine, dried over MgSO₄ and the solvent was removed *in vacuo.* Eluting through a solid phase extraction tube (5g) with 5% ethyl acetate/hexanes afforded the product (21 mg, 36%)

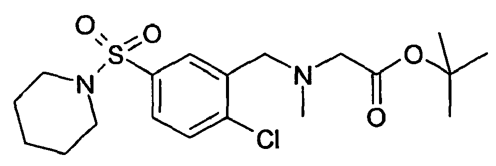

¹H-NMR (CDCl₃) δ: 7.93 (s, 1H) 7.52 (d, 1H), 7.47 (d, 1H) 3.88 (s, 2H), 3.24 (s, 2H), 2.99 (t, 4H), 2.42 (s, 3H), 1.53-1.61 (m, 4H), 1.45 (s, 9H), 1.39-1.42 (m, 2H).

### Synthesis of [2-chloro-5-(piperidine-1-sulphonyl)benzyloxy]acetic acid tert-butyl ester analogs

### Method A

### Example 38

To an ice-cold suspension of NaH (137 mg of a 60% dispersion in mineral oil, 3.42 mmol) in anhydrous THF (7 mL) was added a solution of (2-chloro-5-(piperidine-1-sulphonyl)phenyl)methanol (660 mg, 2.28 mmol) in anhydrous THF (7 mL). After 1 hour tert-butyl bromoacetate (505 µL, 667 mg, 3.42 mmol) was added. The reaction was allowed to slowly warm to room temperature. After 4 hours the reaction was quenched with saturated NH₄Cl and poured into water. This was extracted with EtOAc. The organic phase was washed with brine, dried (Na₂SO₄), filtered, and concentrated. Column chromatography (20% EtOAc/hexanes) provided (2-chloro-5-(piperidine-1-sulphonyl)benzyloxy)acetic acid tert-butyl ester (0.81 g, 88%) as a colourless oil.

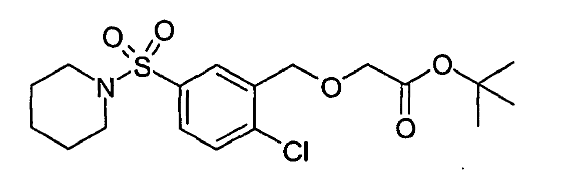

¹H NMR (CDCl₃) δ: 7.94 (s, 1H), 7.61 (dd,1H), 7.49 (d, 1H), 4.76 (s, 2H), 4.12 (s, 2H), 2.99 (t, 4H), 1.61-1.68 (m, 4H), 1.50 (s, 9H), 1.42-1.50 (m, 2H).

### Method B

### Example 39

### 2-[2-Chloro-5-(piperidine-1-sulfonyl)benzyloxy]propionic acid tert-butyl ester

To a stirred solution of (2-chloro-5-(piperidine-1-sulphonyl)phenyl)methanol (100 mg, 0.345 mmol), cesium carbonate (224.8 mg, 0.690 mmol) in anhydrous acetonitrile (6 mL) was added at 0°C a solution of 2-bromopropionic acid tert-butyl ester (144 mg, 0.690 mmol) in anhydrous acetonitrile (1 mL). The reaction was allowed to slowly warm to room temperature. After 15 min, the reaction mixture was refluxed at 100°C for 3 hours. The reaction was allowed to stir for 30 min before it was poured into water and extracted with ethyl acetate. The organic layer was washed with brine, dried over sodium sulphate and the solvent was removed *in vacuo.* After purification on silica gel with 30% ethyl acetate/hexanes afforded the title compound as viscous oil (85.5 mg, 59.9%).

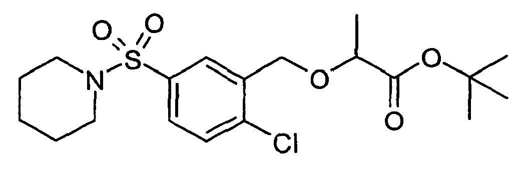

¹H NMR (CDCl₃) δ: 7.98 (s, 1H), 7.61 (dd, 1H), 7.45 (d, 1H), 4.67 (dd, 2H), 4.05 (q, 1H), 3.00 (t, 4H), 1.78-1.60 (m, 4H), 1.48 (s, 9H), 1.47 (d, 3H), 1.49-1.40 (m, 2H).

In a similar fashion the following compound was made.

### Example 40

2-[2-chloro-5-(piperidine-1-sulfonyl)benzyloxy]butyric acid tert-butyl ester was prepared (10.7 mg, 17.2 %) from tert-butyl (R)-2-hydroxybutyrate (50 mg, 0.141 mmol) and piperidinosulphonylphenylmethyl bromide (45.5 mg, 0.283 mmol).

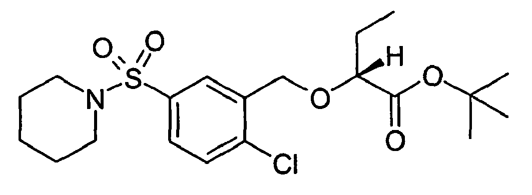

¹H NMR (CDCl₃) δ: 7.95 (s, 1H), 7.62 (dd, 1H), 7.45 (d, 1H), 4.65 (dd, 2H), 3.83 (t, 1H), 3.02 (t, 4H), 2.01-1.81 (m, 2H), 1.78-1.61 (m, 4H), 1.52 (s, 9H), 1.1.50-1.40 (m, 2H), 1.05 (t, 3H).

### Synthesis of (2-chloro-5-(piperidine-1-sulphonyl)benzyloxy)acetic acid

(2-Chloro-5-(piperidine-1-sulphonyl)benzyloxy)acetic acid tert-butyl ester (100 mg) was dissolved in 96% formic acid (1 mL). After 3 hours the reaction mixture was concentrated to provide (2-chloro-5-(piperidine-1-sulphonyl)benzyloxy)acetic acid (86 mg, 100%) as a colourless solid. ¹H NMR (CDCl₃) δ: 7.91 (s, 1H), 7.65 (dd, 1H), 7.52 (d, 1H), 4.80 (s, 2H), 4.31 (s, 2H), 3.00 (t, 4H), 1.63-1.65 (m, 4H), 1.42-1.44 (m, 2H).

### General Synthesis of 2-(2-Chloro-5-piperidine-1-sulphonyl)benzyloxyacetamides

### Method A

### Example 41

To a solution of (2-chloro-5-(piperidine-1-sulphonyl)benzyloxy)acetic acid (50 mg, 0.144 mmol) in anhydrous CH₂Cl₂ was added EDCI (30 mg, 0.158 mmol) and pyrrolidine (13 µL, 11 mg, 0.158 mmol). After 16 hours the reaction mixture was directly loaded onto a 5 g silica SPE tube and eluted with CH₂Cl₂ and then EtOAc. The EtOAc fraction was concentrated to provide 2-(2-chloro-5-(piperidine-1-sulphonyl)benzyloxy)-1-pyrrolidin-1-ylethanone (36 mg, colourless oil)

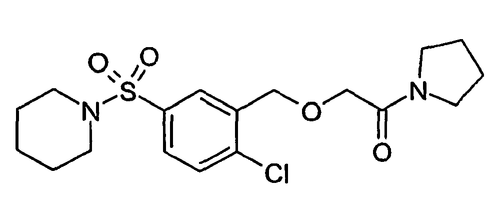

¹H NMR (CDCl₃) δ: 7.91 (d, 1H), 7.60 (dd, 1H), 7.48 (d, 1H), 4.77 (s, 2H), 4.23 (s, 2H), 3.50 (t, 2H), 3.40 (t, 2H), 2.97 (t, 4H), 1.80-2.03 (m, 4H), 1.59-1.66 (m, 4H), 1.37-1.48 (m, 2H).

In a similar fashion the following compounds (Examples 42 - 46) were prepared.

### Example 42

### 2-(2-Chloro-5-(piperidine-1-sulphonyl)benzyloxy)-N-methyl-N-phenylacetamide (51 mg, colourless oil)

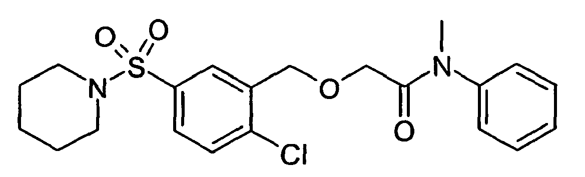

¹H NMR (CDCl₃) δ: 7.84 (s, 1H), 7.58 (dd, 1H), 7.33-7.46 (m, 4H), 7.21 (d, 2H), 4.69 (s, 2H), 4.01 (s, 2H), 3.30 (s, 3H), 2.95 (t, 4H), 1.59-1.68 (m, 4H), 1.40-1.49 (m, 2H).

### Example 43

### N-tert-Butyl-2-(2-chloro-5-(piperidine-1-sulphonyl)benzyloxy)acetamide (38 mg, colourless oil)

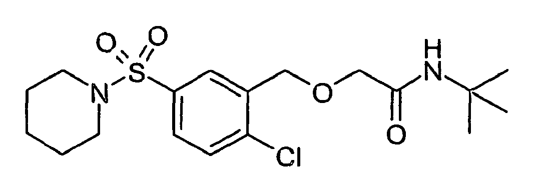

¹H NMR (CDCl₃) δ: 7.82 (d, 1H), 7.65 (dd, 1H), 7.54 (d, 1H), 6.37 (br s, 1H), 4.69 (s, 2H), 3.97 (s, 2H), 2.99 (t, 4H), 1.58-1.68 (m, 4H), 1.37-1.49 (m, 2H), 1.37 (s, 9H).

### Example 44

### (1-(2-(2-Chloro-5-piperidine-1-sulphonyl)benzyloxy)acetyl)pyrrolidin-3-yl)carbamic acid tert-butyl ester (55 mg, colourless oil)

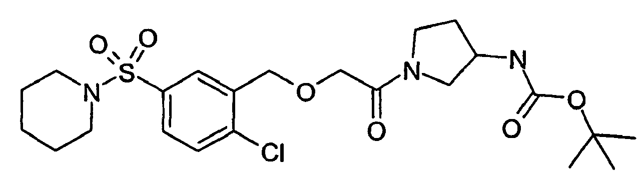

¹H NMR (CDCl₃) δ: 7.90 (s, 1H), 7.62 (d, 1H), 7.50 (d, 1H), 4.77 (d, 2H), 4.69 (br s, 1H), 4.22 (d, 2H), 4.03-4.15 (m, 1H), 3.68-3.76 (m, 1H), 3.49-3.64 (m, 2H), 3.35 (ddd, 1H), 2.98 (t, 4H), 2.11-2.23 (m, 1H), 1.76-2.04 (m, 1H), 1.60-1.67 (m, 5H), 1.47-1.51 (m, 10H).

### Example 45

### (1-(2-(2-Chloro-5-(piperidine-1-sulphonyl)benzyloxy)acetyl)piperidin-4-yl)carbamic Acid tert-butyl ester (43 mg, colourless oil)

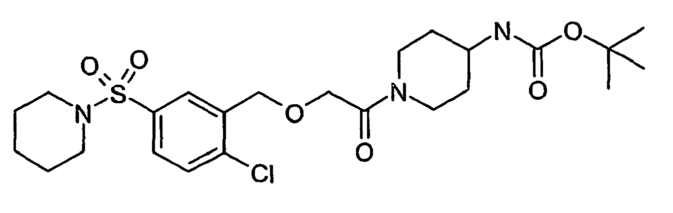

¹H NMR (CDCl₃) δ: 7.88 (d, 1H), 7.61 (dd, 1H), 7.50 (d, 1H), 4.73 (s, 2H), 4.46-4.52 (m, 2H), 4.30 (dd, 2H), 4.03-4.15 (m, 1H), 3.84 (d, 1H), 3.66 (br s, 1H), 3.10 (t, 1H), 2.98 (t, 4H), 2.77 (t, 1H), 1.98 (d, 2H), 1.60-1.66 (m, 4H), 1.43 (s, 9H), 1.25-1.43 (m, 2H).

### Example 46

### 2-[2-Chloro-5-(piperidine-1-sulfonyl)-benzyloxy]-N-pyridin-2-yl-acetamide (28 mg, white powder, 46% yield)

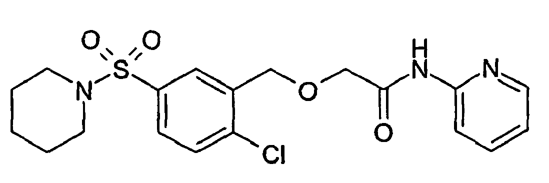

¹H NMR (CDCl₃) δ: 8.88 (br s, 1H), 8.29 (dd, 1H), 8.24 (s, 1H), 7.90 (d, 1H), 7.69-7.72 (m, 2H), 7.58 (d, 1H), 7.10 (dd, 1H), 4.82 (s, 2H), 4.26 (s, 2H), 3.03-3.07 (m, 4H), 1.64-1.72 (m, 4H), 1.25-1.30 (m, 2H).

### Method B

### Example 47

To a suspension of PS-carbodiimide (578 mg, 0.728 mmol) in anhydrous dichloromethane (8 mL) was added piperidine (24 mg, 0.287 mmol) and (2-chloro-5-(piperidine-1-sulphonyl)benzyloxy)acetic acid (25 mg, 0.0718 mmol) and stirred for 12 hours. The solvent was removed *in vacuo* and the compound was purified by eluting through a solid phase extraction tube (5g) with 5% methanol/dichloromethane to afford 2-[2-chloro-5-piperidine-1-sulphonyl)benzyloxy]-1-piperidin-1-ylethanone (17 mg, 57%)

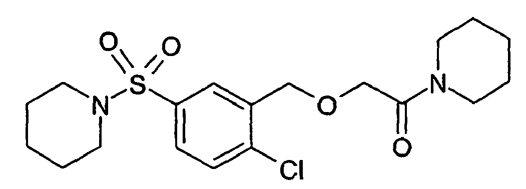

¹H-NMR (CDCl₃) δ: 7.90 (d, 1H), 7.61 (dd, 1H), 7.49 (d, 1H), 4.74 (s, 2H), 4.31 (s, 2H), 3.56 (t, 2H), 3.41 (t, 2H), 2.98 (t, 4H), 1.56-1.67 (m, 8H), 1.42-1.49 (m, 2H).

### Example 48

### Synthesis of 2-[2-Chloro-5-(piperidine-1-sulfonyl)-benzyloxyl-N-methyl-N-pyridin-2-yl-acetamide

To a suspension of NaH (11.43 mg of 60% in mineral oil, 0.286 mmol) in anhydrous THF (2 mL) at 0°C was added 2-[2-Ghloro-5-(piperidine-1-sulfonyl)-benzyloxy]-N-pyridin-2-yl-acetamide (110 mg, 0.26 mmol) in THF (4 mL). After 30 minutes iodomethane (41 mg, 0.286 mmol) was added. The mixture was stirred at room temperature for 16 hours then poured into water and extracted with EtOAc. The organic phase was washed with brine, dried (Na₂SO₄), filtered, and concentrated. Column chromatography (50% EtOAc/hexanes) provided the product as a yellow oil (44 mg, 39% yield)

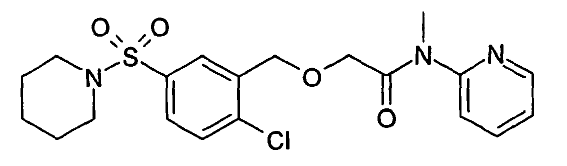

¹H-NMR (CDCl₃) δ: 8.42 (dd, 1H), 7.75-7.78 (m, 2H), 7.58 (d, 1H), 7.46 (d, 1H), 7.28 (s, 1H), 7.20 (dd, 1H), 4.71 (s, 2H), 4.42 (s, 2H), 3.42 (s, 3H), 2.93-2.99 (m, 4H), 1.61-1.68 (m, 4H), 1.39-1.46 (m, 2H).

### General Synthesis of (Alkylaminosulphonyl)benzyloxy-N-methoxy-N-methylacetamides

### Example 49

To an ice-cold suspension of NaH (185 mg of a 60% dispersion in mineral oil, 4.62 mmol) in anhydrous THF (10 mL) was added a solution of (2,4-dichloro-5-(piperidine-1-sulphonyl)phenyl)methanol (1.0 g, 3.08 mmol) in anhydrous THF (10 mL). After 1 hour a solution of 2-chloro-N-methoxy-N-methylacetamide (635 mg, 4.62 mmol) in anhydrous THF (10 mL) was added. The reaction was allowed to slowly warm to room temperature. After 2 hours the reaction was quenched with saturated NH₄Cl and poured into water. This was extracted with EtOAc. The organic phase was washed with brine, dried (Na₂SO₄), filtered, and concentrated. Column chromatography (45% EtOAc/hexanes) provided 2-(2,4-dichloro-5-(piperidine-1-sulphonyl)benzyloxy)-N-methoxy-N-methylacetamide (1.31 g, 72%) as a colourless solid

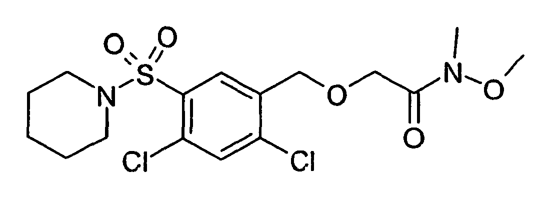

¹H NMR (CDCl₃) δ: 8.15 (s, 1H), 7.45 (s, 1H), 4.68 (s, 2H), 4.36 (s, 2H), 3.64 (s, 3H), 3.14-3.20 (m, 4H), 3.14 (s, 3H), 1.46-1.55 (m, 6H).

In a similar fashion the following compounds (Examples 50 - 56) were prepared.

### Example 50

### 2-(2-chloro-5-(piperidine-1-sulphonyl)benzyloxy)-N-methoxy-N-methylacetamide (88 mg, colourless oil, 65% yield)

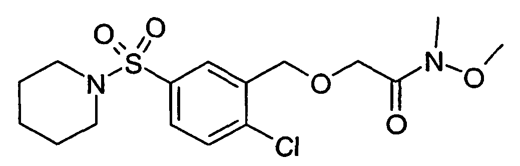

¹H NMR (CDCl₃) δ: 7.94 (d, 1H), 7.58 (dd, 1H), 7.47 (d, 1H), 4.78 (s, 2H), 4.42 (s, 2H), 3.68 (s, 3H), 3.19 (s, 3H), 2.96 (t, 4H), 1.58-1.65 (m, 4H), 1.36-1.43 (m, 2H).

### Example 51

### N-methoxy-2-[2-methoxy-5-(piperidine-1-sulphonyl)-benzyloxy)-N-methyl-acetamide (279 mg, white solid, 52% yield)

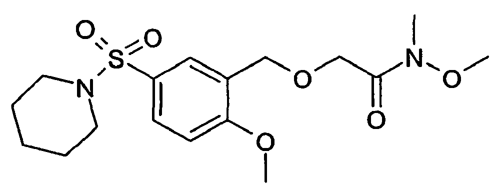

¹H NMR (CDCl₃) δ: 7.72 (s, 1H), 7.58 (dd, 1H), 6.89 (d, 1H), 4.63 (s, 2H), 4.29 (s, 2H), 3.81 (s, 3H), 3.60 (s, 3H), 3.11 (s, 3H), 2.86 (t, 4H),1.50-1.58 (m, 4H), 1.27-1.35 (m, 2H).

### Example 52

### N-methoxy-2-[2-methoxy-5-(piperidine-1-sulphonyl)-benzyloxy)-N-methyl-acetamide (166 mg, colourless oil, 49% yield)

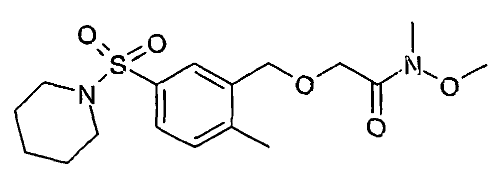

¹H NMR (CDCl₃) δ: 7.73 (s, 1H), 7.55 (d, 1H), 7.29 (d, 1H), 4.68 (s, 2H), 4.33 (s, 2H), 3.65 (s, 3H), 3.18 (s, 3H), 2.93 (t, 4H), 2.41 (s, 3H), 1.57-1.64 (m, 4H), 1.33-1.41 (m, 2H).

### Example 53

### 2-[2-Bromo-5-(piperidine-1-sulphonyl)-benzyloxy)-N-methoxy-N-methyl-acetamide (200 mg, white solid)

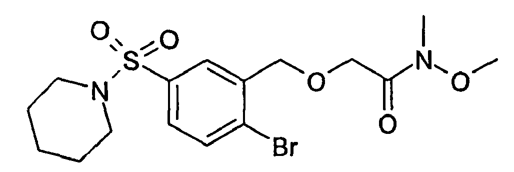

¹H NMR (CDCl₃) δ: 7.96 (s, 1H), 7.70 (d, 2H), 7.54 (dd, 1H), 4.79 (s, 2H), 4.46 (s, 2H), 3.72 (s, 3H), 3.24 (s, 3H), 3.01 (t, 4H), 2.41 (s, 3H), 1.63-1.70 (m, 4H), 1.41-1.48 (m, 2H).

### Example 54

### N-Methoxy-N-methyl-2-[5-(piperidine-1-sulphonyl)-2-trifluoromethoxy-benzyloxy]-acetamide (colourless oil)

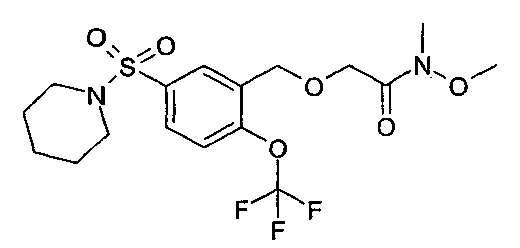

¹H NMR (CDCl₃) δ: 8.05 (s, 1H), 7.74 (d, 1H), 7.36 (dd, 1H), 4.80 (s, 2H), 4.42 (s, 2H), 3.71 (s, 3H), 3.23 (s, 3H), 3.01 (t, 4H), 1.63-1.70 (m, 4H), 1.41-1.49 (m, 2H).

### Example 55

### 2-(1-(2-chloro-5-(piperidine-1-sulfonyl)-phenyl)-ethoxy)-N-methoxy-N-methylacetamide

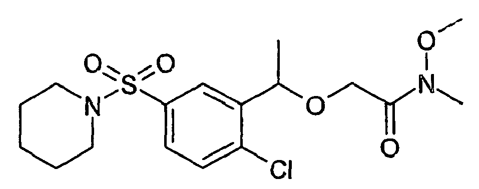

¹H NMR (CDCl₃) δ: 1.47 (m, 2H), 1.56 (d, 3H), 1.66 (m, 4H), 3.01 (t, 4H), 3.20 (s, 3H), 3.64 (s, 3H), 4.06 (d, 1H), 4.29 (d, 1H), 5.08 (q, 1H), 7.50 (d, 1H), 7.64 (d,1H), 7.97 (s, 1H).

### Example 56

### 2-[2-Chloro-5-(thiomorpholine-4-sulfonyl)-benzyloxy]-N-methoxy-N-methylacetamide (370 mg, white solid, 31 % yield)

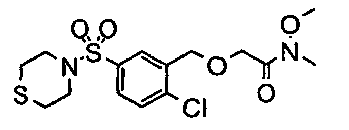

¹H NMR (CDCl₃) δ: 7.98(d, 1H), 7.62 (dd, 1H), 7.52 (d, 1H), 4.82 (s, 2H), 4.46 (s, 2H), 3.72 (s, 3H), 3.35-3.38 (m, 4H), 3.23 (s, 3H), 2.71-2.74 (m, 4H).

### General synthesis of 2-(alkylaminosulphonyl)benzyloxyethanones

### Method A: Using commercially available Grignard reagent solutions

To an ice-cold solution of 2-(2-chloro-5-(piperidine-1-sulphonyl)benzyloxy)-N-methoxy-N-methylacetamide (80 mg, 0.205 mmol) in anhydrous THF (3 mL) was added phenylmagnesium bromide (205 µL of a 1.0M solution in THF, 0.205 mmol). After 1 hour more phenylmagnesium bromide (50 µL of a 1.0M solution in THF, 0.050 mmol) was added. After an additional hour the reaction was quenched with saturated NH₄Cl and poured into water. This was extracted with EtOAc. The organic phase was washed with brine, dried (MgSO₄), filtered, and concentrated. Column chromatography (20% EtOAc/hexanes) provided 2-(2-chloro-5-(piperidine-1-sulphonyl)benzyloxy)-1-phenylethanone (28 mg, 34%) as a colourless oil. ¹H NMR (CDCl₃) δ: 7.93-7.96 (m, 3H), 7.58-7.64 (m, 2H), 7.46-7.51 (m, 3H), 4.92 (s, 2H), 4.82 (s, 2H), 2.98 (t, 4H), 1.59-1.67 (m, 4H), 1.41-1.43 (m, 2H).

In a similar fashion, the following compounds (Example 57 - 70) were made.

### Example 57

### 2-[2-Chloro-5-(piperidine-1-sulphonyl)benzyloxy]-1-(3-methoxyphenyl)ethanone (48 mg, colourless oil, 86% yield)

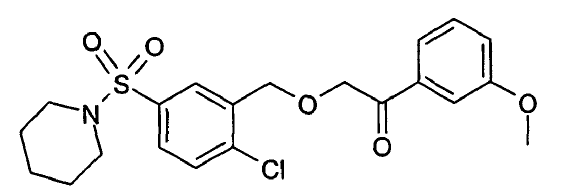

¹H-NMR (CDCl₃) δ: 7.96 (d, 1H), 7.60 (dd, 1H), 7.47 (dd, 3H), 7.37 (t, 1H), 7.13 (dd, 1H), 4.90 (s, 2H), 4.81 (s, 1H), 3.80 (s, 3H), 2.97 (t, 4H), 1.59-1.66 (m, 4H), 1.40-1.44 (m, 2H).

### Example 58

### 2-[2-Chloro-5-(piperidine-1-sulphonyl)benzyloxy]-1-cyclohexyletbanone (29 mg, colourless oil, 55% yield)

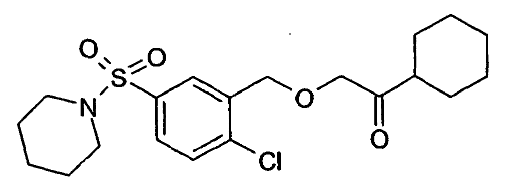

¹H-NMR (CDCl₃) δ: 7.91 (d, 1H), 7.62 (dd, 1H), 7.49 (d, 1H), 4.70 (s, 2H), 4.28 (s, 2H), 2.99 (t, 4H), 2.47-2.56 (m, 1H), 1.80 (t, 4H), 1.60-1.68 (m, 4H), 1.18-1.44 (m, 7H).

### Example 59

### 2-[2-Chloro-5-(piperidine-1-sulfonyl)benzyloxy]-1-(2,5-dimethoxyphenyl)ethanone (30 mg, colourless oil, 49% yield)

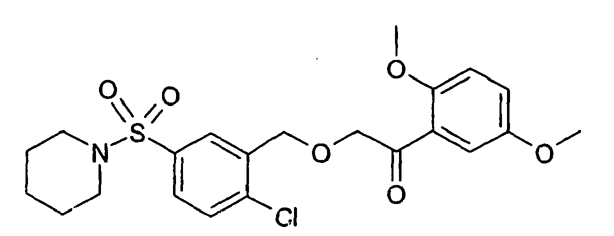

¹H-NMR (CDCl₃) δ: 7.99 (d, 1H), 7.61 (dd, 1H), 7.46 (m, 2H), 7.08 (dd, 1H), 6.92 (d, 1H), 4.86 (s, 2H), 4.81 (s, 2H), 3.89 (s, 3H), 3.81 (s, 3H), 2.99 (t, 4H), 1.63 (m, 4H), 1.42 (m, 2H).

### Example 60

### 2-[2,4-Dichloro-5-(piperidine-1-sulphonyl)benzyloxy]-1-(3-methoxyphenyl)ethanone (41.5 mg, white solid, 57% yield)

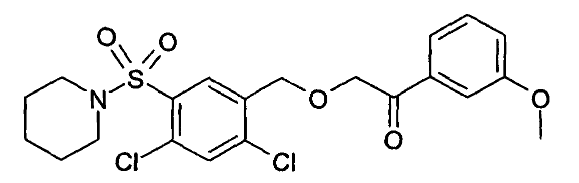

¹H-NMR (CDCl₃) δ: 8.22 (s, 1H), 7.46-7.52 (m, 3H), 7.38 (t, 1H), 7.13 (dd, 1H), 4.89 (s, 2H), 4.75 (d, 2H), 3.85 (s, 3H), 3.25 (t, 4H), 1.53-1.61 (m, 6H).

### Example 61

### 1-(3-Chlorophenyl)-2-[2-chloro-5-(piperidine-1-sulphonyl)benzyloxy]-ethanone (20 mg, white solid, 35% yield)

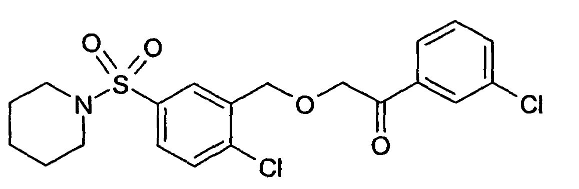

¹H-NMR (CDCl₃) δ: 7.93 (m, 2H), 7.83 (dd, 1H), 7.62 (dd, 1H), 7.58 (dd, 1H), 7.55-7.40 (m, 2H), 4.88 (s, 2H), 4.80 (s, 1H), 2.98 (t, 4H), 1.63 (m, 4H), 1.42 (m, 2H).

### Example 62

### 1-(3-Chlorophenyl)-2-[2,4-dichloro-5-(piperidine-1-sulphonyl)benzyloxy]-ethanone (53.2 mg, white solid, 73% yield)

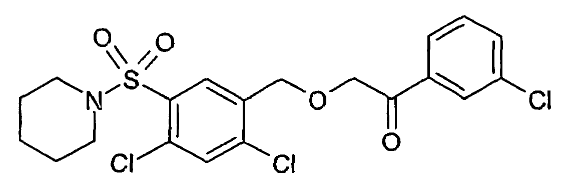

¹H-NMR (CDCl₃) δ: 5.20 (s, 1H), 7.91 (s, 1H), 7.81 (s, 1H), 7.54 (dd, 2H), 7.43 (t, 1H), 4.91 (s, 2H), 4.74 (s, 1H), 3.25 (t, 4H), 1.53-1.63 (m, 6H).

### Example 63

### 2-[2,4-Dichloro-5-(piperidine-1-sulphonyl)benzyloxy]-1-naphthalen-2-ylethanone (47.6 mg, white solid, 63% yield)

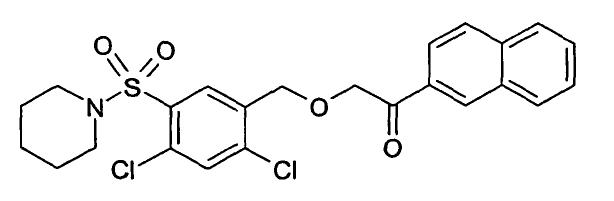

¹H-NMR (CDCl₃) δ: 8.47 (s, 1H), 8.26 (s, 1H), 7.86-8.01 (m, 4H), 7.51-7.64 (m, 2H), 7.43 (t, 1H), 5.02 (s, 2H), 4.80 (s, 1H), 3.24 (t, 4H), 1.51-1.59 (m, 6H).

### Example 64

### 2-[2-chloro-5-(piperidine-1-sulphonyl)benzyloxy]-1-naphthalen-2-ylethanone (29 mg, colourless oil, 49% yield)

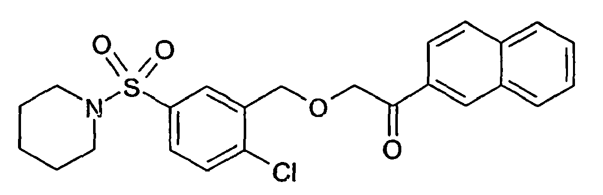

¹H-NMR (CDCl₃) δ: 8.48 (s, 1H), 8.02-7.87 (m, 5H), 7.64-7.48 (m, 4H), 5.05 (s, 2H), 4.86 (s, 2H), 2.96 (t, 4H), 1.60 (m, 4%, 1.40 (m, 2H).

### Example 65

### 4-Chloro-N,N-dimethyl-5-(2-oxo-2-phenylethoxymethyl)benzenesulphonamide (43 mg, white solid, 68% yield)

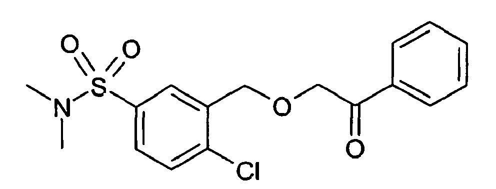

¹H-NMR (CDCl₃) δ: 8.00 (s, 1H), 7.93 (d, 2H), 7.57-7.66 (m, 2H), 7.45-7.52 (m, 3H), 4.92 (s, 2H), 4.82 (s, 2H), 2.71(s, 6H).

### Example 66

### 4-Chloro-N,N-diethyl-3-(2-oxo-2-phenylethoxymethyl)benzenesulphonamide (50 mg, pale yellow oil, 80% yield)

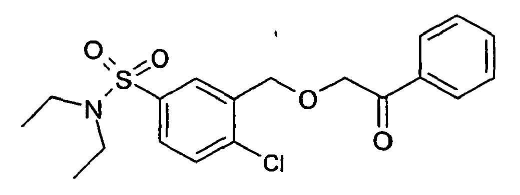

¹H-NMR (CDCl₃) δ: 8.04 (s, 1H), 7.96 (dd, 2H), 7.70 (dd, 1H), 7.59-7.62 (m, 1H), 7.48 (t, 3H) 4.92 (s, 2H), 4.82 (s, 2H),3.25 (q, 4H), 1.15(t, 6H).

### Example 67

### 4-Chloro-N-ethyl-N-methyl-3-(2-oxo-2-phenylethoxymethyl)benzenesulphonamide (45 mg, pale yellow oil, 71% yield)

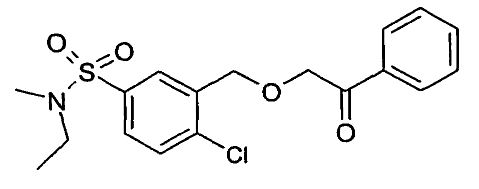

¹H-NMR (CDCl₃) δ: 8.02 (s, 1H), 7.96 (dd, 2H), 7.59-7.66 (m, 2H), 7.47-7.52 (m, 3H) 4.94 (s, 2H), 4.83 (s, 2H), 3.12 (q, 2H), 2.76 (s, 3H), 1.15(t, 3H).

### Example 68

### 4-Chloro-N-methyl-3-(2-oxo-2-phenylethoxymethyl)-N-propyl-benzenesulphonamide (47 mg, pale yellow oil, 75% yield)

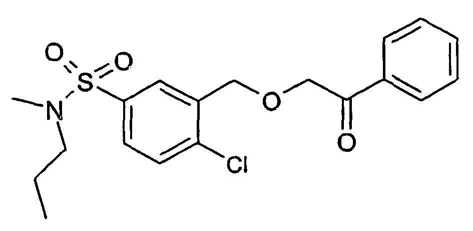

¹H-NMR (CDCl₃) δ: 8.02 (s, 1H), 7.95 (dd, 2H), 7.59-7.66 (m, 2H), 7.47-7.52 (m, 3H) 4.94 (s, 2H), 4.83 (s, 2H), 2.99 (t, 2H), 2.75 (s, 3H), 1.57 (q, 2H), 0.95(t, 3H).

### Example 69

### 2-[2-Bromo-5(piperidine-1-sulphonyl)-benzyloxyl-1-phenyl-ethanone (206 mg, oil, 99% yield)

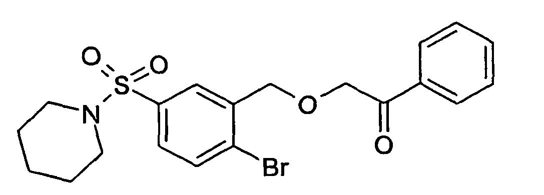

¹H-NMR (CDCl₃) δ: 7.94 (t, 3H), 7.69 (d, 1H), 7.45-7.60 (m, 4H), 4.94 (s, 2H), 4.78 (s, 2H), 2.97 (t, 4H), 1.55-1.66 (m, 4H), 1.36-1.44(m, 2H).

### Example 70

### 2-[2-Chloro-5-(thiomorpholine-4-sulfonyl)-benzyloxy]-1-phenyl-ethanone (150 mg, colourless oil, 80 % yield)

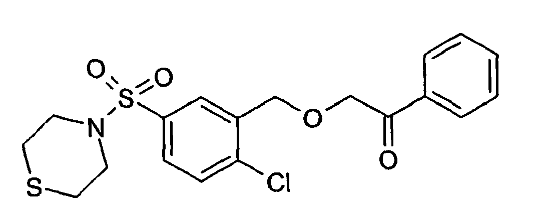

¹H-NMR (CDCl₃) δ: 7.95-8.01 (m, 3H), 7.62-7.66 (m, 2H), 7.49-7.61 (m, 3H), 4.95 (s, 2H), 4.85 (s, 2H), 3.35-3.38 (m, 4H), 2.71-2.74 (m, 4H).

### Example 71

### Synthesis of 2-(2-chloro-5-(piperidine-1-sulfonyl)-benzyloxyl)-1-(1H-pyrrolo(2,3-b)-pyridin-3-yl)-ethanone

To a solution of 1H-pyrrolo(2,3-b)-pyridine (71 mg, 0.6 mmol) in THF (2 ml) was added a Et₂O solution of t-BuMgBr (2M, 0.33 ml, 0.66 mmol) at -50°C and stirred at room temperature for 10 minutes. The mixture was cooled to -50°C and was added a solution of 2-(2-chloro-5-(piperidine-1-sulfonyl)-benzyloxyl)-acetyl chloride (200 mg, 0.6 mmol), prepared from corresponding acetic acid, oxylchloride, catalytic amount of DMF and CH₂Cl₂, in THF (2 ml) at this temperature and stirred at room temperature for 1 h. To this resulting mixture was added H₂O (2 ml) and extracted with CH₂Cl₂ (5 ml x 3). The combined extract was dried and the solvent was removed under reduced pressure to leave residue which was purified on silica gel column to provide the product (12 mg, 10%)

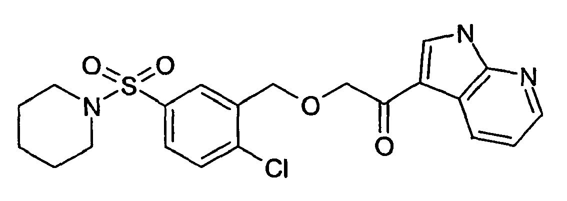

¹H NMR (CDCl₃): δ 1.46 (m, 2H), 1.61 (m, 4H), 3.00 (t, 4H), 4.73 (s, 2H), 4.86 (s, 2H), 7.32-7.67 (m, 3H), 8.02 (s, 1H), 8.40 (s, 1H), 8.50 (m, 1H), 8.73 (d, 1H).

### Method B: Using lithium/halogen exchange

### Example 72

### Synthesis of 2-[2-chloro-5-(4-methylpiperazine-1-sulfonyl)benzyloxy]-1-pyridin-2-yl-ethanone

To a solution of 2-bromopyridine (80 mg, 0.5 mmol) in Et₂O (2 ml) was added n-BuLi (1.6 M, 0.35 ml, 0.55 mmol) in hexane at -78°C. The mixture was stirred at this temperature for 1 h. To this was added a solution of 4-{4-chloro-3-[(methoxymethylcarbamoyl) methoxymethyl]benzenesulfonyl}piperazine-1-carboxylic acid tert-butyl ester (200 mg, 0.4 mmol) in THF (1 ml) at -78°C and was kept stirring at this temperature for 2 hours. To this resulting mixture was added H₂O (1 ml) at rt and extracted with AcOEt (3 x 3 ml). The combined extract was dried and concentrated under reduced pressure to afford residue. This residue was treated with CH₂Cl₂:TFA (1:1,2 ml) at 0°C for 30 minutes. The resulting mixture was neutralized with saturated Na₂CO₃ aq and extracted with AcOEt (5 x 5 ml). The combined extract was dried and the solvent was removed under reduced pressure to afford residue, which was purified on silica gel column to give the deprotected intermediate 2-[2-chloro-5-(piperazine-1-sulfonyl)benzyloxy]-N-methoxy-N-methylacetamide (92 mg). The intermediate was diluted with formic acid (0.6 ml) and was added H₂CO (37 % aq, 1.2 ml) and a solution of NaCNBH₃ in THF (1 M, 0.16 ml, 0.16 mmol) at 0°C. The mixture was stirred at 0°C for 20 minutes. The resulting mixture was neutralized with saturated Na₂CO₃ aq and extracted with AcOEt (5 x 5 ml). The combined extract was dried and concentrated under reduced pressure to give residue, which was purified on silica gel column to afford the product (11 mg, 6.5 % allover yield)

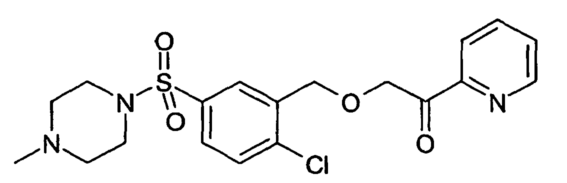

¹H NMR (CDCl₃) δ: 2.275 (s, 3H), 2.464 (t, J = 5.0 Hz, 4H), 3.062 (t, J = 5.0 Hz, 4H), 4.875 (s, 2H), 5.250 (s, 2H), 7.535 (m, 2H), 7.640 (d, J = 8.6 Hz, 1H), 7.888 (m, 1H), 8.074 (m, 2H), 8.644 (s, 1H).

In a similar fashion, the following compounds (Examples 73-101) were synthesized.

### Example 73

### 2-[2-Chloro-5-(piperidine-1-sulphonyl)benzyloxy]-1-pyridin-3-ylethanone (42 mg, 42% yield)

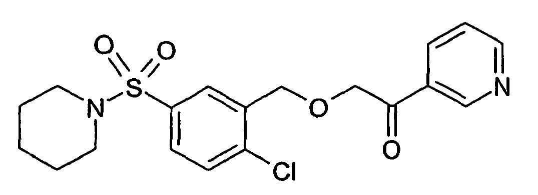

¹H-NMR (CDCl₃) δ: 9.14 (s, 1H), 8.80 (s, 1H), 8.23 (dd, 1H), 7.92 (s, 1H), 7.60 (dd, 1H), 7.42-7.50 (m, 2H), 4.89 (s, 2H), 4.80 (s, 1H), 2.97 (t, 4H), 1.59-1.66 (m, 6H), 1.41-1.45 (m, 4H).

### Example 74

### 2-[2-Chloro-5-(piperidine-1-sulphonyl)benzyloxy]-1-pyridin-4-ylethanone (33 mg, colourless oil, 33% yield)

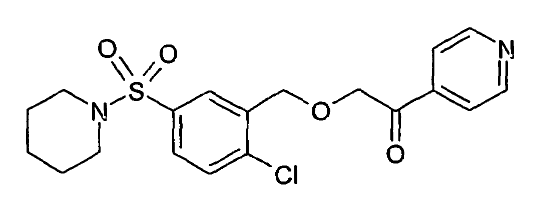

¹H-NMR (CDCl₃) δ: 8.85 (d, 2H), 7.96 (d, 1H), 7.74 (dd, 2H), 7.66 (dd, 1H), 7.53 (d, 1H), 4.92 (s, 2H), 4.87 (s, 2H), 3.03 (t, 4H), 1.63-1.70 (m, 6H), 1.41-1.49 (m, 4H).

### Example 75

### 2-[2-Chloro-5-(piperidine-1-sulphonyl)benzyloxy]-1-pyridin-2-ylethanone (16.8 mg, pale yellow solid, 19% yield)

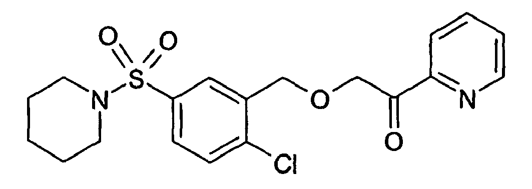

¹H-NMR (CDCl₃) δ: 8.32 (d, 1H), 8.10 (d, 1H), 8.03 (s, 1H), 7.89 (t, 1H), 7.64 (dd, 1H), 7.51-7.54 (m, 2H), 5.26 (s, 2H), 4.87 (s, 2H), 3.02 (t, 4H), 1.62-1.69 (m, 6H), 1.40-1.47 (m, 4H).

### Example 76

### 4-Chloro-N,N-dimethyl-3-(2-oxo-2-pyridin-3-ylethoxymethyl)benzenesulphonamide (10 mg, yellow oil, 11% yield)

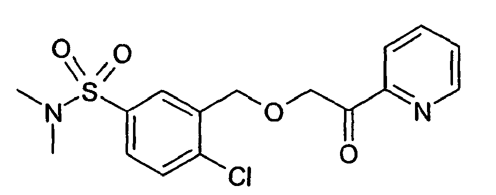

¹H-NMR (CDCl₃) δ: 9.19 (s, 1H), 8.85 (s, 1H), 8.30 (dd, 1H), 7.99 (s, 1H), 7.68 (dd, 1H), 7.48-7.56 (m, 2H), 4.92 (s, 2H), 4.87 (s, 2H), 2.74 (s, 6H).

### Example 77

### 2-[2-Chloro-5-(piperidine-1-sulphonyl)benzyloxy]-1-quinolin-8-ylethanone (51 mg, pale yellow solid, 43% yield)

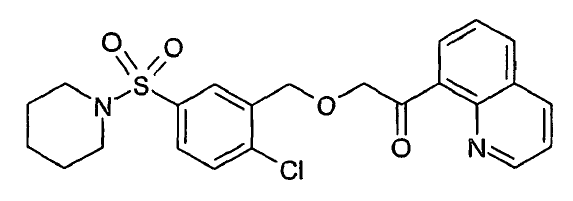

¹H-NMR (CDCl₃) δ: 8.96 (t, 1H), 8.20 (t, 2H), 7.97 (t, 2H), 7.60-7.67 (m, 2H), 7.48-7.51 (m, 2H), 5.37 (s, 2H), 4.92 (s, 2H), 2.98 (t, 4H), 1.58-1.66 (m, 4H), 1.36-1.44 (m, 6H).

### Example 78

### 4-Chloro-N,N-dimethyl-3-(2-oxo-2-pyridin-2-ylethoxymethyl)benzenesulphonamide (28 mg, yellow solid, 27% yield)

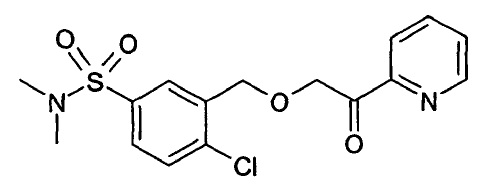

¹H-NMR (CDCl₃) δ: 8.65 (d, 1H), 8.09 (d, 2H), 7.89 (t, 1H), 7.67 (dd, 1H), 7.51-7.55 (m, 2H), 5.27 (s, 2H), 4.88 (s, 2H), 2.75 (s, 6H).

### Example 79

### 4-Chloro-N-ethyl-N-methyl-3-(2-oxo-2-pyridin-2-ylethoxymethyl)benzenesulphonamide (38 mg, yellow solid, 30% yield)

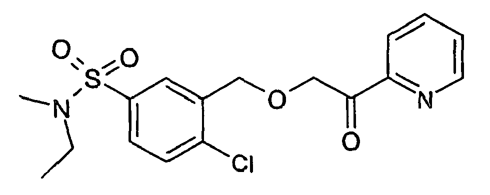

¹H-NMR (CDCl₃) δ: 8.64 (d, 1H), 8.07 (d, 2H), 7.89 (t, 1H), 7.66 (dd, 1H), 7.49-7.55 (m, 2H), 5.24 (s, 2H), 4.86 (s, 2H), 3.13 (q, 2H), 2.77 (s, 6H), 1.18 (t, 3H).

### Example 80

### 4-Chloro-N,N-diethyl-3-(2-oxo-2-pyridin-2-ylethoxymethyl)benzenesulphonamide (46 mg; white solid, 36% yield)

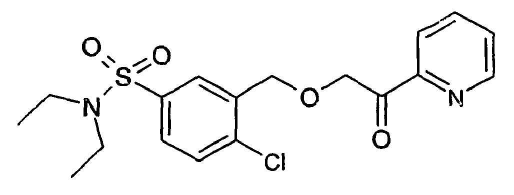

¹H-NMR (CDCl₃) δ: 8.64 (d, 1H), 8.08 (d, 2H), 7.89 (t, 1H), 7.70 (dd, 1H), 7.47-7.55 (m, 2H), 5.25 (s, 2H), 4.86 (s, 2H), 3.27 (q, 4H), 1.16 (t, 6H).

### Example 81

### 4-Chloro-N-methyl-3-(2-oxo-2-pyridin-2-ylethoxymethyl)-N-propylbenzenesulphonamide (57 mg, white solid, 45% yield)

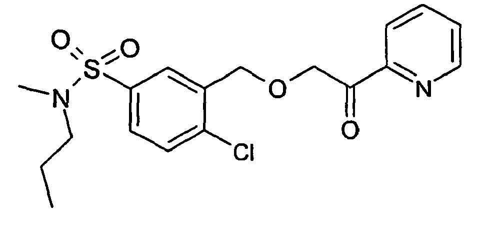

¹H-NMR (CDCl₃) δ: 8.64 (d, 1H), 8.07 (d, 2H), 7.85 (t, 1H), 7.65 (dd, 1H), 7.50-7.55 (m, 2H), 5.25 (s, 2H), 4.86 (s, 2H), 2.99 (t, 2H), 2.75 (s, 3H), 1.63-1.51 (m, 2H), 0.93 (t, 6H).

### Example 82

### 4-Chloro-N-ethyl-N-methyl-3-(2-oxo-2-pyridin-3-ylethoxymethyl)benzenesulphonamide (44 mg, yellow solid, 35% yield)

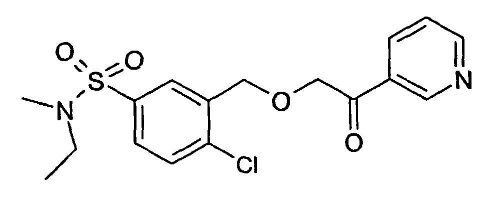

¹H-NMR (CDCl₃) δ: 9.16 (d, 1H), 8.82 (dd, 1H), 8.26 (dd, 1H), 7.98 (d, 1H), 7.67 (dd, 1H), 7.46-7.50. (m, 2H), 4.91 (s, 2H), 4.82 (s, 2H), 3.12 (q, 2H), 2.75 (s, 3H), 1.15 (t, 3H).

### Example 83

### 4-Chloro-N,N-diethyl-3-(2-oxo-2-pyridin-3-ylethoxymethyl)benzenesulphonamide (38 mg, yellow solid, 30% yield)

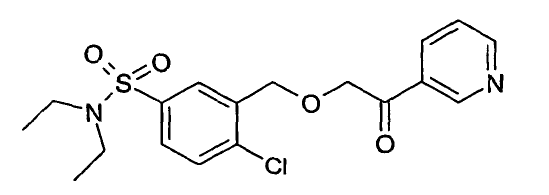

¹H-NMR (CDCl₃) δ: 9.17 (d, 1H), 8.83 (dd, 1H), 8.27 (dd, 1H), 8.01 (d, 1H),7.70 (dd, 1H), 7.45-7.47 (m, 2H), 4.90 (s, 2H), 4.82 (s, 2H), 3.24 (q, 4H), 1.15 (t, 6H).

### Example 84

### 2-[2-Chloro-5-(piperidine-1-sulpfonyl)-benzyloxy]-1-(4-methyl-pyridin-2-yl)-ethanone (41 mg, yellow oil, 32% yield)

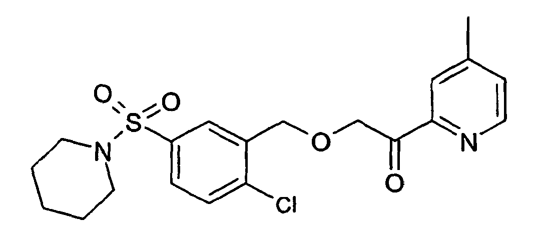

¹H-NMR (CDCl₃) δ: 8.49 (s, 1H), 8.02 (s, 1H), 7.90 (s, 1H), 7.64 (dd, 1H), 7.51 (d, 1H), 7.33 (d, 1H), 5.23 (s, 2H), 4.86 (s, 2H), 3.01 (t, 4H), 2.45 (s, 3H), 1.60-1.68 (m, 4H), 1.3 9-1.46 (m, 2H).

### Example 85

### 2-[2-Chloro-5-(piperidine-1-sulpfonyl)-benzyloxy]-1-(6-methyl-pyridin-2-yl)-ethanone (41 mg, yellow oil, 32% yield)

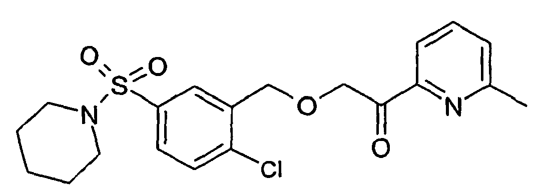

¹H-NMR (CDCl₃) δ: 8.03 (s, 1H), 7.87 (d, 1H), 7.74 (t, 1H), 7.64 (dd, 1H), 7.51 (d, 1H), 7.37 (d, 1H), 5.26 (s, 2H), 4.87 (s, 2H), 3.01 (t, 4H), 2.59 (s, 3H), 1.61-1.68 (m, 4H), 1.39-1.46 (m, 2H).

### Example 86

### 2-[2-Chloro-5-(piperidine-1-sulpfonyl)-benzyloxy]-1-(5-methyl-pyridin-2-yl)-ethanone (43 mg, colourless oil, 33% yield)

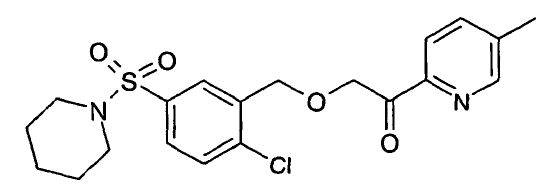

¹H-NMR (CDCl₃) δ: 8.45 (s, 1H), 8.02 (s, 1H) 7.98 (d, 1H), 7.65 (t, 2H), 7.52 (d, 1H), 5.23 (s, 2H), 4.87 (s, 2H), 3.01 (t, 4H), 2.43 (s, 3H), 1.61-1.68 (m, 4H), 1.39-1.46 (m, 2H).

### Example 87

### 4-Chloro-N-ethyl-N-metliyl-3-[2-(4-methyl-pyridin-2-yl)-2-oxo-ethoxymethyl]-benzenesulfonamide (49 mg, oil, 37% yield)

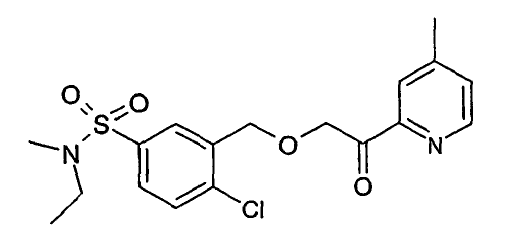

¹H-NMR (CDCl₃) δ: 8.48 (d, 1H), 8.05 (s, 1H) 7.90 (s, 1H), 7.66 (d, 2H), 7.51 (d, 1H), 7.32 (dd, 1H), 5.23 (s, 2H), 4.85 (s, 2H), 3.13 (q, 2H), 2.76 (s, 3H), 2.46 (s, 3H), 1.15 (t, 3H).

### Example 88

### 4-Chloro-N-ethyl-N-methyl-3-[2-(6-methyl-pyridin-2-yl)-2-oxo-ethoxymethyl]-benzenesulfonamide (42.5 mg, pale yellow oil, 32% yield)

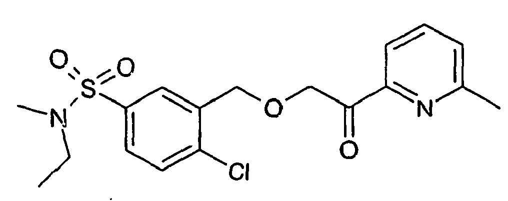

¹H-NMR (CDCl₃) δ: 8.07 (s, 1H), 7.88 (d, 1H) 7.66-7.86 (m, 2H), 7.51 (d, 1H), 7.37 (d, 1H), 5.26 (s, 2H), 4.86 (s, 2H), 3.14 (q, 2H), 2.80 (s, 3H), 2.59 (s, 3H), 1.15 (t, 3H).

### Example 89

### 4-Chloro-N-ethyl-N-methyl-3-[2-(5-methyl-pyridin-2-yl)-2-oxo-ethoxymethyl]-benzenesulfonamide (58 mg, yellow solid, 44% yield)

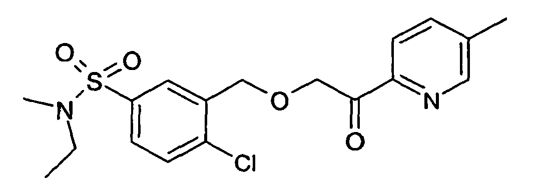

¹H-NMR (CDCl₃) δ: 8.44 (s, 1H), 8.05 (s, 1H) 7.97 (d, 1H), 7.66 (dd, 2H), 7.49 (d, 1H), 5.22 (s, 2H), 4.85 (s, 2H), 3.12 (q, 2H), 2.76 (s, 3H), 2.43 (s, 3H), 1.15 (t, 3H).

### Example 90

### 2-[2-Chloro-5-(piperidine-1-sulpfonyl)-benzyloxy]-1-(3-methyl-pyridin-2-yl)-ethanone (34 mg, white solid, 26% yield)

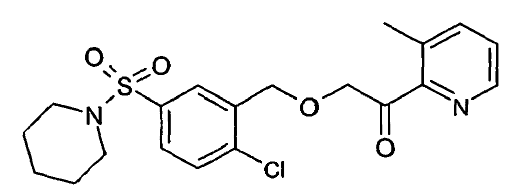

¹H-NMR (CDCl₃) δ: 8.47 (d, 1H), 8.04 (s, 1H) 7.64 (d, 2H), 7.52 (d, 1H), 7.39 (dd, 1H), 5.20 (s, 2H), 4.87 (s, 2H), 3.02 (t, 4H), 2.66 (s, 3H), 1.59-1.69 (m, 4H), 1.39-1.47 (m, 2H).

### Example 91

### 4-Chloro-N-ethyl-N-methyl-3-[2-(3-methyl-pyridin-2-yl)-2-oxo-ethoxymethyl]-benzenesulfonamide (41 mg, colourless oil, 31% yield)

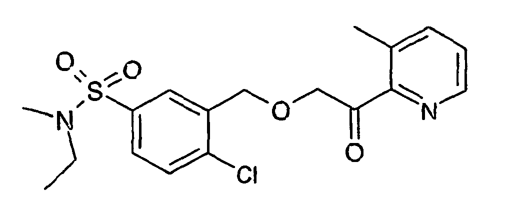

¹H-NMR (CDCl₃) δ: 8.47 (d, 1H), 8.07 (s, 1H) 7.65-7.69 (m, 2H), 7.51 (d, 2H), 7.39 (dd, 1H), 5.20 (s, 2H), 4.86 (s, 2H), 3.14 (q, 2H), 2.77 (s, 3H), 2.66 (s, 3H), 1.16 (t, 3H).

### Example 92

### 1-(2-bromo-5-dimethylamino-pyridin-4-yl)-2-[2-chloro-5-(piperidine-1-sulpfonyl)-benzyloxy]-ethanone (yellow oil)

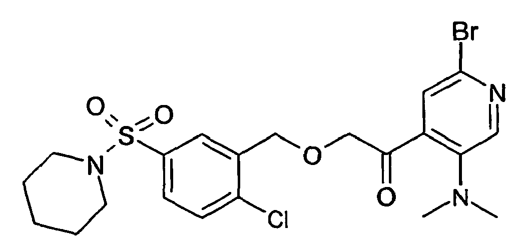

¹H-NMR (CDCl₃) δ: 8.16 (s, 1H), 8.86 (s, 1H) 7.64 (d, 1H), 7.53 (d, 1H), 7.34 (s, 1H), 4.80 (s, 2H), 4.76 (s, 2H), 2.98 (t, 4H), 2.86 (s, 3H), 1.63-1.70 (m, 4H), 1.41-1.48 (m, 2H).

### Example 93

### 2-[2-Methoxy-5-(piperidine-1-sulpfonyl)-benzyloxy]-1-pyridin-2-yl-ethanone (39 mg, pale yellow solid, 50% yield)

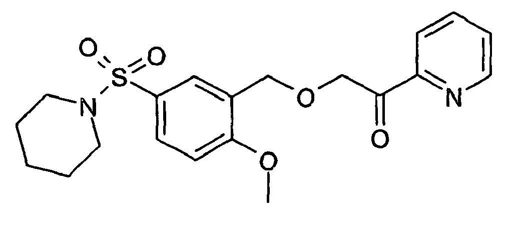

¹H-NMR (CDCl₃) δ: 8.62 (d, 1H), 8.07 (d, 1H) 7.87 (t, 2H), 7.70 (d, 1H), 7.52 (dd, 1H), 6.96 (d, 1H), 5.19 (s, 2H), 4.80 (s, 2H), 3.90 (s, 3H), 2.97 (s, 3H), 1.59-1.73 (m, 4H), 1.27-1.43 (m, 2H).

### Example 94

### 2-[2-Methyl-5-(piperidine-1-sulpfonyl)-benzyloxy]-1-pyridin-2-yl-ethanone (5.0 mg, pale yellow solid, 5% yield)

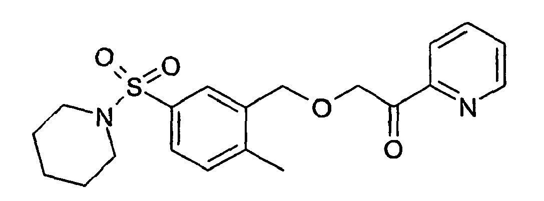

¹H-NMR (CDCl₃) δ: 8.64 (d, 1H), 8.08 (d, 1H) 7.89 (t, 1H), 7.81 (s, 1H), 7.61 (d, 1H), 7.54 (dd, 1H), 7.31 (d, 1H), 5.18 (s, 2H), 4.79 (s, 2H), 2.49 (s, 3H), 1.57-1.68 (m, 4H), 1.25-1.30 (m, 2H).

### Example 95

### 2-5-(Piperidine-1-sulpfonyl)-2-trifluoromethoxy-benzyloxy]-1-pyridin-2-yl-ethanone (17.5 mg, pale yellow solid, 17% yield)

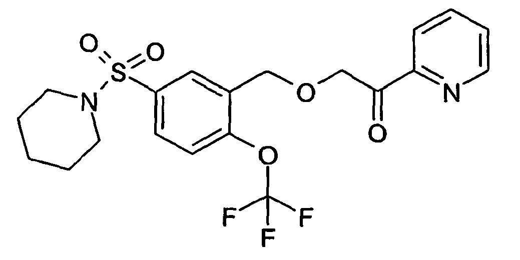

¹H-NMR (CDCl₃) δ: 8.65 (d, 1H), 8.09 (d, 2H) 7.89 (t, 1H), 7.74 (d, 1H), 7.55 (dd, 1H), 7.41 (d, 1H), 5.24 (s, 2H), 4.86 (s, 2H), 3.05(t, 3H), 1.63-1.71 (m, 4H), 1.41-1.49 (m, 2H).

### Example 96

### 2-(1-(2-chloro-5-(piperidine-1-sulfonyl)-phenyl)-ethoxy)-1-pyridine-2-yl-ethanone (70 mg, 69 % yield)

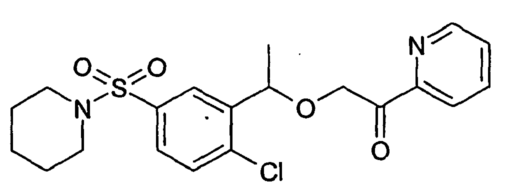

¹H-NMR (CDCl₃) δ: 1.40 (m, 2H), 1.52-1.64 (m, 7H), 2.98 (t, 4H), 4.93-5.15 (m, 3H), 7.49-7.64 (m, 3H), 7.87 (d, 1H), 8.02 (s, 1H), 8.06 (d, 1H), 8.56 (m, 1H).

### Example 97

### 2-(1-(2-chloro-5-(piperidine-1-sulfonyl)-phenyl)-ethoxy)-1-pyridine-3-yl-ethanone (52 mg, 51 % yield)

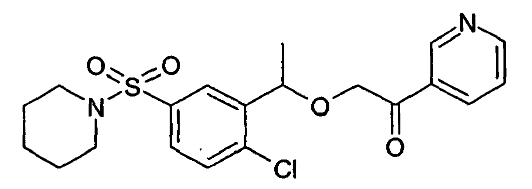

¹H-NMR (CDCl₃) δ: 1.44 (m, 2H), 1.51-1.63(m, 7H), 3.00 (t, 4H), 4.60 (d, 1H), 4.71 (d, 1H), 5.09 (q, 1H), 7.46-7.66 (m, 3H), 7.96 (s, 1H), 8.22 (m, 1H), 8.82 (d, 1H), 9.12 (s, 1H).

### Example 98

### 2-(2-chloro-5-(piperidine-1-sulfonyl)-benzyloxy)-1-pyrazine-2-yl-ethanone (22 mg, 19 % yield)

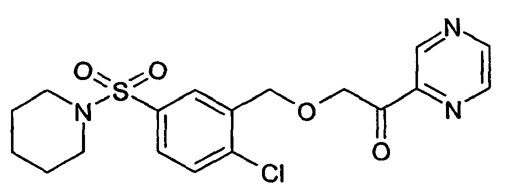

¹H-NMR (CDCl₃) δ : 1.46 (m, 2H), 1.66 (m, 4H), 3.03(t, 4H), 4.88 (s, 2H), 5.21 (s, 2H), 7.52-7.67 (m, 2H), 8.03 (s, 1H), 8.64 (d, 1H), 8.83 (d, 1H), 9.27 (s, 1H).

### Example 99

### 2-(2-chloro-5-(piperidine-1-sulfonyl)-benzyloxy)- 1-(1-methyl-1H-imidazol-2-yl)-ethanone (95 mg, 77 %)

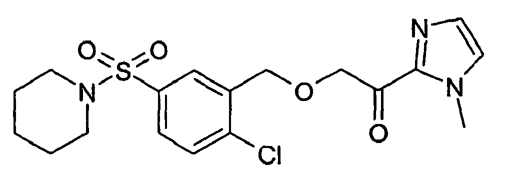

¹H-NMR (CDCl₃) δ: 1.44 (m, 2H), 1.66 (m, 4H), 3.02 (t, 4H), 4.06 (s, 3H), 4.85 (s, 2H), 5.09 (s, 2H), 7.10 (d, 1H), 7.15 (d, 1H), 7.50-7.66 (m, 2H). 8.02 (s, 1H).

### Example 100

### 2-(2-chloro-5-(piperidine-1-sulfonyl)-benzyloxy)-1-(1-pyridin-2-yl-1H-imidazol-2-yl)ethanone (75 mg, 59 % yield) was prepared from 2-imidazol-1-yl-pyridine instead of its bromide in a similar procedure

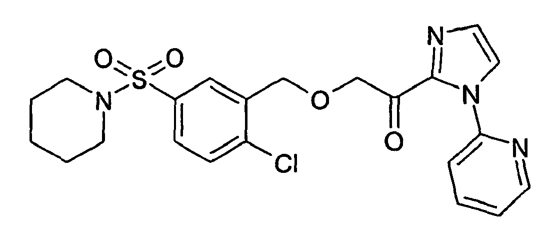

¹H-NMR (CDCl₃) δ: 1.43 (m, 2H), 1.65 (m, 4H), 2.98 (t, 4H), 4.80 (s, 2H), 5.14 (s, 2H), 7.28 (d, 1H), 7.44-7.61 (m, 5H), 7.90 (m, 1H), 7.97 (s, 1H), 8.58 (d, 1H)

### Example 101

### 2-(2-chloro-5-(piperidine-1-sulfonyl)-benzyloxy)-1-(1-methyl-1H-indol-5-yl)-ethanone (35 mg, 33 % yield)

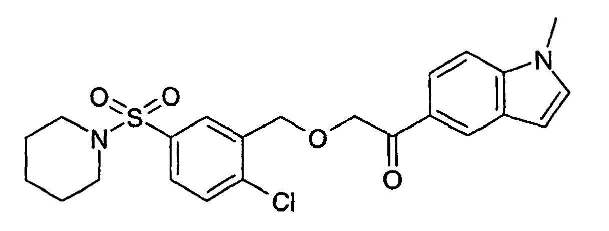

¹H-NMR (CDCl₃) δ: 1.44 (m, 2H), 1.66 (m, 4H), 3.00 (t, 4H), 3.86 (s, 3H), 4.87 (s, 2H), 5.02 (s, 2H), 6.63 (d, 1H), 7.15 (d, 1H), 7.38 (d, 1H), 7.51 (d, 1H), 7.63 (d, 1H), 7.89 (d, 1H) 8.02 (s, 1H), 8.31 (s, 1H).

### Example 102

### Synthesis of 2-[2-Chloro-5-(piperidine-1-sulfonyl)benzyloxy]-1-pyridin-2-yl-propan-1-one

A mixture of 2-[2-chloro-5-(piperidine-1-sulfonyl)benzyloxy]propionic acid tert-butyl ester (171 mg, 0.4 mmol) and HCO₂H (1.5 ml) was stirred at room temperature overnight. HCO₂H was removed under reduced pressure to give the acid, which was treated with oxalyl chloride (2M, 0.4 ml, 0.8 mmol) in CH₂Cl₂ (3 ml) in the presence of catalytic amount of DMF at room temperature for 2 hours to afford corresponding acetyl chloride. The acetyl chloride was diluted with CH2Cl2 (5 ml) and cooled to 0°C. To this were added ClH₂N(OMe)Me (48 mg, 0.5 mmol) and Et₃N (155 µl, 1.3 mmol) at this temperature and kept stirring for 1 h. To this resulting mixture was added H₂O (1 ml) and extracted with CH₂Cl₂ (5 ml x 3). The combined extract was dried and the solvent was removed under reduced pressure to give the amide which was treated with 2-lithiopyridine in a similar procedure mentioned above to afford the final product (106 mg, 61 %)

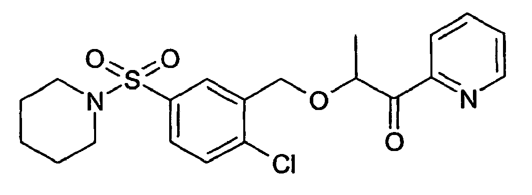

¹H-NMR (CDCl₃) δ: 1.46 (m, 2H), 1.53-1.64 (m, 7H), 3.01 (t, 4H), 4.51 (d, 1H), 4.85 (d, 1H), 5.59 (q, 1H), 7.47-7.64 (m, 3H), 7.89 (d, 1H), 8.01 (s, 1H), 8.09(d, 1H), 8.69 (m, 1H).

### Method C: Forming the Grignard reagent using magnesium metal

To a solution of magnesium turnings (8.0 equiv) in THF was added 1,2-dibromoethane (2 drops). 1-Bromo-3-methoxymethylbenzene (1.0 equiv) dissolved in THF was added dropwise and the reaction stirred at room temperature for 2 hours. The Grignard solution was added dropwise to the alkylaminosulphonylbenzyloxy-N-methoxy-N-methylacetamide dissolved in THF at 0°C. After stirring for an additional 30 minutes the reaction was quenched with water and diluted with ethyl acetate and separated. The organic was washed with brine and dried over anhydrous sodium sulphate. The organic solvent was removed *in vacuo* and the final product was purified by eluting through a solid phase extraction tube (10 g) with 30% ethyl acetate/hexanes.

### Example 103

### 2-[2,4-Dichloro-5-(pipendine-1-sulphonyl)benzyloxy]-1-(3-methoxymethylphenyl)ethanone (59 mg, pale orange solid, 87% yield)

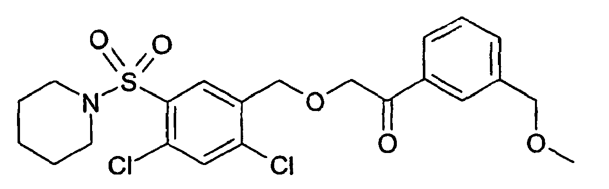

¹H-NMR (CDCl₃) δ: 8.22 (s, 1H), 7.87 (t, 2H), 7.57 (d, 1H), 7.44-7.52 (m, 2H), 4.91 (s, 2H), 4.75 (s, 2H), 4.50 (s, 2H), 3.41 (s, 3H), 3.25 (t, 4H), 1.52-1.61 (m, 6H).

### Example 104

### 2-[2,4-Dichloro-5-(piperidine-1-sulphonyl)benzyloxy]-1-(4-methoxymethylphenyl)ethanone (50 mg, colourless oil, 72% yield)

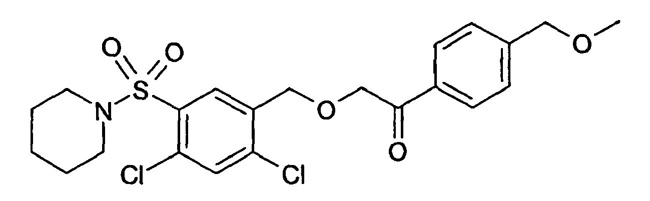

¹H-NMR (CDCl₃) δ: 8.24 (s, 1H), 7.94 (dd, 2H), 7.54 (s, 1H), 7.46 (d, 2H), 4.91 (s, 2H), 4.77 (s, 2H), 4.54 (s, 2H), 3.44 (s, 3H), 3.27 (t, 4H), 1.52-1.63 (m, 6H).

### Example 105

### 2-[2,4-Dichloro-5-(piperidine-1-sulphonyl)benzyloxy]-1-(3-dimethylarninomethylphenyl)ethanone (65 mg, yellow oil, 93% yield)

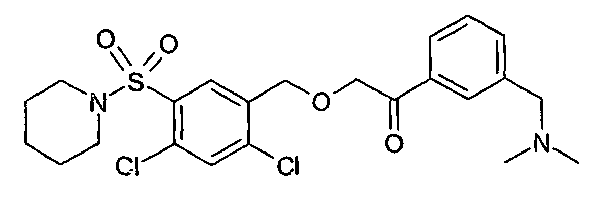

¹H-NMR (CDCl₃) δ: 8.25 (s, 1H), 7.83-7.88 (m, 2H), 7.57 (t, 2H), 7.45 (t, 1H), 7.43 (t, 1H), 4.94 (s, 2H), 4.77 (s, 2H), 3.49 (s, 2H), 3.27 (t, 4H), 2.26 (6H), 1.55-1.63 (m, 6H).

### Example 106

### 2-[2-Chloro-5-(piperidine-1-sulfonyl)benzyloxy]-1-(3-dimethylaminomethylphenyl) ethanone (80 mg, yellow oil, 84% yield)

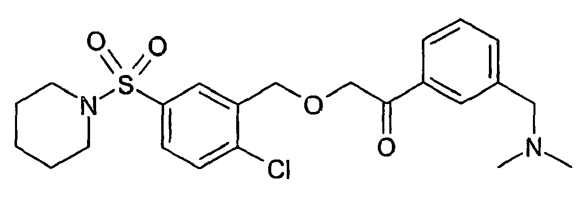

¹H-NMR (CDCl₃) δ: 7.96 (s, 1H), 7.84 (m, 2H), 7.63-7.40 (m, 4H), 4.93 (s, 2H), 4.81 (s, 2H), 3.46 (s, 2H), 2.98 (t, 4H), 2.23 (6H), 1.63 (m, 4H), 1.42 (m, 2H).

### Example 107

### Synthesis of 2-(2-Oxo-2-phenyl-ethoxymethyl)-4-(piperidine-1-sulfonyl)-benzonitrile

To a mixture of 2-[2-bromo-5(piperidine-1-sulphonyl)-benzyloxy]-1-phenyl-ethanone (0.215 g, 0.475 mmol) in N-methylpyrrolidinone (3.0 mL), was added zinc cyanide (0.039 g, 0.333 mmol) and tetrakis(triphenylphosphine)palladium(0) (0.055g, 0.0475 mmol). The reaction flask was sealed and stirred at 80°C overnight. The reaction mixture was cooled to R.T. and extracted with AcOEt (2 x 20 mL). The organic extracts were washed with brine, dried over anhydrous sodium sulfate, filtered and the solvent was removed *in vacuo.* The residue was purified on silica gel to afford 2-(2-oxo-2-phenyl-ethoxymethyl)-4-(piperidine-1-sulfonyl)-benzonitrile as a pale yellow solid (59 mg,31%)

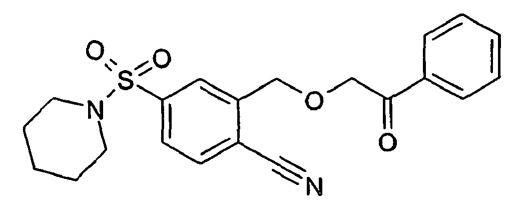

¹H-NMR (CDCl₃) δ: 8.10 (s, 1H), 7.95 (d, 2H), 7.81 (t, 2H), 7.63 (t, 1H), 7.51 (t, 2H), 5.00 (s, 2H), 4.96 (s, 2H), 3.04 (t, 4H), 1.62-1.70 (m, 4H), 1.42-1.47 (m, 2H).

### Example 108

### Synthesis of 4-[2-chloro-5-(piperidine-1-sulfonyl)phenyl]-4-oxobutyraldehyde

To a mixture of 2-chloro-5-chlorosulfonyl benzoic acid (6.66 g, 21.9 mmol) and CH₂Cl₂ (18 ml) were added a few drops of DMF and a CH₂Cl₂ solution of Cl₂C₂O₂ (2 M, 40 ml, 80 mmol) at 0°C. The mixture was stirred at room temperature overnight. The solvent and excess Cl₂C₂O₂ were removed *in vacuo.* To this carbonyl chloride was added dry THF (30 ml) and cooled to -78°C followed by addition of a Grignard solution, generated from BrCH₂CH₂CH(OMe₂)₂ (3.4 ml, 25 mmol), Mg (1.5 g, 60 mmol) and THF (40 ml), rt, 2 hs, over 30 minutes. The mixture was allowed to warm to room temperature overnight with stirring. To this resulting mixture was added HCl aq (10 %, 13 ml) and stirred at room temperature for 2 hours. The resulting mixture was extracted with CH₂Cl₂ (2 x 80 ml) and the combined extract was dried over Na₂SO₄, filtered. The solution was concentrated under reduced pressure to afford a residue which was purified on silica gel column to give the pure product 4-[2-chloro-5-(piperidine-1-sulfonyl)phenyl]-4-oxobutyraldehyde (6.2 g, 91 %)

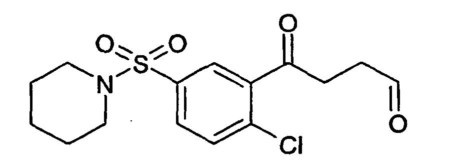

¹H NMR (CDCl₃) δ: 1.46 (m, 2H), 1.64 (m, 4H), 2.95-3.03 (m, 6H), 3.26 (t, 2H), 7.59 (d, 1H), 7.76 (d, 1H), 7.928 (s, 1H), 9.87 (s, 1H).

In a similar fashion the following compound was prepared.

### Example 109

### 4-[2-methyl-5-(piperidine-1-sulfonyl)phenyl]-4-oxobutyraldehyde (330 mg, 50 %)

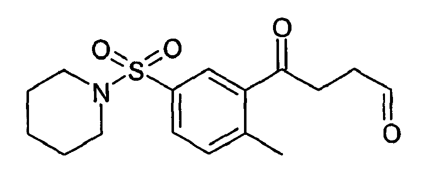

¹H NMR (CDCl₃) δ: 1.46 (m, 2H), 1.66 (m, 4H), 2.57 (s, 3H), 2.97-3.03 (m, 6H), 3.25 (t, 2H), 7.28 (d, 1H), 7.75 (d, 1H), 8.06 (s, 1H), 9.91 (s, 1H).

### Example 110

### Synthesis of 4-[2-chloro-5-(piperidine-1-sulfonyl)phenyl]-butyraldehyde

To a solution of NaBH₄ (114 mg, 3 mmol) in H₂O (2 ml) was added slowly a solution of 4-[2-chloro-5-(piperidine-1-sulfonyl)phenyl]-4-oxobutyraldehyde (171 mg, 0.5 mmol) in THF (3 ml) at 0°C. The mixture was stirred at this temperature for 30 minutes and neutralized with diluted HCl aq (1N) followed by extraction with CH₂Cl₂ (5 ml x 3). The combined extract was dried and the solvent was removed under reduced pressure to leave the corresponding diol as a white solid. To the diol was added CH₂ClCH₂Cl (1.5 ml), Ph₂SiHCl (360 µl, 1.68 mmol) and InCl₃ (18 mg, 0.08 mmol) and the mixture was heated to 80°C for 3 hours. The resulting mixture was treated with diluted HF aq (1 N, 1 ml) at room temperature for 30 minutes and extracted with CH₂Cl₂ (5 ml x 3). After normal work-up, purification on silica gel column afforded a mixture containing unsaturated primary monoalcohol which was hydrogenated with H₂ (1 atm) in the presence of Pd/C (10 %, 15 mg) in EtOH (2 ml) at room temperature for 2 hours to provide pure saturated primary monoalcohol. The alcohol was oxidized with PCC (250 mg, 1 mmol) and CH₂Cl₂ (5 ml) at room temperature for 2 hours. Purification on silica gel gave the final product (54 mg, 33%) after normal work-up.

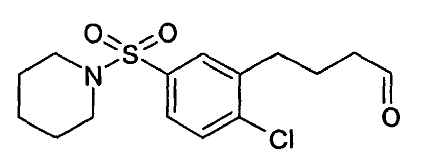

¹H NMR (CDCl₃) δ: 1.45 (m, 2H), 1.67 (m, 4H), 2.00 (m, 2H), 2.55 (t, 2H), 2.85 (t, 2H), 3.00 (t, 4H), 7.50-7.66 (m, 3H), 9.83 (s, 1H).

### General Synthesis of 1-[2-Chloro-5-(piperidine-1-sulfonyl)phenyl]arylbutane-1,4-diones

To a THF solution of aryl bromide (or iodide, 1.15 eqv.) was added n-BuLi (1.6 M, 1.15 eqv.) in hexane at -78°C. The mixture was stirred at this temperature for 1 h. To this was added a solution of 4-[2-chloro-5-(piperidine-1-sulfonyl)-phenyl]-4-oxo-butyraldehyde (1 eqv.) in THF at -78°C and was kept stirring at this temperature for 2 hours. To this resulting mixture was added H₂O (1 ml) at rt. and extracted with AcOEt (3 x 3 ml). The combined extract was dried and concentrated to afford residue. The residue was treated with PCC (3 eqv.) and CH₂Cl₂ at room temperature for 2 hours. The resulting solution was taken and was concentrated to give a residue, which was purified on silica gel column to afford the products.

### Example 111

### 1-[2-Chloro-5-(piperidine-1-sulfonyl)phenyl]-4-naphthalen-2-ylbutane-1,4-dione (50 mg, 36 % yield)

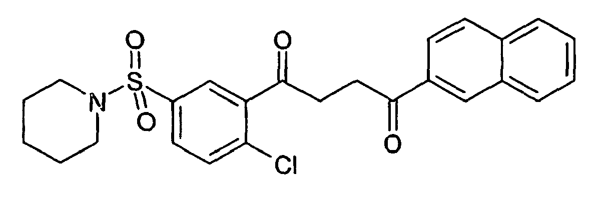

¹H NMR (CDCl₃) δ: 1.455 (m, 2H), 1.672 (m, 4H), 3.044 (t, J = 5.3 Hz, 4H), 3.430 (t, J = 6.2 Hz, 2H), 3.655 (t, J = 6.2 Hz, 2H), 7.596 - 8.045 (m, 9H), 8.559 (s, 1H).

### Example 112

### 1-[2-Chloro-5-(piperidine-1-sulfonyl)phenyl]-4-naphthalen-1-ylbutane-1,4-dione (59 mg, 36 % yield).

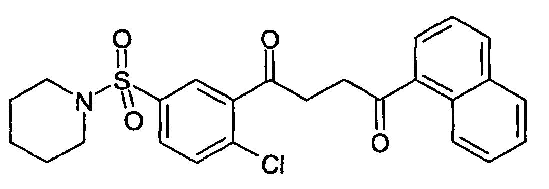

¹H NMR (CDCl₃) δ: 1.437 (m, 2H), 1.639 (m, 4H), 3.045 (t, J = 5.4 Hz, 4H), 3.440 (t, J = 5.7 Hz, 2H), 3.586 (t, J = 5.7 Hz, 2H), 7.515 - 7.627 (m, 4H), 7.7,88 (dd, J = 2.1 Hz, J = 8.2 Hz, 1H), 7.89 (d, J = 7.7 Hz, 1H), 8.010 - 8.073 (m, 3H), 8.603(d, J = 9.0 Hz, 1H).

### Example 113

### 1-[2-Chloro-5-(piperidine-1-sulfonyl)phenyl]-4-phenylbutane-1,4-dione (78 mg, 30 % yield).

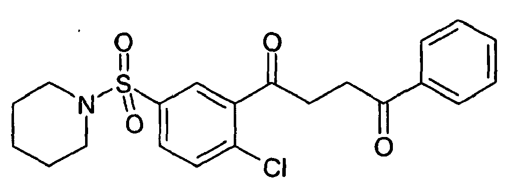

¹H NMR (CDCl₃) δ:1.467 (m, 2H), 1.664 (m, 4H), 3.033 (t, J = 5.4 Hz, 4H), 3.369 (t, J = 5.8 Hz, 2H), 3.511 (t, J = 5.8 Hz, 2H), 7.464-7.610 (m, 4H), 7.772 (dd, J = 2.1 Hz, J = 8.4 Hz, 1H), 8.015(m, 3H).

### Example 114

### 1-[2-Chloro-5-(piperidine-1-sulfonyl)phenyl]-4-pyridin-3-ylbutane-1,4-dione (100 mg, 28 % yield)

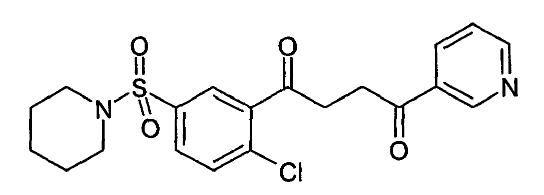

¹H NMR (CDCl₃) δ: 1.465 (m, 2H), 1.658 (m, 4H), 3.027 (t, J = 5.4 Hz, 4H), 3.368 - 3.522 (m, 4H), 7.455 (m, 1H), 7.602 (d, J = 8.4 Hz, 1H), 7.775 (dd, J = 2.1 Hz, J = 8.4 Hz, 1H), 8.015 (d, J = 2.1 Hz, 1H), 8.276 (m, 1H), 8.813 (t, J = 2.4 H, 1H), 9.238 (d, J = 1.8 Hz, 1H).

### Example 115

### 1-[2-Chloro-5-(piperidine-1-sulfonyl)phenyl]-4-(3-dimethylaminomethylphenyl)butane-1,4-dione (38 mg, 18 % yield)

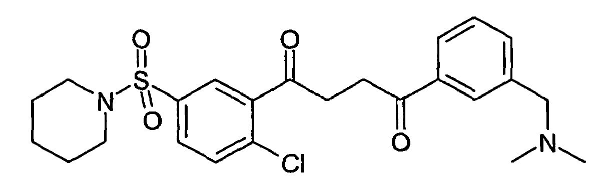

¹H NMR (CDCl₃) δ: 1.462 (m, 2H), 1.643 (m, 4H), 2.251 (s, 6H), 3.028 (t, J = 5.3 Hz, 4H), 3.335 - 3.535 (m, 6H), 7.436 (t, J = 7.6 Hz, 1H), 7.539 - 7.605 (m, 2H), 7.765 (dd, J = 2.4 Hz, J = 8.6 Hz, 1H), 7.897 (d, J = 7.6 Hz, 1H), 7.934 (s, 1H), 8.009 (d, J = 2.4 Hz, 1H).

### Example 116

### 4-[2-chloro-5-(piperidine-1-sulfonyl)phenyl]-1-phenyl-butan-1-one (26 mg, 70 % yield)

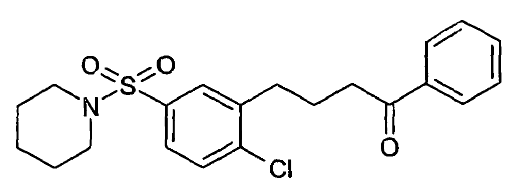

¹H NMR (CDCl₃) δ: 1.45(m, 2H), 1.67 (m, 4H), 2.13 (m, 2H), 2.90-3.28 (m, 8H), 7.28-7.66(m, 6H), 7.96 (d, 2H).

### Example 117

### 4-[2-chloro-5-(piperidine-1-sulfonyl)phenyl]-1-pyridine-3-yl-butan-1-one (7.4 mg, 11 %)

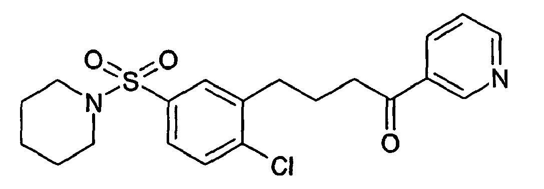

¹H NMR (CDCl₃) δ: 1.46(m, 2H), 1.68 (m, 4H), 2.153 (m, 2H), 2.91-3.13(m, 8H), 7.47-7.71(m, 4H), 8.24 (d, 1H), 8.83 (broad, 1H), 9.09 (broad, 1H).

The following compounds (Examples 118 -120) were prepared in a similar fashion to that stated above with the exception that in the final oxidation step KMnO₄, (1 eqiv.), acetone (3 ml) and H₂O (1 ml) were used at 0°C for oxidation.

### Example 118

### 1-[2-Chloro-5-(piperidine-1-sulfonyl)phenyl]-4-pyridin-2-ylbutane-1,4-dione (50 mg, 12 % yield)

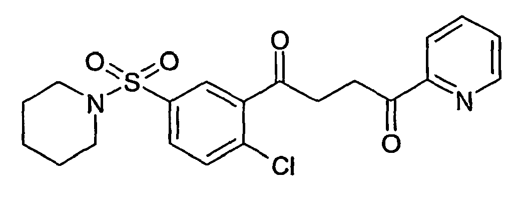

¹H NMR (CDCl₃) δ : 1.48 (m, 2H), 1.66 (m, 4H), 3.04 (t, 4H), 3.39 (t, 2H), 3.74 (t, 2H), 7.56-8.65 (m, 6H), 8.74 (d, 1H).

### Example 119

### 1-[2-Methyl-5-(piperidine-1-sulfonyl)phenyl]-4-pyridin-2-ylbutane-1,4-dione (25 mg, 18 % yield)

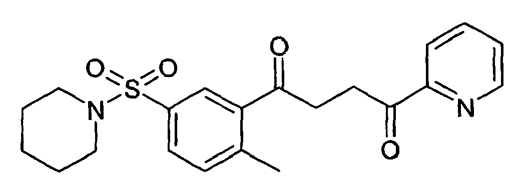

¹H NMR (CDCl₃) δ: 1.45 (m, 2H), 1.67 (m, 4H), 2.56 (s, 3H), 3.02(t, 4H), 3.37 (t, 2H), 3.72 (t, 2H), 7.42-7.53(m, 2H), 7.73-7.89(m, 2H), 8.04 (d, 1H), 8.12 (s, 1H), 8.74 (d, 1H).

### Example 120

### 4-[2-chloro-5-(piperidine-1-sulfonyl)phenyl]-1-pyridine-2-yl-butan-1-one (19 mg, 28 % yield)

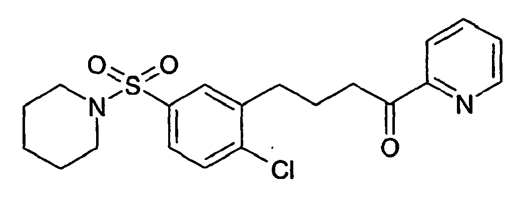

¹H NMR (CDCl₃) δ: 1.45(m, 2H), 1.65(m, 4H), 2.91-3.00 (m, 6H), 3.35 (t, 2H), 7.49-7.53(m, 3H), 7.67 (s, 1H), 7.87 (d, H), 8.05 (d, 1H), 8.71 (d, 1H).

### Example 121

### Synthesis of 1-(4-chloro-3-(5-thiophene-2-yl-(1,2,4)oxadiazol-3-yl methoxy methyl)-benzene sulfonyl)-piperidine

To a solution of (2-chloro-5-(piperidine-1-sulfonyl)-phenyl)methanol (54 mg, 0.18 mmol) in THF (1.5 ml) was added NaH (60% in oil, 20 mg, 0.5 mmol) at 0°C and kept stirring at this temperature for 40 minutes. To this reaction mixture was added a solution of 3-chloromethyl-5-thiophen-2-yl-(1,2,4) oxadoazole (30 mg, 0.15 mmol) in THF (1 ml) at 0°C and stirred at room temperature for 150 minutes. To this resulting mixture was added H₂O (1 ml) at 0°C and extracted with CH₂Cl₂ (5 ml x 3). The combined extract was dried and the solvent was removed with rotary evaporator to give residue, which was purified on silica gel column to afford pure product (65 mg, 96 %)

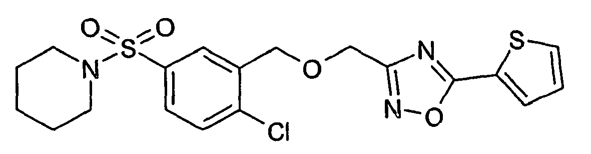

¹H NMR (CDCl₃) δ: 1.45 (m, 2H), 1.65 (m, 4H), 3.01 (t, 4H), 4.85 (s, 4H), 7.50 (m, 2H), 7.66 (d, 1H), 7.72 (m, 1H), 7.99 (s, 1H), 8. 30 (d, 1H).

### Example 122

### Synthesis of (3-(3-benzenesulfinyl-propenyl)-4-chloro-benzenesulfonyl)-piperidine

To a solution of 2-chloro-5-(piperidine-1-sulfonyl)-benzaldehyde (86 mg, 0.3 mmol) in THF (2 ml) was added vinylmagnesium bromide (1M, 0.4 ml, 0.4 mmol) in THF at - 60°C and allowed to warm to room temperature over 1 h. To this resulting mixture was added a saturated NH₄Cl aq (1 ml) and extracted with CH₂Cl₂ (5 ml x 3). The combined extract was dried and the solvent was removed with rotary evaporator to leave residue. To this was added Cl₂SO (118 mg, 1 mmol) and Et₂O (3 ml) at 0°C and stirred at room temperature overnight. After standard work-up, purification on silica gel column afforded a mixture containing regioisomers. The isomers were heated at 110°C overnight in DMF (1.5 ml) and cooled to room temperature. To this were added KI (50 mg, 0.3 mmol), HSPh (40 mg, 0.36 mmol) and K₂CO₃ (50 mg, 0.36 mmol) and stirred at room temperature overnight. Standard work-up and purification on silica gel column gave the intermediate sulfide, which was oxidized with 3-chloroperoxybenzoic acid (52 mg, 0.3 mmol) in CH₂Cl₂ (2 ml) at room temperature overnight. The CH₂Cl₂ was removed and the residue was purified on silica gel column to provide final product (79 mg, 47%)

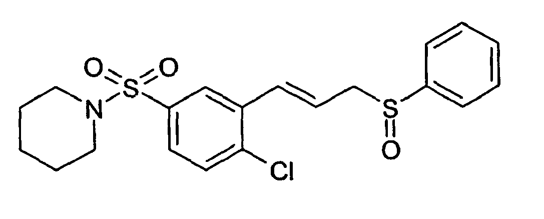

¹H NMR (CDCl₃) δ: 1.46 (m, 2H), 1.68 (m, 4H), 3.01 (t, 4H), 3.70-3.84 (m, 2H), 6.10 (m, 1H), 6.85 (d, 1H) 7.28-7.72(m, 7H), 8.09(s, 1H).

### Example 123

### Synthesis of 2-[2-Chloro-5-(piperidine-1-sulfonyl)benzyloxy]-1-pyrrolo[2,3-b]pyridin-1-ylethanone

A mixture of (125 mg, 0.36 mmol), CH₂Cl₂ solution of oxalyl chloride (2 M, 0.8 ml, 1.6 mmol) and CH₂Cl₂ (2.5 ml) was heated to 50°C for 2 hours. The excess oxalyl chloride and solvent were removed *in vacuo* to afford solid acid chloride, which was diluted with dry THF (2.5 ml) and cooled to -50°C. To this solution was added a mixture, generated from 1H-pyrrolo[2,3-b] pyridine (85 mg, 0.72 mmol), sodium (hexamethyldisilyl)amide (1 M, 0.6 ml, 0.6 mmol), THF (2 ml) at 0°C for 30 minutes, at - 50°C. The mixture was allowed to warm to room temperature overnight. To this resulting mixture was added H₂O (1 ml) and extracted with AcOEt (3 x 5 ml). The combined extract was dried and the solvent was removed *in vacuo* to give a residue, which was purified on silica gel column to afford the pure product (50.7 mg, 31 %)

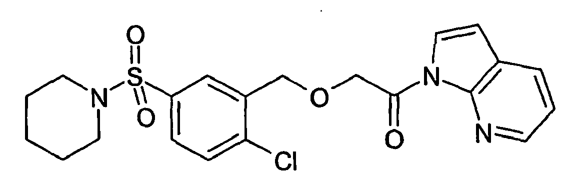

¹H NMR (CDCl₃) δ: 1.431 (m, 2H), 1.656 (m, 4H), 3.027 (t, J = 5.6 Hz, 4H), 4.971 (s, 2H), 5.447 (s, 2H), 6.688 (d, J = 4.2 Hz, 1H), 7.258 (m, 1H), 7.535 (d, J = 8.1 Hz, 1H), 7.663 (dd, J = 2.1 Hz, J = 8.1 Hz, 1H), 7.920 (dd, J = 2.1 Hz, J = 8.1 Hz, 1H), 8.030 (d, J = 3.9 Hz, 1H), 8.082 (s, 1H), 8.365 (d, J = 5.1 Hz, 1H).

### Example 124

### Synthesis of 2-[2-Chloro-5-(piperidine-1-sulfonyl)benzylamino]-1-piperidin-1-ylethanone

To a mixture of the N-Boc-glycine (350 mg, 2 mmol), and HOBt (297 mg, 2.2 mmol) in DMF(10 mL) was added piperidine (187 mg, 2.2 mmol) and EDCI (422 mg, 2.2 mmol) at 0°C. The mixture was allowed to warm slowly to room temperature overnight (18hrs). The mixture was diluted with ethyl acetate (300 mL) and washed with H₂O (3x 250 mL) followed by brine. The organic extract was dried over Na₂SO₄ (anhydrous) and concentrated *in vacuo* giving a product which pure enough to use in the next step.

To the above product, dissolved in CH₂Cl₂ (5 mL) at 0°C, was added slowly trifluoroacetic acid (5 mL). The mixture was stirred at this temperature for 1 hr and then poured into cold NaHCO₃ (saturated) and the neutralised mixture was then extracted with CH₂Cl₂. The organic extract was washed with H₂O (3 x 250 mL) followed by brine. The organic extract was dried over Na₂SO4 (anhydrous) and concentrated *in vacuo* and the residue was purified by silica gel flash chromatography giving the 2-amino-1-piperidin-1-ylethanone as a colourless oil (82 mg, 29% overall yield).

To a solution of the 2-chloro-5-(piperidine-1-sulfonyl)benzaldehyde and 2-amino-1-piperidin-1-ylethanone in dichloroethane at room temperature was added NaBH(OAc)₃ and the mixture was allowed to stir at room temperature overnight. The mixture was then diluted with ethyl acetate and the washed with NaHCO₃ (saturated). The organic layer was then washed with brine and then dried over Na₂SO₄ (anhydrous) and the organic solvent was removed *in vacuo.* The residue was purified by flash chromatography on silica gel using hexane/ethyl acetate (0-100%) as eluant giving 62 mg (62% yield) of product as a colourless oil

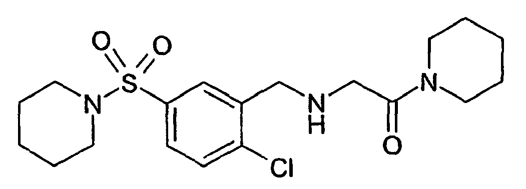

¹H-NMR (CDCl₃) δ: 7.90 (d, 1H), 7.59 (dd, 1H), 7.51 (d, 1H), 3.98 (s, 2H), 3.58 (t, 2H), 3.46 (s, 2H), 3.30 (t, 2H), 3.00 (t, 4H), 2.06 (br, 1H), 1.69-1.50 (m, 10H), 1.42 (m, 2H).

The foregoing examples are meant to illustrate the invention and are not to be construed to limit the invention in any way.

## Claims

1. A compound of formula I, a pharmaceutically acceptable salt thereof, diastereomers, enantiomers, or mixtures thereof: 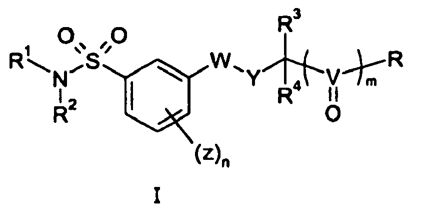 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 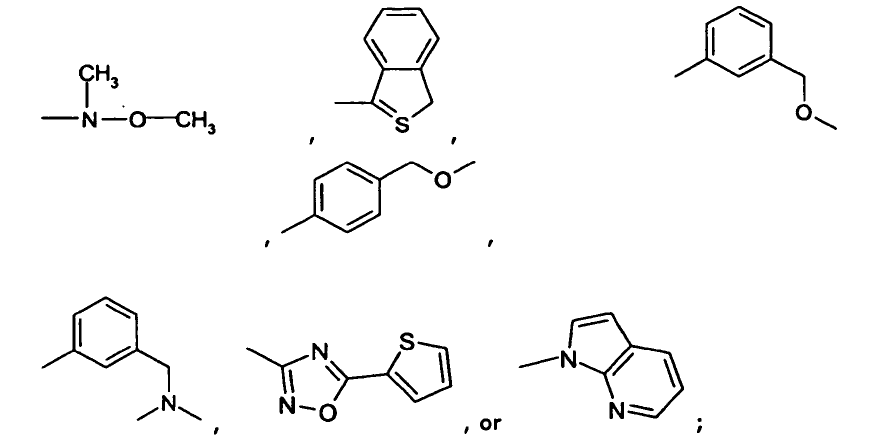
R¹ and R² are each independently C₁₋₆ alkyl, or R¹, R², and the N to which they are attached, in combination, are 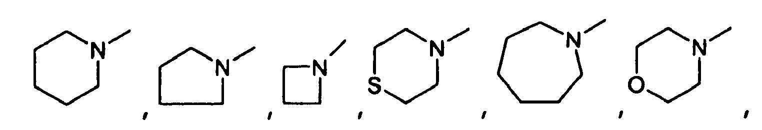 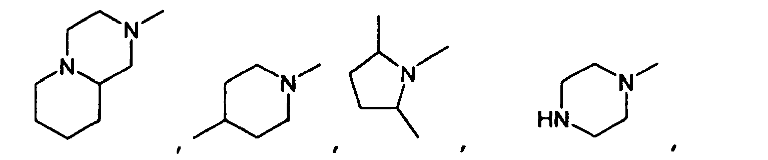 or 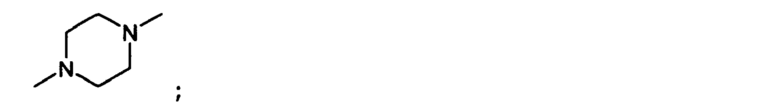
W is CH₂ or C=O;
Y is CH₂, NH, NCH₃, or O;
V is C;
m is 1;
n is 0, 1, 2, 3, or 4;
Z is, at each position, independently, Cl, F, methoxy, or methyl; and
R³ and R⁴ are each, independently, H, methyl, or ethyl;
with the proviso that the compound is not
2-methyl-5-(4-morpholinylsulfonyl)-benzoic acid, 2-oxo-2-phenylethyl ester;
4-fluoro-3-(4-morpholinylsulfonyl)-benzoic acid, 2-oxo-2-phenylethyl ester;
4-methoxy-3-(4-morpholinylsulfonyl)-benzoic acid, 2-oxo-2-phenylethyl ester;
4-methyl-3-(4-morpholinylsulfonyl)-benzoic acid, 2-oxo-2-phenylethyl ester;
2-chloro-5-(1-pyrrolidinylsulfonyl)-benzoic acid, 2-oxo-2-phenylethyl ester;
4-chloro-3-(1-piperidinylsulfonyl)-benzoic acid, 2-oxo-2-phenylethyl ester;
4-chloro-3-[(hexahydro-1H-azepin-1-yl)sulfonyl]-benzoic acid, 2-oxo-2-phenylethyl ester;
2,4-dichloro-5-(1-piperidinylsulfonyl)-benzoic acid, 2-oxo-2-phenylethyl ester;
4-methyl-3-(1-piperidinylsulfonyl)-benzoic acid, 2-oxo-2-phenylethyl ester;
2-chloro-5-(1-piperidinylsulfonyl)-benzoic acid, 2-oxo-2-phenylethyl ester;
2,4-dichloro-5-(4-morphoinylsulfonyl)-benzoic acid, 2-oxo-2-phenylethyl ester;
4-chloro-3-(4-morpholinylsulfonyl)-benzoic acid, 2-oxo-2-phenylethyl ester;
2-chloro-5-(4-morpholinylsulfonyl)-benzoic acid, 2-oxo-2-phenylethyl ester; and
3-(4-morpholinylsulfonyl)-benzoic acid, 2-oxo-2-phenylethyl ester.

2. The compound of claim 1, wherein
R¹, R², and the N to which they are attached, in combination, are

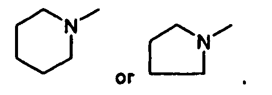

3. The compound of claim 1, wherein Z is 2-chloro.

4. A compound according to any of claims 1-3, said compound being selected from:
2,4-dichloro-5-(piperidine-1-sulfonyl)-benzoic acid 2-oxo-2-phenyl-ethyl ester;
2,4-dichloro-5-(pyrrolidine-1-sulfonyl)-benzoic acid 2-oxo-2-phenyl-ethyl ester;
5-(azetidine-1-sulfonyl)-2,4-dichloro-benzoic acid 2-oxo-2-phenyl-ethyl ester;
2,4-dichloro-5-(thiomorpholine-4-sulfonyl)benzoic acid 2-oxo-2-phenylethyl ester;
2,4-dichloro-5-diethylsulfamoylbenzoic acid 2-oxo-2-phenylethyl ester;
5-(azepane-1-sulfonyl)-2,4-dichlorobenzoic acid 2-oxo-2-phenelethyl ester;
2-methoxy-5-(piperidine-1-sulfonyl)benzoic add 2-oxo-2-phenylethyl ester;
2-chloro-5-(piperidine-1-sulfonyl)benzoic acid 2-oxo-2-phenylethyl ester;
2-chloro-4-fluoro-5-(piperidine-1-sulphonyl)benzoic acid 2-oxo-phenylethyl ester;
2-chloro-5-(piperidine-1-sulfonyl)benzoic acid 2-(2,5-dimethoxyphenyl)-2-oxoethyl ester;
2-chloro-5-(piperidine-1-sulfonyl)benzoic acid 2-(3-methoxyphenyl)-2-oxoethyl ester;
2-chloro-5-(piperidine-1-sulfonyl)benzoic acid 2-(3-chlorophenyl)-2-oxoethyl ester;
2-methyl-5-(piperidine-1-sulfonyl)benzoic acid 2-oxo-2-phenylethyl ester;
2-fluoro-5-(piperidine-1-sulfonyl)benzoic acid 2-oxo-2-phenylethyl ester;
2-chloro-5-(piperidine-1-sulfonyl)benzoic acid 2-oxo-2-pyridin-2-ylethyl ester;
2-chloro-5-(piperidine-1-sulphonyl)benzoic Acid 1-methyl-2-oxo-2-phenylethyl ester;
2-chloro-5-(piperidine-1-sulphonyl)benzoic acid 2-(4-fluorophenyl)-2-oxoethyl ester;
2-chloro-5-(piperldine-1-sulphonyl)benzoic acid 2-oxo-2-p-tolylethyl ester;
2-chloro-5-(piperidine-1-sulphonyl)benzoic acid 2-oxobutyl ester;
2-chicro-5-(piperidine-1-sulphonyl)benzoic acid 2-naphthalen-2-yl-2-oxoethyl ester;
2-chloro-5-(piperidine-1-sulphonyl)benzoic acid 2-benzo[b]thiophen-3-yl-2-oxoethyl ester;
2-chloro-5-(piperldine-1-sulphonyl)benzoic acid 3,3-dimethyl-2-oxobutyl ester;
2-chloro-5-(morpholine-4-sulphonyl)benzoic acid 2-oxo-2-phenylethyl ester;
5-(butylethylsulphamoyl)-2-chlorobenzoic acid 2-oxo-2-phenylethyl ester;
2-chloro-5-dimethylsulphamoylbenzoic acid 2-oxo-2-phenylethyl ester;
2-chloro-5-(octahydropyrido[1,2-a]pyrazine-2-sulphonyl)benzoic acid 2-oxo-2-phenylethyl ester;
2-chloro-5-(4-methylpiperidine-1-sulphonyl)benzoic add 2-oxo-2-phenylethyl ester;
2-chloro-5-(2,5-dimethylpyrollidine-1-sulphonyl)benzoic acid 2-oxo-2-phenylethyl ester;
3-(piperidine-1-sulphonyl)benzoic acid 2-oxo-2-phenylethyl ester;
N-2-oxo-2-phenethyl 2,4-dichloro-5-[piperidinosulfonyl] benzamide;
2-chloro-4-fluoro-N-(2-oxo-2-phenylethyl)-5-(piperidine-1-sulphonyl) benzamide;
2-oxo-2-phenethyl 2,4-dichloro-5-[piperazinosulfonyl] benzoate hydrochloride;
2-oxo-2-phenethyl 2,4-dichloro-5-[4-methyl-piperazinosulfonyl] benzoate hydrochloride;
2,4-dichloro-5-[piperidiinosulfonyl]benzyloxyacetophenone;
2-chloro-5-(piperidine-1-sulphonyl)benzoic acid 1,1-dimethyl-2-oxo-2-phenylethyl ester;
(2-chloro-5-(piperidine-i-sulphonyl)benzylamino]acetic acid tert-butyl ester;
{[2-chloro-5-(piperidine-1-sulfonyl)-benzyl]-methyl-amino}-acetic acid tert-butyl ester;
(2-chloro-5-(piperidine-1-sulphonyl)benzyloxy)acetic acid tert-butyl ester;
2-[2-chloro-5-(piperidine-1-sulfonyl)benzyloxy]propionic add tert-butyl ester;
2-[2-chloro-5-(piperidine-1-sulfonyl)benzyloxy]butyric acid tert-butyl ester;
2-(2-chloro-5-(piperidine-1-sulphonyl)benzyloxy)-1-pyrrolidin-1-ylethanone;
2-(2-chloro-5-(piperidine-1-sulphonyl)benzyloxy)-N-methyl-N-phenylacetamide;
N-tert-butyl-2-(2-chloro-5-(piperidine-1-sulphonyl)benzyloxy)acetamide;
(1-(2-(2-chloro-5-piperidine-1-sulphonyl)benzyloxy)acetyl)pyrrolidin-3-yl)carbamic acid tert-butyl ester;
(1-(2-(2-chloro-5-(piperidine-1-sulphonyl)benzyloxy)acetyl)piperidin-4-yl)carbamic acid tert-butyl ester;
2-[2-chloro-5-(piperidine-1-sulfonyl)-benzyloxy]-N-pyridin-2-yl-acetamide;
2-[2-chloro-5-piperidine-1-sulphonyl)benzyloxy]-1-piperidin-1-ylethanone;
2-[2-chloro-5-(piperidine-1-sulfonyl)-benzyloxy]-N-methyl-N-pyridin-2-yl-acetamide;
2-(2,4-dichloro-5-(piperidine-1-sulphonyl)benzyloxy)-N-methoxy-N-methylacetamide;
2-(2-chloro-5-(plperidine-1-sulphonyl)benzyloxy)-N-methoxy-N-methylacetamide;
N-methoxy-2-(2-methoxy-5-(piperidine-1-sulphonyl)-benzyloxy)-N-methyl-acetamide;
N-methoxy-2-(2-methoxy-5-(piperidine-1-sulphonyl)-benzyloxy)-N-methylacetamide;
2-[2-bromo-5-(piperidine-1-sulphonyl)-benzyloxy)-N-methoxy-N-methylacetamide;
N-methoxy-N-methyl-2-[5-(piperidine-1-sulphonyl)-2-trifluoromethoxy-benzyloxy]-acetamide;
2-(1-(2-chloro-5-(piperidine-1-sulfonyl)-phenyl)-ethoxy)-N-methoxy-N-methyl-acetamide;
2-[2-chloro-5-(thiomorpholine-4-sulfonyl)-benzyloxy]-N-methoxy-N-methylacetamide;
2-[2-chloro-5-(piperidine-1-sulphonyl)benzyloxy]-1-(3-methoxyphenyl)ethanone;
2-[2-chloro-5-(piperidine-1-sulphonyl)benzyloxy]-1-cyclohexylethanone;
2-[2-chloro-5-(piperidine-1-sulfonyl)benzyloxy]-1-(2,5-dimethoxyphenyl)ethanone;
2-[2,4-dichloro-5-(piperidine-1-sulphonyl)benzyloxy]-1-(3-methoxyphenyl)ethanone;
1-(3-chlorophenyl)-2-[2-chloro-5-(piperidine-1-sulphonyl)benzyloxy]-ethanone;
1-(3-chlorophenyl)-2-[2,4-dichloro-5-(piperidine-1-sulphonyl)benzyloxy]-ethanone;
2-[2,4-dichloro-5-(piperidine-1-sulphonyl)benzyloxy]-1-naphthalen-2-ylethanone;
2-[2-chloro-5-(piperidine-1-sulphonyl)benzyloxy]-1-naphthalen-2-ylethanone;
4-chloro-N,N-dimethyl-5-(2-oxo-2-phenylethoxymethyl)benzenesulphonamide;
4-chloro-N,N-diethyl-3-(2-oxo-2-phenylethoxymethyl)benzenesulphonamide;
4-chloro-N-ethyl-N-methyl-3-(2-oxo-2-phenylethoxymethyl)benzenesulphonamide;
4-chloro-N-methyl-3-(2-oxo-2-phenylethoxymethyl)-N-propyl-benzenesulphonamide;
2-[2-bromo-5(piperidine-1-sulphonyl)-benzyloxy]-1-phenyl-ethanone;
2-[2-chloro-5-(thiomorpholine-4-sulfonyl)-benzyloxy]-1-phenyl-ethanone;
2-(2-chloro-5-(piperidine-1-sulfonyl)-benzyloxyl)-1-(1H-pyrrolo(2,3-b)-pyridi n-3-yl)-ethanone;
2-[2-chloro-5-(4-methylpiperazine-1-sulfonyl)benzyloxy]-1-pyridin-2-yl-ethanone;
2-[2-chloro-5-(piperidine-1-sulphonyl)benzyloxy]-1-pyridin-3-ylethanone;
2-[2-chloro-5-(piperidine-1-sulphonyl)benzyloxy]-1-pyridin-4-ylethanone;
2-[2-chloro-5-(piperidine-1-sulphonyl)benzyloxy]-1-pyridin-2-ylethanone;
4-chloro-N,N-dimethyl-3-(2-oxo-2-pyridin-3-ylethoxymethyl)benzenesulphonamide;
2-[2-chloro-5-(piperidine-1-sulphonyl)benzyloxy]-1-quinolin-8-ylethanone;
4-chloro-N,N-dimethyl-3-(2-oxo-2-pyridin-2-ylethoxymethyl)benzenesulphonamide;
4-chloro-N-ethyl-N-methyl-3-(2-oxo-2-pyridin-2-ylethoxymethyl)benzenesulphonamide;
4-chloro-N,N-diethyl-3-(2-oxo-2-pyridin-2-ylethoxymethyl)benzenesulphonamide;
4-chloro-N-methyl-3-(2-oxo-2-pyridin-2-ylethoxymethyl)-N-propyl-benzenesulphonamide;
4-chloro-N-ethyl-N-methyl-3-(2-oxo-2-pyridin-3-ylethoxymethyl)benzenesulphonamide;
4-chloro-N,N-diethyl-3-(2-oxo-2-pyridin-3-ylethoxymethyl)benzenesulphonamide;
2-[2-chloro-5-(piperidine-1-sulpfonyl)-benzyloxy]-1-(4-methyl-pyridin-2-yl)-ethanone;
2-[2-chloro-5-(piperidine-1-sulpfonyl)-benzyloxy]-1-(6-methyl-pyridin-2-yl)-ethanone;
2-[2-chloro-5-(piperidine-1-sulpfonyl)-benzyloxy]-1-(5-methyl-pyridin-2-yl)-ethanone;
4-chloro-N-ethyl-N-methyl-3-[2-(4-methyl-pyridin-2-yl)-2-oxo-ethoxymethyl]-benzenesulfonamide;
4-chloro-N-ethyl-N-methyl-3-[2-(6-methyl-pyridin-2-yl)-2-oxo-ethoxymethyl]-benzenesulfonamide;
4-chloro-N-ethyl-N-methyl-3-[2-(5-methyl-pyridin-2-yl)-2-oxo-ethoxymethyl] - benzenesulfonamide;
2-[2-chloro-5-(piperidine-1-sulpfonyl)-benzyloxy]-1-(3-methyl-pyridin-2-yl)-ethanone;
4-chloro-N-ethyl-N-methyl-3-[2-(3-methyl-pyridin-2-yl)-2-oxo-ethoxymethyl]-benzenesulfonamide;
1-(2-bromo-5-dimethylamino-pyridin-4-yi)-2-[2-chloro-5-(piperidine-1-sulpfonyl)-benzyloxy]-ethanone;
2-[2-methoxy-5-(piperidine-1-sulpfonyl)-benzyloxy]-1-pyridin-2-yl-ethanone;
2-[2-methyl-5-(piperidine-1-sulpfonyl)-benzyloxy]-1-pyridin-2-yl-ethanone;
2-5-(piperidine-1-sulpfonyl)-2-trifluoromethoxy-benzyloxy]-1-pyridin-2-yl-ethanone;
2-(1-(2-chloro-5-(piperidine-1-sulfonyl)-phenyl)-ethoxy)-1-pyridine-2-yl-ethanone;
2-(1-(2-chloro-5-(piperidine-1-sulfonyl)-phenyl)-ethoxy)-1-pyridine-3-yl-ethanone;
2-(2-chloro-5-(piperidine-1-sulfonyl)-benzyloxy)-1-pyrazine-2-yl-ethanone;
2-(2-chloro-5-(piperidine-1-sulfonyl)-benzyloxy)-1-(1-methyl-1H-imidazol-2-yl)-ethanone;
2-(2-chloro-5-(piperidine-1-sulfonyl)-benzyloxy)-1-(1-pyridin-2-yl-1H-imidazol-2-yl)ethanone;
2-(2-chloro-5-(piperldine-1-sulfonyl)-benzyloxy)-1-(1-methyl-1H-indol-5-yl)-ethanone;
2-[2-chloro-5-(piperidine-1-sulfonyl)benzyloxy]-1-pyridin-2-yl-propan-1-one;
2-[2,4-dichloro-5-(piperidine-1-sulphonyl)benzyloxy]-1-(3-methoxymethylphenyl)ethanone;
2-[2,4-dichloro-5-(piperidine-1-sulphonyl)benzyloxy]-1-(4-methoxymethylphenyl)ethanone;
2-[2,4-dichloro-5-(piperidine-1-sulphonyl)benzyloxy]-1-(3-dimethylaminomethylphenyl)ethanone;
2-[2-chloro-5-(piperidine-1-sulfonyl)benzyloxy]-1-(3-dimethylaminomethylphenyl) ethanone;
2-(2-Oxo-2-phenyl-ethoxymethyl)-4-(piperidine-1-sulfonyl)-benzonitrile;
4-[2-chloro-5-(piperidine-1-sulfonyl)phenyl]-4-oxobutyraldehyde;
4-[2-methyl-5-(piperidine-1-sulfonyl)phenyl]-4-oxobutyraldehyde;
4-[2-chloro-5-(piperidine-1-sulfonyl)phenyl]-butyraldehyde;
1-[2-chloro-5-(piperidine-1-sulfonyl)phenyl]-4-naphthalen-2-ylbutane-1,4-dione;
1-[2-chloro-5-(piperidine-1-sulfonyl)phenyl]-4-naphthalen-1-ylbutane-1,4-dione;
1-[2-chloro-5-(piperidine-1-sulfonyl)phenyl]-4-phenylbutane-1,4-dione;
1-[2-chloro-5-(piperidine-1-sulfonyl)phenyl]-4-pyridin-3-ylbutane-1,4-dione;
1-[2-chloro-5-(piperidine-1-sulfonyl)phenyl]-4-(3-dimethylaminomethylphenyl)butane-1,4-dione;
4-[2-chloro-5-(piperidine-1-sulfonyl)phenyl]-1-phenyl-butan-1-one;
4-[2-chloro-5-(piperidine-1-sulfonyl)phenyl]-1-pyridine-3-yl-butan-1-one;
1-[2-chloro-5-(piperidine-1-sulfonyl)phenyl]-4-pyridin-2-ylbutane-1,4-dione;
1-[2-methyl-5-(piperidine-1-sulfony)phenyl]-4-pyridin-2-ylbutane-1,4-dione;
4-[2-chloro-5-(piperidine-1-sulfonyl)phenyl]-1-pyridine-2-yl-butan-1-one;
1-(4-chloro-3-(S-thiophene-2-yl-(1,2,4)oxadiazol-3-yl methoxy methyl)-benzene sulfonyl)-piperidine;
(3-(3-benzenesulfinyl-propenyl)-4-chtoro-benzenesulfonyl)-piperidine;
2-[2-chtoro-5-(piperidine-1-sulfonyl)benzyloxy]-1-pyrrolo[2,3-b]pyridin-1-ylethanone; and
2-[2-chloro-5-(piperidine-1-sulfonyl)benzylamino]-1-piperidin-1-ylethanone.

5. A compound according to any one of claims 1 - 4 for use as a medicament.

6. The use of a compound according to any one of claims 1 - 4 in the manufacture of a medicament for the therapy of neurological and psychiatric disorders associated with glutamate dysfunction.

7. A method for preparing a compound of formula IV 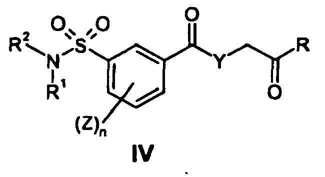 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 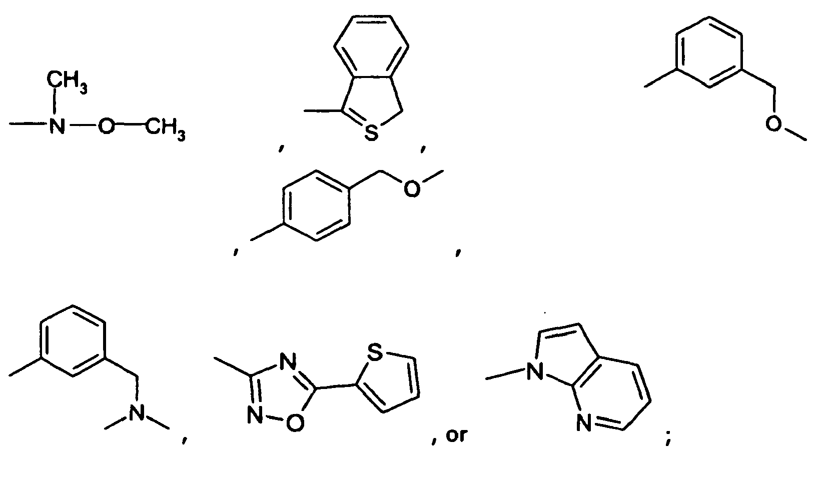
R¹ and R² are each independently C₁₋₆ alkyl, or R¹, R², and the N to which they are attached, in combination, are 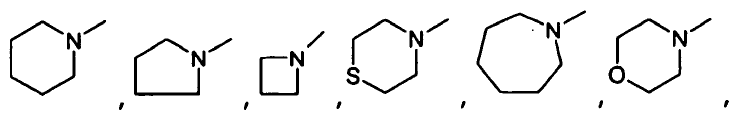 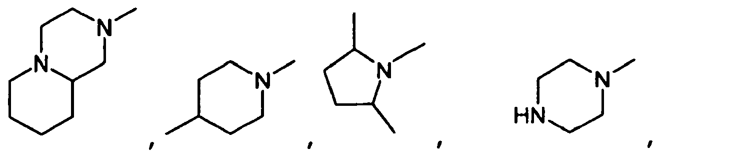 or 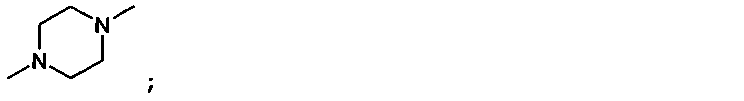
Y is CH₂, NH, NCH₃, or O;
Z is, at each position, independently, Cl, F, methoxy, or methyl; and
n is 0, 1, 2, 3, or 4;
said method comprising:
a) reacting a compound of the formula 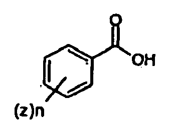 with chlorosulphonic acid to provide a compound of formula II 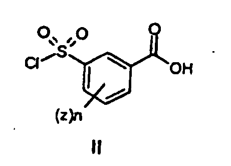
b) reacting the compound of formula II with an amine to provide a compound of formula III 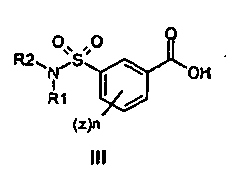 and
c) alkylating the compound of formula III to give a compound of formula IV.

8. A method for preparing a compound of formula VI 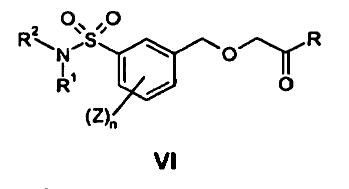 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 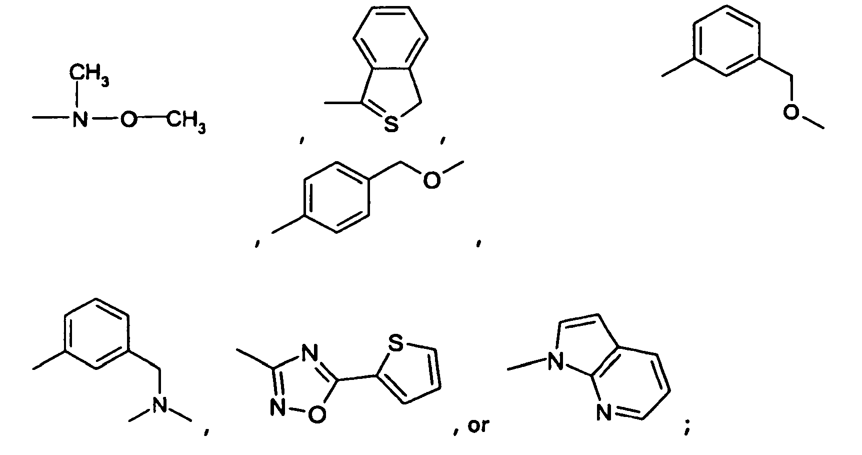
R¹ and R² are each independently C₁₋₆ alkyl, or R¹, R², and the N to which they are attached, in combination, are 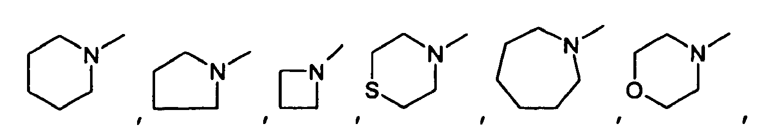 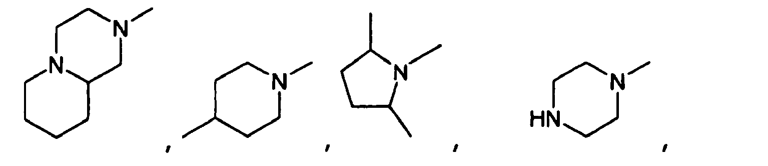 or 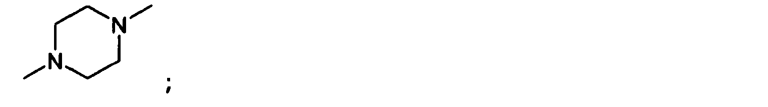
Z is, at each position, independently, Cl, F, methoxy, or methyl; and n is 0, 1, 2, 3, or 4; and
said method comprising:
a) reducing a compound of formula III as defined above to a compound of formula V 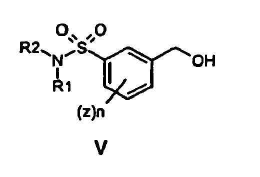 ; and
b) alkylating the compound of formula V to obtain a compound of formula VI.

9. A method for preparing a compound of formula IX 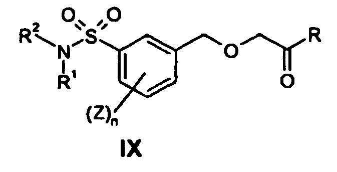 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 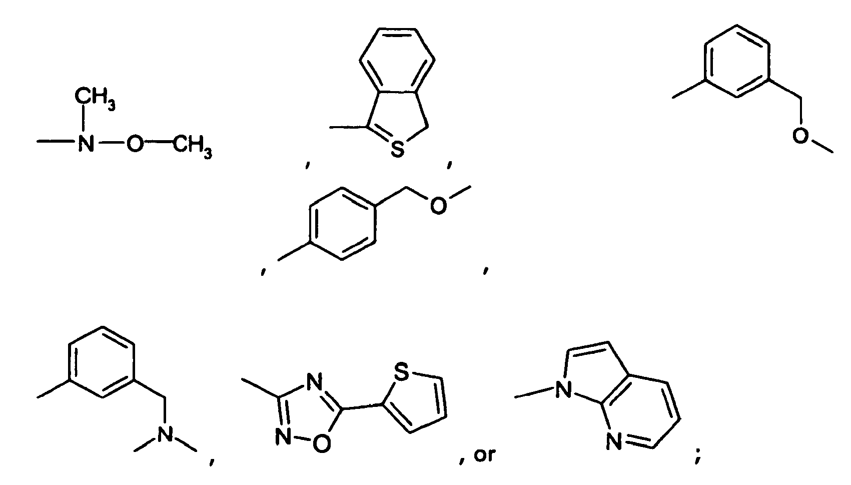
R¹ and R² are each independently C₁₋₆ alkyl, or R¹, R², and the N to which they are attached, in combination, are 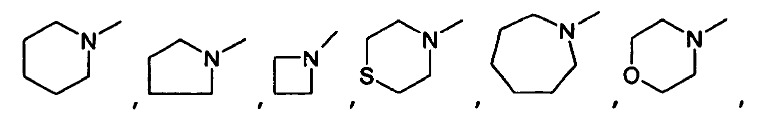 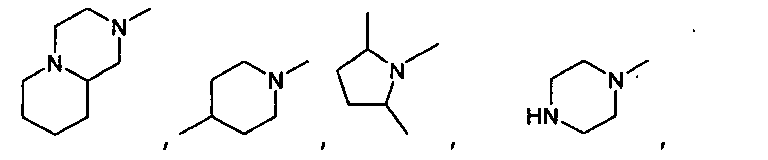 or 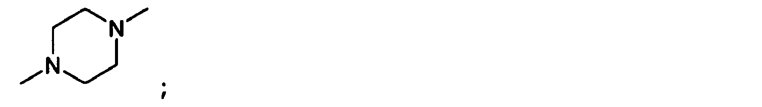
Z is, at each position, independently, Cl, F, methoxy, or methyl; and n is 0, 1, 2, 3, or 4;
said method comprising:
a) alkylating a compound of formula V as defined above to provide a compound of formula VII 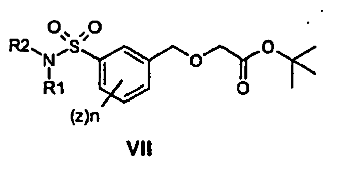
b) deprotecting the compound of formula VII with formic acid to yield a compound of formula VIII 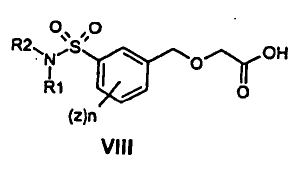 and
c) reacting the compound of formula VIII to yield a compound of formula IX.

10. A method for preparing a compound of formula XI 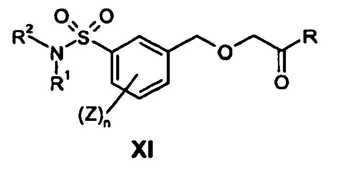 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 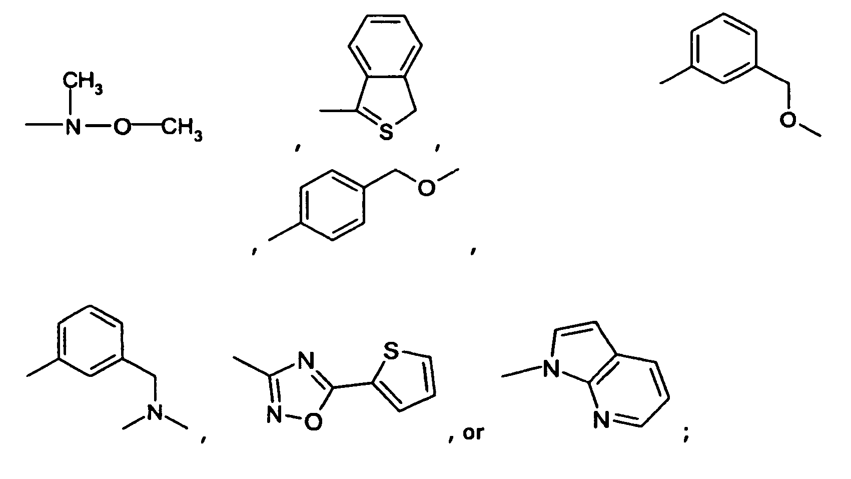
R¹ and R² are each independently C₁₋₆ alkyl, or R¹, R², and the N to which they are attached, in combination, are 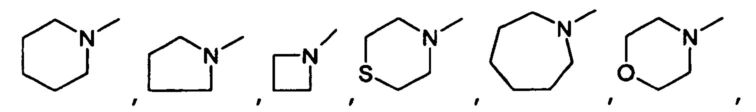 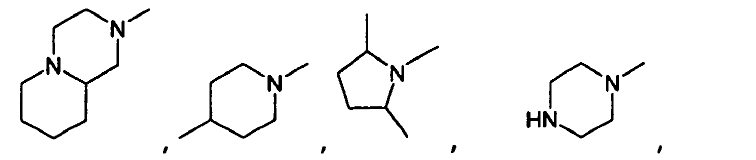 or 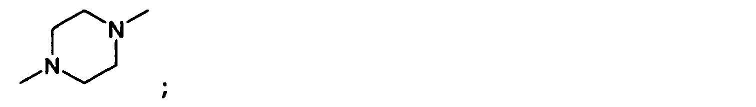
Z is, at each position, independently, Cl, F, methoxy, or methyl; and n is 0, 1, 2, 3, or 4;
said method comprising:
a) alkylating a compound of formula V as defined above with 2-chloro-N-methoxy-N-methylacetamide to provide a compound of formula X 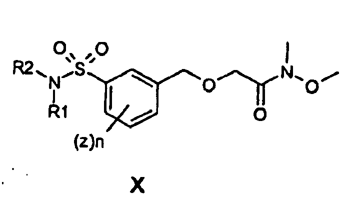 and
b) reacting the compound of formula X with an organometallic reagent to yield a compound of formula XI.

11. The method of claim 10, wherein the organometallic agent is selected from a Grignard reagent and an organolithium reagent.

12. A method for preparing a compound of formula XV 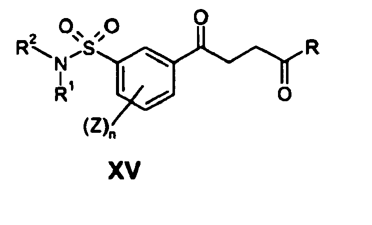 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 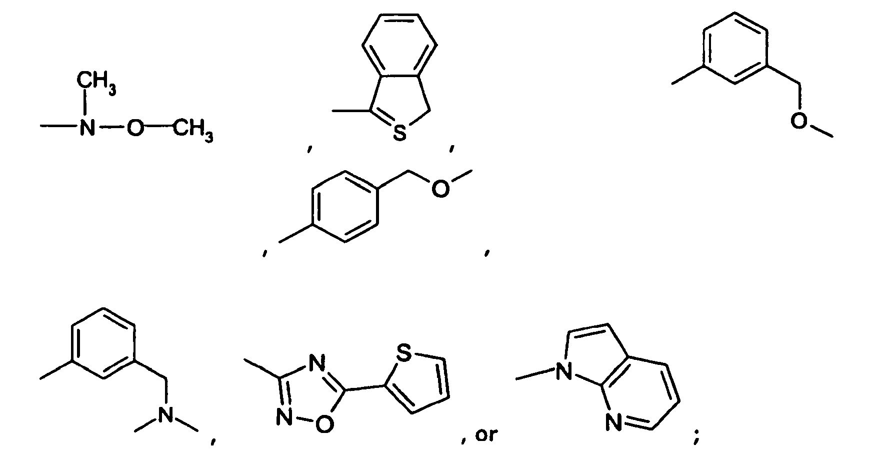
R¹ and R² are each independently C₁₋₆ alkyl, or R¹, R², and the N to which they are attached, in combination, are 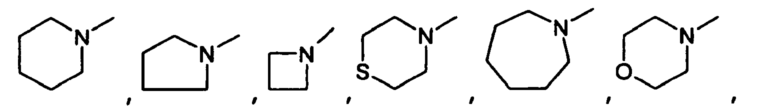 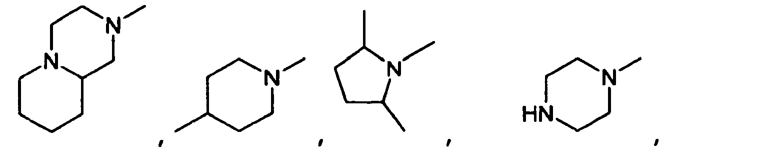 or 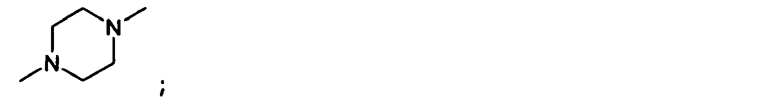
Z is, at each position, independently, Cl, F, methoxy, or methyl; and
n is 0, 1, 2, 3, or 4; and
said method comprising:
a) converting a compound of formula III as defined above to the corresponding acid chloride followed by reaction with a Grignard reagent derived from 1-bromo-3,3-dimethoxypropane to yield a compound of formula XII 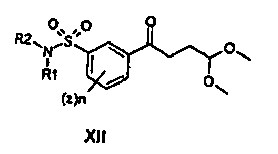
b) converting the compound of formula XII to a compound of formula XIII 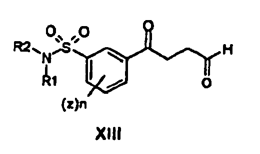
c) reacting the compound of formula XIII with an organometallic reagent to provide a compound of formula XIV 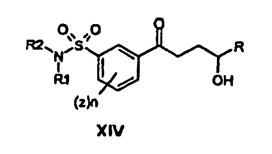 and
d) oxidizing the compound of formula XIV to yield a compound of formula XV.

13. The method of claim 12, wherein converting step b) occurs under acidic conditions.

14. The method of claim 12, wherein the organometallic reagent of step c) is selected from a Grignard reagent and an organolithium reagent.

15. A method for preparing a compound of formula XX 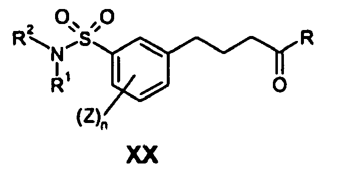 wherein
R is phenyl, dimethoxyphenyl, methoxyphenyl, chlorophenyl, pyridyl, fluorophenyl, ethyl, naphthyl, t-butyl, optionally substituted pyrrolidinyl, N-methylphenylamino, optionally substituted piperidinyl, cyclohexyl, quinolinyl, 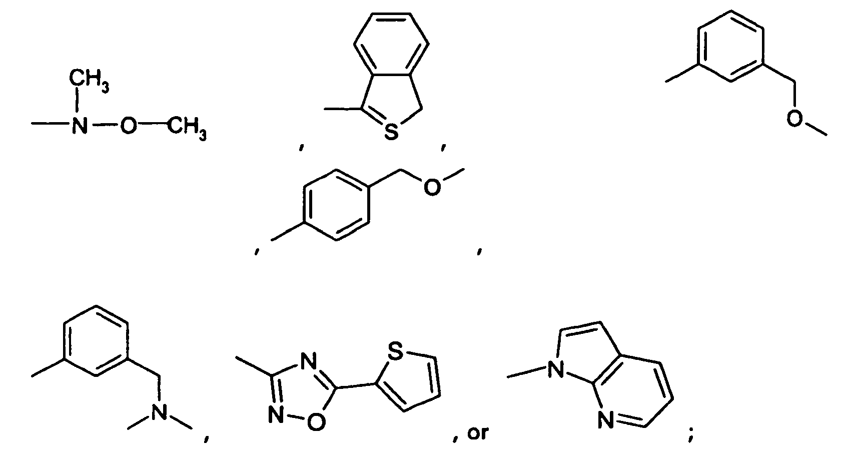
R¹ and R² are each independently C₁₋₆ alkyl, or R¹, R², and the N to which they are attached, in combination, are 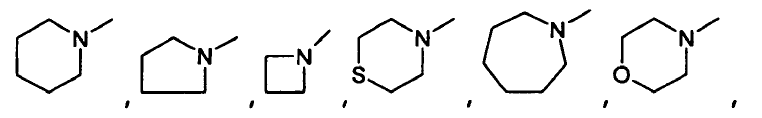 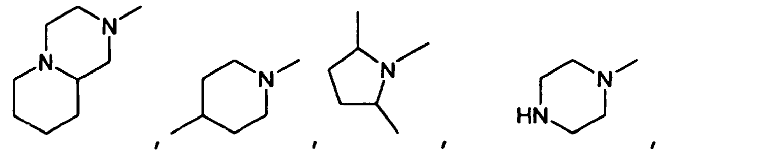 or 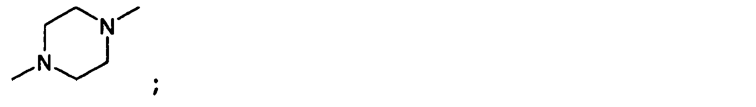
Z is, at each position, independently, Cl, F, methoxy, or methyl; and
n is 0, 1, 2, 3, or 4; and
said method comprising:
a) reducing a compound of formula XII as defined above to a compound of formula XVI 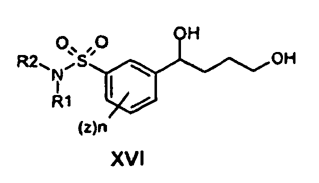
b) reducing the compound of formula XVI to provide a compound of formula XVII 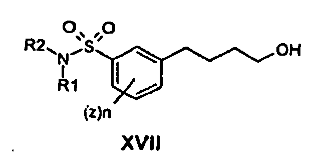
c) oxidizing the compound of formula XVII to a compound of formula XVIII 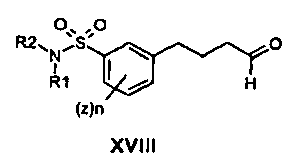
d) alkylating the compound of formula XVIII to provide a compound of formula XIX 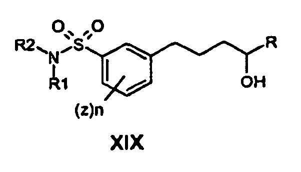 and
e) oxidizing the compound of formula XIX to provide a compound of formula XX.

## Patentansprüche

1. Verbindung der Formel I, ein pharmazeutisch akzeptables Salz davon, Diastereomere, Enantiomere oder Mischungen davon: 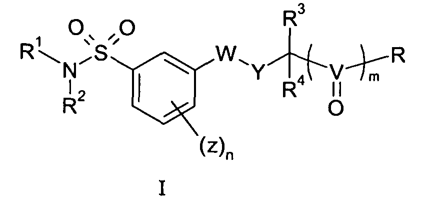 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 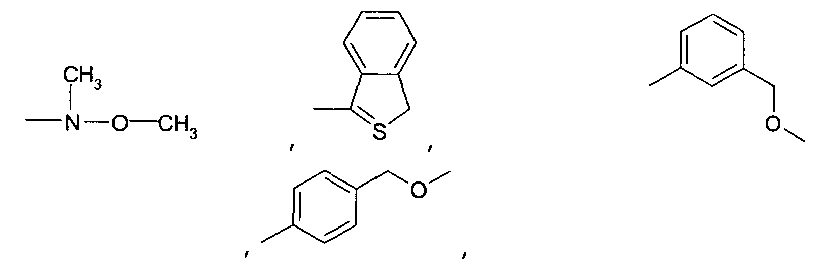 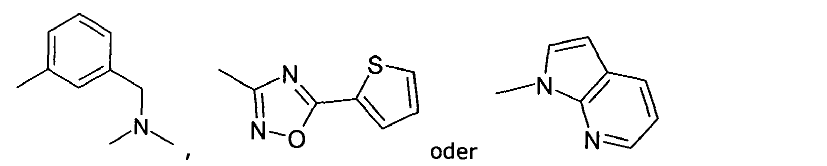 ist;
R¹ und R² jeweils unabhängig voneinander C₁₋₆-Alkyl sind, oder R¹, R² und das N, an das sie gebunden sind, zusammen 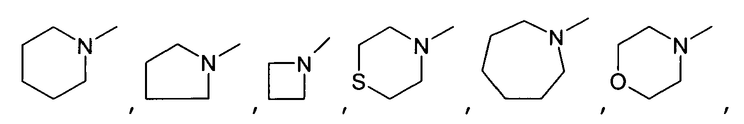 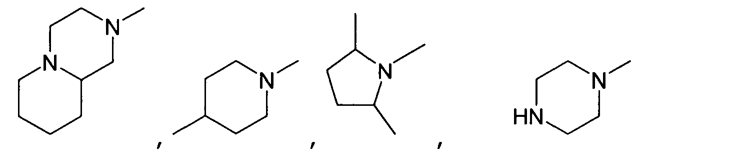 oder 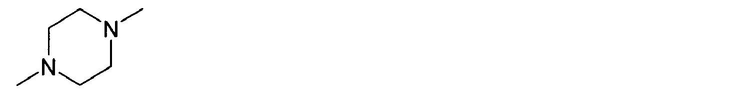 sind;
W CH₂ ist oder C=O ist;
Y CH₂, NH, NCH₃ oder O ist;
V C ist;
m 1 ist;
n 0, 1, 2, 3 oder 4 ist;
Z in jeder Position unabhängig voneinander Cl, F, Methoxy oder Methyl ist; und
R³ und R⁴ jeweils unabhängig voneinander H, Methyl oder Ethyl sind;
unter der Voraussetzung, dass die Verbindung nicht
2-Methyl-5-(4-morpholinylsulfonyl)-Benzoesäure-2-oxo-2-phenylethylester;
4-Fluor-3-(4-morpholinylsulfonyl)-Benzoesäure-2-oxo-2-phenylethylester;
4-Methoxy-3-(4-morpholinylsulfonyl)-Benzoesäure-2-oxo-2-phenylethylester;
4-Methyl-3-(4-morpholinylsulfonyl)-Benzoesäure-2-oxo-2-phenylethylester;
2-Chlor-5-(1-pyrrolidinylsulfonyl)-Benzoesäure-2-oxo-2-phenylethylester;
4-Chlor-3-(1-piperidinylsulfonyl)-Benzoesäure-2-oxo-2-phenylethylester;
4-Chlor-3-[(hexahydro-1H-azepin-1-yl)sulfonyl]-Benzoesäure-2-oxo-2-phenylethylester;
2,4-Dichlor-5-(1-piperidinylsulfonyl)-Benzoesäure-2-oxo-2-phenylethylester;
4-Methyl-3-(1-piperidinylsulfonyl)-Benzoesäure-2-oxo-2-phenylethylester;
2-Chlor-5-(1-piperidinylsulfonyl)-Benzoesäure-2-oxo-2-phenylethylester;
2,4-Dichlor-5-(4-morphoinylsulfonyl)-Benzoesäure-2-oxo-2-phenylethylester;
4-Chlor-3-(4-morpholinylsulfonyl)-Benzoesäure-2-oxo-2-phenylethylester;
2-Chlor-5-(4-morpholinylsulfonyl)-Benzoesäure-2-oxo-2-phenylethylester; und
3-(4-Morpholinylsulfonyl)-Benzoesäure-2-oxo-2-phenylethylester ist.

2. Verbindung nach Anspruch 1, worin
R¹, R² und das N, an das sie gebunden sind, zusammen 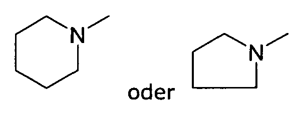 sind;

3. Verbindung nach Anspruch 1, worin Z 2-Chlor ist.

4. Verbindung nach irgendeinem der Ansprüche 1-3, die ausgewählt ist aus:
2,4-Dichlor-5-(piperidin-1-sulfonyl)-Benzoesäure-2-oxo-2-phenyl-ethylester;
2,4-Dichlor-5-(pyrrolidin-1-sulfonyl)-Benzoesäure-2-oxo-2-phenyl-ethylester;
5-(Azetidin-1-sulfonyl)-2,4-dichlor-Benzoesäure-2-oxo-2-phenyl-ethylester;
2,4-Dichlor-5-(thiomorpholin-4-sulfonyl)Benzoesäure-2-oxo-2-phenylethylester;
2,4-Dichlor-5-diethylsulfamoylbenzoesäure-2-oxo-2-phenylethylester;
5-(Azepan-1-sulfonyl)-2,4-dichlorbenzoesäure-2-oxo-2-phenylethylester;
2-Methoxy-5-(piperidin-1-sulfonyl)benzoesäure-2-oxo-2-phenylethylester;
2-Chlor-5-(piperidin-1-sulfonyl)benzoesäure-2-oxo-2-phenylethylester;
2-Chlor-4-fluor-5-(piperidin-1-sulfonyl)benzoesäure-2-oxo-phenylethylester;
2-Chlor-5-(piperidin-1-sulfonyl)benzoesäure-2-(2,5-dimethoxyphenyl)-2-oxoethylester;
2-Chlor-5-(piperidin-1-sulfonyl)benzoesäure-2-(3-methoxyphenyl)-2-oxoethylester;
2-Chlor-5-(piperidin-1-sulfonyl)benzoesäure-2-(3-chlorphenyl)-2-oxoethylester;
2-Methyl-5-(piperidin-1-sulfonyl)benzoesäure-2-oxo-2-phenylethylester;
2-Fluor-5-(piperidin-1-sulfonyl)benzoesäure-2-oxo-2-phenylethylester;
2-Chlor-5-(piperidin-1-sulfonyl)benzoesäure-2-oxo-2-pyridin-2-ylethylester;
2-Chlor-5-(piperidin-1-sulfonyl)benzoesäure-1-methyl-2-oxo-2-phenylethylester;
2-Chlor-5-(piperidin-1-sulfonyl)benzoesäure-2-(4-fluorphenyl)-2-oxoethylester;
2-Chlor-5-(piperidin-1-sulfonyl)benzoesäure-2-oxo-2-p-tolylethylester;
2-Chlor-5-(piperidin-1-sulfonyl)benzoesäure-2-oxobutylester;
2-Chlor-5-(piperidin-1-sulfonyl)benzoesäure-2-naphthalen-2-yl-2-oxoethylester;
2-Chlor-5-(piperidin-1-sulfonyl)benzoesäure-2-benzo[b]thiophen-3-yl-2-oxoethylester;
2-Chlor-5-(piperidin-1-sulfonyl)benzoesäure-3,3-dimethyl-2-oxobutylester;
2-Chlor-5-(morpholin-4-sulfonyl)benzoesäure-2-oxo-2-phenylethylester;
5-(Butylethylsulphamoyl)-2-chlorbenzoesäure-2-oxo-2-phenylethylester;
2-Chlor-5-dimethylsulphamoylbenzoesäure-2-oxo-2-phenylethylester;
2-Chlor-5-(octahydropyrido[1,2-a]pyrazin-2-sulfonyl)benzoesäure-2-oxo-2-phenylethylester;
2-Chlor-5-(4-methylpiperidin-1-sulfonyl)benzoesäure-2-oxo-2-phenylethylester;
2-Chlor-5-(2,5-dimethylpyrollidin-1-sulfonyl)benzoesäure-2-oxo-2-phenylethylester;
3-(Piperidin-1-sulfonyl)benzoesäure-2-oxo-2-phenylethylester;
N-2-oxo-2-phenethyl 2,4-dichlor-5-[piperidinosulfonyl]-benzamid;
2-Chlor-4-fluor-N-(2-oxo-2-phenylethyl)-5-(piperidin-1-sulfonyl)-benzamid;
2-oxo-2-phenethyl-2,4-dichlor-5-[piperazinosulfonyl]-benzoat-hydrochlorid;
2-oxo-2-phenethyl 2,4-dichlor-5-[4-methyl-piperazinosulfonyl]-benzoat-hydrochlorid;
2,4-dichlor-5-[piperidiinosulfonyl]-benzyloxyacetophenon;
2-Chlor-5-(piperidin-1-sulfonyl)benzoesäure-1,1-dimethyl-2-oxo-2-phenylethylester;
[2-Chlor-5-(piperidin-1-sulfonyl)benzylamino]essigsäure-tert-butylester;
{[2-Chlor-5-(piperidin-1-sulfonyl)-benzyl]-methyl-amino}-essigsäure-tert-butylester;
(2-Chlor-5-(piperidin-1-sulfonyl)benzyloxy)essigsäure-tert-butylester;
2-[2-Chlor-5-(piperidin-1-sulfonyl)benzyloxy]propionsäure-tert-butylester;
2-[2-Chlor-5-(piperidin-1-sulfonyl)benzyloxy]buttersäure-tert-butylester;
2-(2-Chlor-5-(piperidin-1-sulfonyl)benzyloxy)-1-pyrrolidin-1-ylethanon;
2-(2-Chlor-5-(piperidin-1-sulfonyl)benzyloxy)-N-methyl-N-phenylacetamid;
N-tert-butyl-2-(2-chlor-5-(piperidin-1-sulfonyl)benzyloxy)acetamid;
(1-(2-(2-Chlor-5-piperidin-1-sulfonyl)benzyloxy)acetyl)pyrrolidin-3-yl)carbamidsäure-tert-butylester;
(1-(2-(2-Chlor-5-(piperidin-1-sulfonyl)benzyloxy)acetyl)piperidin-4-yl)carbamidsäure-tert-butylester;
2-[2-Chlor-5-(piperidin-1-sulfonyl)-benzyloxy]-N-pyridin-2-yl-acetamid;
2-[2-Chlor-5-piperidin-1-sulfonyl)benzyloxy]-1-piperidin-1-ylethanon;
2-[2-Chlor-5-(piperidin-1-sulfonyl)-benzyloxy]-N-methyl-N-pyridin-2-yl-acetamid;
2-(2,4-Dichlor-5-(piperidin-1-sulfonyl)benzyloxy)-N-methoxy-N-methylacetamid;
2-(2-Chlor-5-(piperidin-1-sulfonyl)benzyloxy)-N-methoxy-N-methylacetamid;
N-methoxy-2-[2-methoxy-5-(piperidin-1-sulfonyl)-benzyloxy)-N-methylacetamid;
N-methoxy-2-[2-methoxy-5-(piperidin-1-sulfonyl)-benzyloxy)-N-methylacetamid;
2-[2-Brom-5-(piperidin-1-sulfonyl)-benzyloxy)-N-methoxy-N-methyl-acetamid;
N-methoxy-N-methyl-2-[5-(piperidin-1-sulfonyl)-2-trifluormethoxy-benzyloxy]-acetamid;
2-(1-(2-Chlor-5-(piperidin-1-sulfonyl)-phenyl)-ethoxy)-N-methoxy-N-methylacetamid;
2-[2-Chlor-5-(thiomorpholin-4-sulfonyl)-benzyloxy]-N-methoxy-N-methylacetamid;
2-[2-Chlor-5-(piperidin-1-sulfonyl)benzyloxy]-1-(3-methoxyphenyl)ethanon;
2-[2-Chlor-5-(piperidin-1-sulfonyl)benzyloxy]-1-cyclohexylethanon;
2-[2-Chlor-5-(piperidin-1-sulfonyl)benzyloxy]-1-(2,5-dimethoxyphenyl)ethanon;
2-[2,4-Dichlor-5-(piperidin-1-sulfonyl)benzyloxy]-1-(3-methoxyphenyl)ethanon;
1-(3-Chlorphenyl)-2-[2-chlor-5-(piperidin-1-sulfonyl)benzyloxy]-ethanon;
1-(3-Chlorphenyl)-2-[2,4-dichlor-5-(piperidin-1-sulfonyl)benzyloxy]-ethanon;
2-[2,4-dichlor-5-(piperidin-1-sulfonyl)benzyloxy]-1-naphthalen-2-ylethanon;
2-[2-Chlor-5-(piperidin-1-sulfonyl)benzyloxy]-1-naphthalen-2-ylethanon;
4-Chlor-N,N-dimethyl-5-(2-oxo-2-phenylethoxymethyl)benzolsulfonamid;
4-Chlor-N,N-diethyl-3-(2-oxo-2-phenylethoxymethyl)benzolsulfonamid;
4-Chlor-N-ethyl-N-methyl-3-(2-oxo-2-phenylethoxymethyl)benzolsulfonamid;
4-Chlor-N-methyl-3-(2-oxo-2-phenylethoxymethyl)-N-propyl-benzolsulfonamid;
2-[2-Brom-5(piperidin-1-sulfonyl)-benzyloxy]-1-phenyl-ethanon;
2-[2-Chlor-5-(thiomorpholin-4-sulfonyl)-benzyloxy]-1-phenyl-ethanon;
2-(2-Chlor-5-(piperidin-1-sulfonyl)-benzyloxyl)-1-(1H-pyrrolo(2,3-b)-pyridin-3-yl)-ethanon;
2-[2-Chlor-5-(4-methylpiperazin-1-sulfonyl)benzyloxy]-1-pyridin-2-yl-ethanon;
2-[2-Chlor-5-(piperidin-1-sulfonyl)benzyloxy]-1-pyridin-3-ylethanon;
2-[2-Chlor-5-(piperidin-1-sulfonyl)benzyloxy]-1-pyridin-4-ylethanon;
2-[2-Chlor-5-(piperidin-1-sulfonyl)benzyloxy]-1-pyridin-2-ylethanon;
4-Chlor-N,N-dimethyl-3-(2-oxo-2-pyridin-3-ylethoxymethyl)benzolsulfonamid;
2-[2-Chlor-5-(piperidin-1-sulfonyl)benzyloxy]-1-chinolin-8-ylethanon;
4-Chlor-N,N-dimethyl-3-(2-oxo-2-pyridin-2-ylethoxymethyl)benzolsulfonamid;
4-Chlor-N-ethyl-N-methyl-3-(2-oxo-2-pyridin-2-ylethoxymethyl)benzolsulfonamid;
4-Chlor-N,N-diethyl-3-(2-oxo-2-pyridin-2-ylethoxymethyl)benzolsulfonamid;
4-Chlor-N-methyl-3-(2-oxo-2-pyridin-2-ylethoxymethyl)-N-propyl-benzolsulfonamid;
4-Chlor-N-ethyl-N-methyl-3-(2-oxo-2-pyridin-3-ylethoxymethyl)benzolsulfonamid;
4-Chlor-N,N-diethyl-3-(2-oxo-2-pyridin-3-ylethoxymethyl)benzolsulfonamid;
2-[2-Chlor-5-(piperidin-1-sulfonyl)-benzyloxy]-1-(4-methyl-pyridin-2-yl)-ethanon;
2-[2-Chlor-5-(piperidin-1-sulfonyl)-benzyloxy]-1-(6-methyl-pyridin-2-yl)-ethanon;
2-[2-Chlor-5-(piperidin-1-sulfonyl)-benzyloxy]-1-(5-methyl-pyridin-2-yl)-ethanon;
4-Chlor-N-ethyl-N-methyl-3-[2-(4-methyl-pyridin-2-yl)-2-oxo-ethoxymethyl]-benzolsulfonamid;
4-Chlor-N-ethyl-N-methyl-3-[2-(6-methyl-pyridin-2-yl)-2-oxo-ethoxymethyl]-benzolsulfonamid;
4-Chlor-N-ethyl-N-methyl-3-[2-(5-methyl-pyridin-2-yl)-2-oxo-ethoxymethyl]-benzolsulfonamid;
2-[2-Chlor-5-(piperidin-1-sulpfonyl)-benzyloxy]-1-(3-methyl-pyridin-2-yl)-ethanon;
4-Chlor-N-ethyl-N-methyl-3-[2-(3-methyl-pyridin-2-yl)-2-oxo-ethoxymethyl]-benzolsulfonamid;
1-(2-Brom-5-dimethylamino-pyridin-4-yl)-2-[2-chlor-5-(piperidin-1-sulfonyl)-benzyloxy]-ethanon;
2-[2-Methoxy-5-(piperidin-1-sulpfonyl)-benzyloxy]-1-pyridin-2-yl-ethanon;
2-[2-Methyl-5-(piperidin-1-sulpfonyl)-benzyloxy]-1-pyridin-2-yl-ethanon;
2-5-(Piperidin-1-sulpfonyl)-2-trifluormethoxy-benzyloxy]-1-pyridin-2-yl-ethanon;
2-(1-(2-Chlor-5-(piperidin-1-sulfonyl)-phenyl)-ethoxy)-1-pyridin-2-yl-ethanon;
2-(1-(2-Chlor-5-(piperidin-1-sulfonyl)-phenyl)-ethoxy)-1-pyridin-3-yl-ethanon;
2-(2-Chlor-5-(piperidin-1-sulfonyl)-benzyloxy)-1-pyrazin-2-yl-ethanon;
2-(2-Chlor-5-(piperidin-1-sulfonyl)-benzyloxy)-1-(1-methyl-1H-imidazol-2-yl)-ethanon;
2-(2-Chlor-5-(piperidin-1-sulfonyl)-benzyloxy)-1-(1-pyridin-2-yl-1H-imidazol-2-yl)ethanon;
2-(2-Chlor-5-(piperidin-1-sulfonyl)-benzyloxy)-1-(1-methyl-1H-indol-5-yl)-ethanon;
2-[2-Chlor-5-(piperidin-1-sulfonyl)benzyloxy]-1-pyridin-2-yl-propan-1-on;
2-[2,4-Dichlor-5-(piperidin-1-sulfonyl)benzyloxy]-1-(3-methoxymethylphenyl)ethanon;
2-[2,4-Dichlor-5-(piperidin-1-sulfonyl)benzyloxy]-1-(4-methoxymethylphenyl)ethanon;
2-[2,4-Dichlor-5-(piperidin-1-sulfonyl)benzyloxy]-1-(3-dimethylaminomethylphenyl)ethanon;
2-[2-Chlor-5-(piperidin-1-sulfonyl)benzyloxy]-1-(3-dimethylaminomethylphenyl)-ethanon;
2-(2-Oxo-2-phenyl-ethoxymethyl)-4-(piperidin-1-sulfonyl)-benzonitril;
4-[2-Chlor-5-(piperidin-1-sulfonyl)phenyl]-4-oxobutyraldehyd;
4-[2-Methyl-5-(piperidin-1-sulfonyl)phenyl]-4-oxobutyraldehyd;
4-[2-Chlor-5-(piperidin-1-sulfonyl)phenyl]-butyraldehyd;
1-[2-Chlor-5-(piperidin-1-sulfonyl)phenyl]-4-naphthalen-2-ylbutan-1,4-dion;
1-[2-Chlor-5-(piperidin-1-sulfonyl)phenyl]-4-naphthalen-1-ylbutan-1,4-dion;
1-[2-Chlor-5-(piperidin-1-sulfonyl)phenyl]-4-phenylbutan-1,4-dion;
1-[2-Chlor-5-(piperidin-1-sulfonyl)phenyl]-4-pyridin-3-ylbutan-1,4-dion;
1-[2-Chlor-5-(piperidin-1-sulfonyl)phenyl]-4-(3-dimethylaminomethylphenyl)butan-1,4-dion;
4-[2-Chlor-5-(piperidin-1-sulfonyl)phenyl]-1-phenyl-butan-1-on;
4-[2-Chlor-5-(piperidin-1-sulfonyl)phenyl]-1-pyridin-3-yl-butan-1-on;
1-[2-Chlor-5-(piperidin-1-sulfonyl)phenyl]-4-pyridin-2-ylbutan-1,4-dion;
1-[2-Methyl-5-(piperidin-1-sulfonyl)phenyl]-4-pyridin-2-ylbutan-1,4-dion;
4-[2-Chlor-5-(piperidin-1-sulfonyl)phenyl]-1-pyridin-2-yl-butan-1-on;
1-(4-Chlor-3-(5-thiophen-2-yl-(1,2,4)oxadiazol-3-yl-methoxy-methyl)-benzolsulfonyl)-piperidin;
(3-(3-Benzolsulfinyl-propenyl)-4-chlor-benzolsulfonyl)-piperidin;
2-[2-Chlor-5-(piperidin-1-sulfonyl)benzyloxy]-1-pyrrolo[2,3-b]pyridin-1-ylethanon; und
2-[2-Chlor-5-(piperidin-1-sulfonyl)benzylamino]-1-piperidin-1-ylethanon.

5. Verbindung nach irgendeinem der Ansprüche 1-4 zur Anwendung als ein Arzneimittel.

6. Verwendung einer Verbindung nach irgendeinem der Ansprüche 1-4 zur Herstellung eines Arzneimittels zur Behandlung von neurologischen und psychiatrischen Störungen, die mit Glutamat-Dysfunktion verbunden sind.

7. Verfahren zur Herstellung einer Verbindung der Formel IV 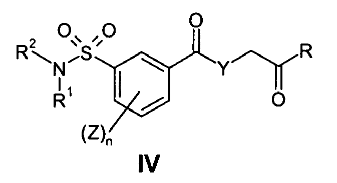 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 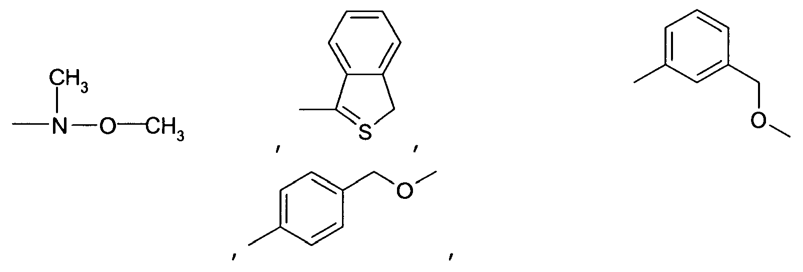 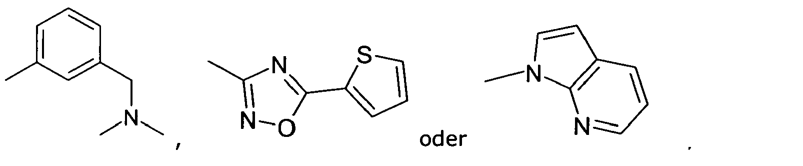 ist;
R¹ und R² jeweils unabhängig voneinander C₁₋₆-Alkyl sind, oder R¹, R² und das N, an das sie gebunden sind, zusammen 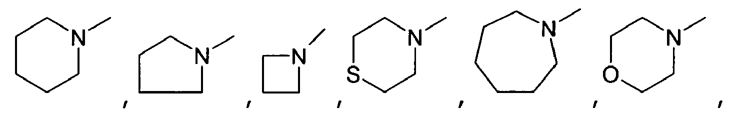 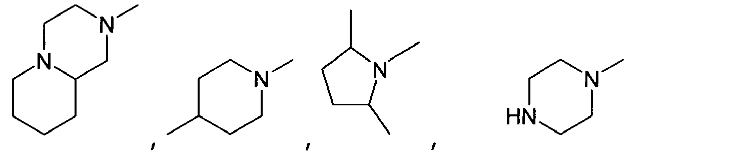 oder 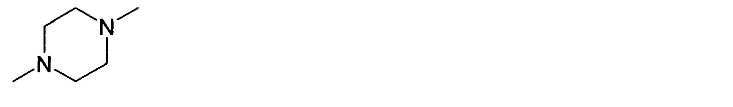 sind;
Y CH₂, NH, NCH₃ oder O ist;
Z in jeder Position unabhängig voneinander Cl, F, Methoxy oder Methyl ist; und
n 0, 1, 2, 3 oder 4 ist;
wobei das Verfahren umfasst:
a) Umsetzen einer Verbindung der Formel 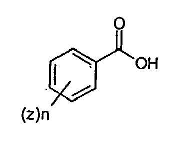 mit Chlorsulfonsäure zur Bereitstellung einer Verbindung der Formel II 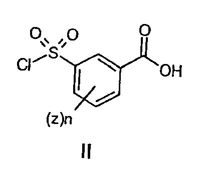
b) Umsetzen der Verbindung der Formel II mit einem Amin zur Bereitstellung einer Verbindung der Formel III 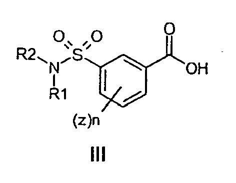 und
c) Alkylieren der Verbindung der Formel III zur Bildung einer Verbindung der Formel IV.

8. Verfahren zur Herstellung einer Verbindung der Formel VI 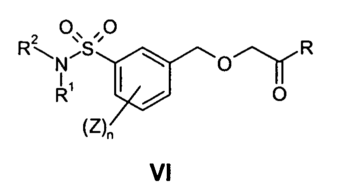 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 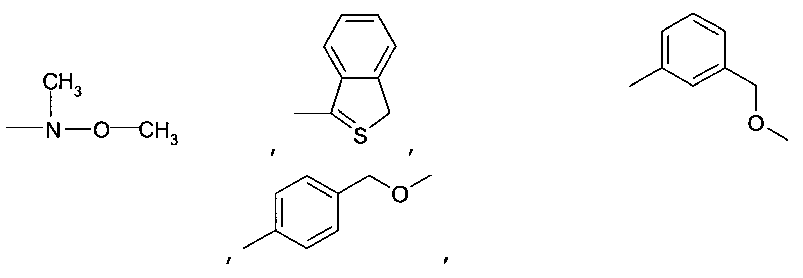 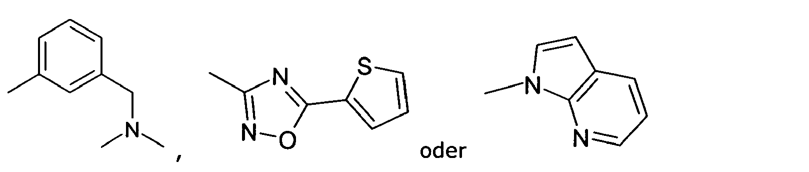 ist;
R¹ und R² jeweils unabhängig voneinander C₁₋₆-Alkyl sind, oder R¹, R² und das N, an das sie gebunden sind, zusammen 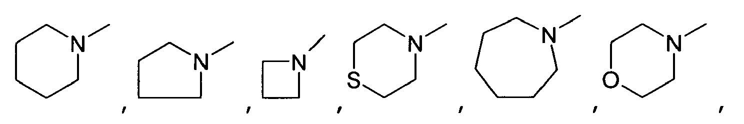 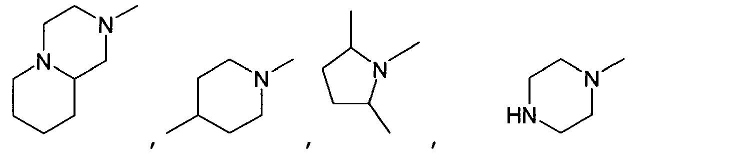 oder 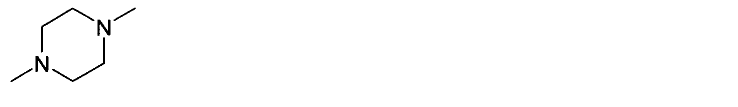 sind;
Z in jeder Position unabhängig voneinander Cl, F, Methoxy oder Methyl ist; und
n 0, 1, 2, 3 oder 4 ist; und
wobei das Verfahren umfasst:
a) Reduzieren einer Verbindung der Formel III, wie oben definiert, zu einer Verbindung der Formel V 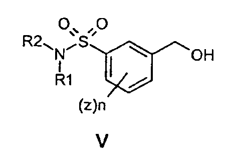 ; und
b) Alkylieren der Verbindung der Formel V, um eine Verbindung der Formel VI zu erhalten.

9. Verfahren zur Herstellung einer Verbindung der Formel IX 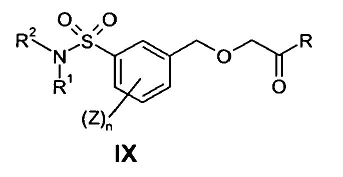 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 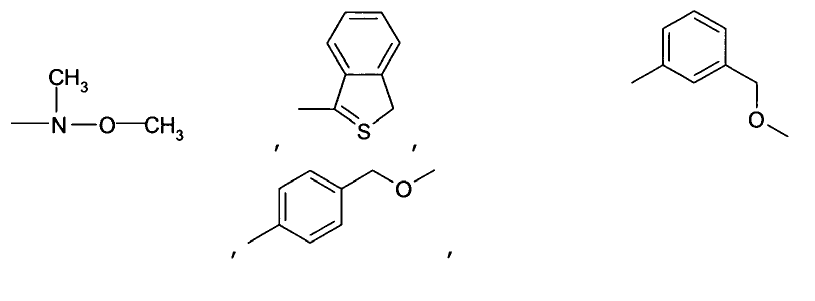 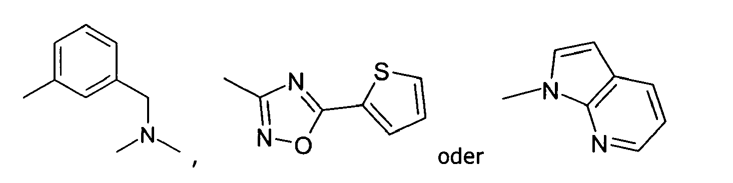 ist;
R¹ und R² jeweils unabhängig voneinander C₁₋₆-Alkyl sind, oder R¹, R² und das N, an das sie gebunden sind, zusammen 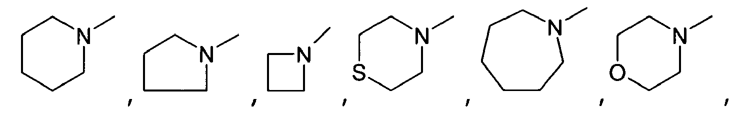 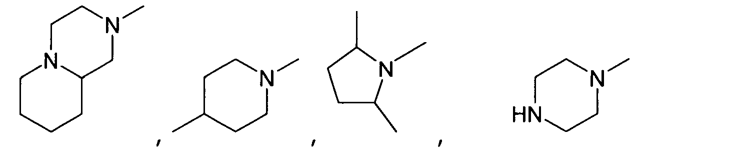 oder 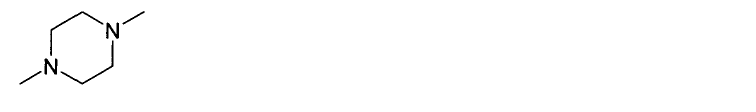 sind;
Z in jeder Position unabhängig voneinander Cl, F, Methoxy oder Methyl ist; und
n 0, 1, 2, 3 oder 4 ist;
welches Verfahren umfasst:
a) Alkylieren einer Verbindung der Formel V, wie oben definiert, zur Bereitstellung einer Verbindung der Formel VII 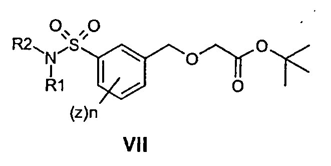
b) Entschützen der Verbindung der Formel VII mit Hilfe von Ameisensäure, um eine Verbindung der Formel VIII zu erhalten 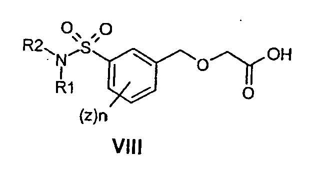 und
c) Umsetzten der Verbindung der Formel VIII, um eine Verbindung der Formel IX zu erhalten.

10. Verfahren zur Herstellung einer Verbindung der Formel XI 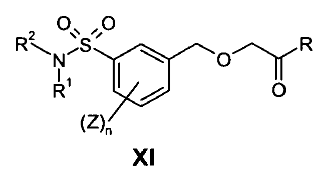 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 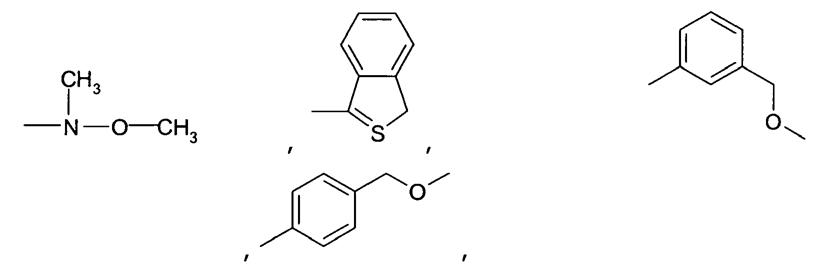 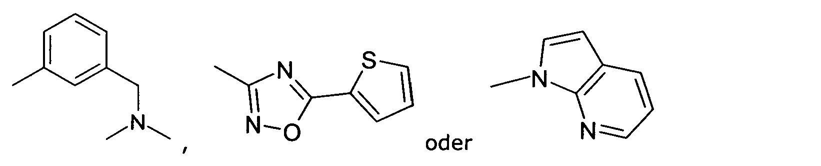 ist;
R¹ und R² jeweils unabhängig voneinander C₁₋₆-Alkyl sind, oder R¹, R² und das N, an das sie gebunden sind, zusammen 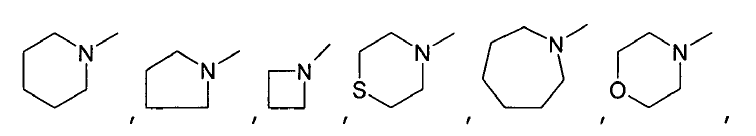 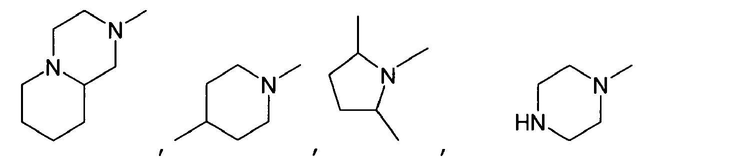 oder 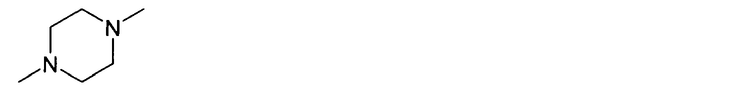 sind;
Z in jeder Position unabhängig voneinander Cl, F, Methoxy oder Methyl ist; und
n 0, 1, 2, 3 oder 4 ist;
wobei das Verfahren umfasst:
a) Alkylieren einer Verbindung der Formel V, wie oben definiert, mit Hilfe von 2-Chlor-N-methoxy-N-methylacetamid zur Bereitstellung einer Verbindung der Formel X 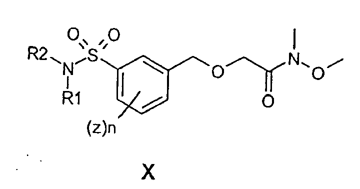 und
b) Umsetzen der Verbindung der Formel X mit einem organometallischen Reagens, um eine Verbindung der Formel XI zu erhalten.

11. Verfahren nach Anspruch 10, worin der organometallische Wirkstoff aus einem Grignard-Reagens und einem Organolithium-Reagens ausgewählt ist.

12. Verfahren zur Herstellung einer Verbindung der Formel XV 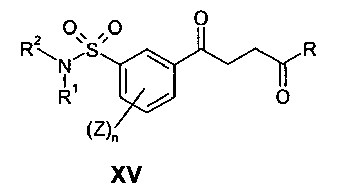 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 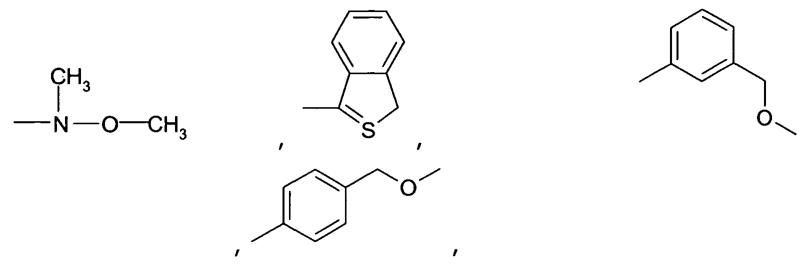 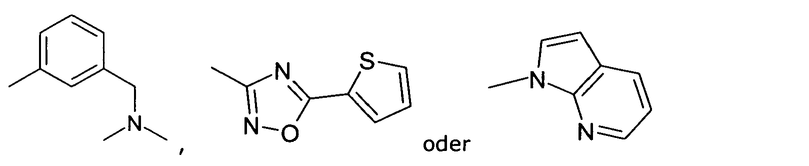 ist;
R¹ und R² jeweils unabhängig voneinander C₁₋₆-Alkyl sind, oder R¹, R² und das N, an das sie gebunden sind, zusammen 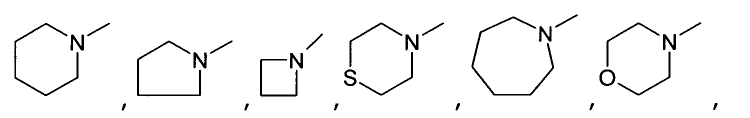 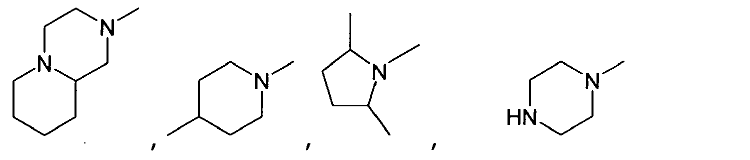 oder 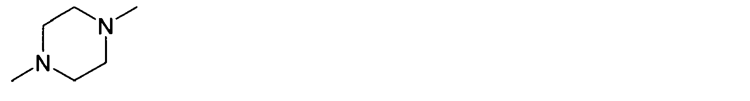 sind;
Z in jeder Position unabhängig voneinander Cl, F, Methoxy oder Methyl ist; und
n 0, 1, 2, 3 oder 4 ist; und
wobei das Verfahren umfasst:
a) Umwandeln einer Verbindung der Formel III, wie oben definiert, ins entsprechende Säurechlorid, gefolgt von Umsetzen mit einem Grignard-Reagens, das aus 1-Brom-3,3-dimethoxypropan abgeleitet ist, um eine Verbindung der Formel XII zu erhalten 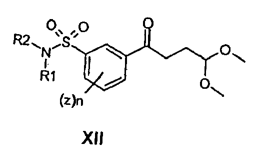
b) Umwandeln der Verbindung der Formel XII in eine Verbindung der Formel XIII 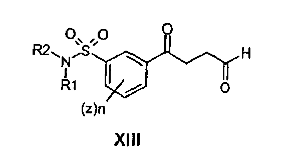
c) Umsetzen der Verbindung der Formel XIII mit einem organometallischen Reagens zur Bereitstellung einer Verbindung der Formel XIV. 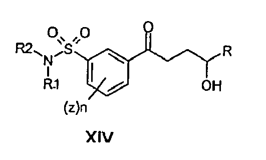 und
d) Oxidieren der Verbindung der Formel XIV, um eine Verbindung der Formel XV zu erhalten.

13. Verfahren nach Anspruch 12, worin Umwandeln der Stufe b) unter sauren Bedingungen erfolgt.

14. Verfahren nach Anspruch 12, worin das organometallische Reagens der Stufe c) aus einem Grignard-Reagens und einem Organolithium-Reagens ausgewählt ist.

15. Verfahren zur Herstellung einer Verbindung der Formel XX 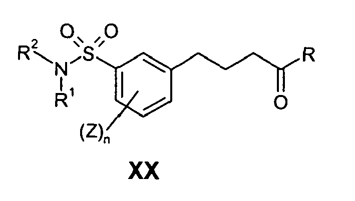 worin
R Phenyl, Dimethoxyphenyl, Methoxyphenyl, Chlorphenyl, Pyridyl, Fluorphenyl, Ethyl, Naphthyl, t-Butyl, gegebenenfalls substituiertes Pyrrolidinyl, N-Methylphenylamino, gegebenenfalls substituiertes Piperidinyl, Cyclohexyl, Chinolinyl, 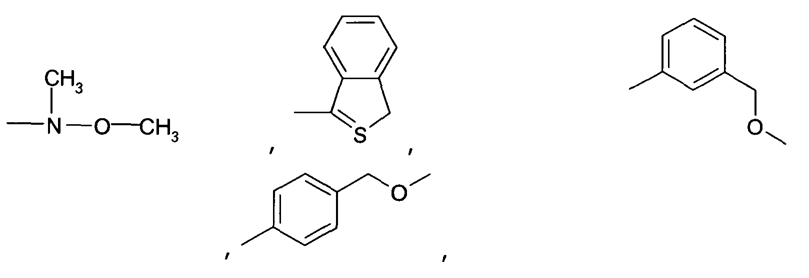 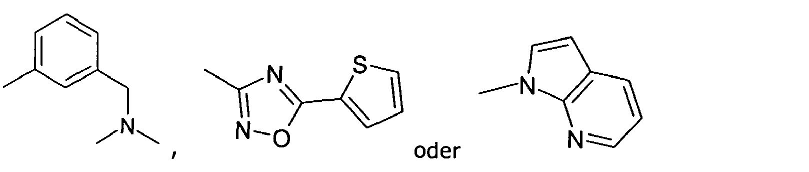 ist;
R¹ und R² jeweils unabhängig voneinander C₁₋₆-Alkyl sind, oder R¹, R² und das N, an das sie gebunden sind, zusammen 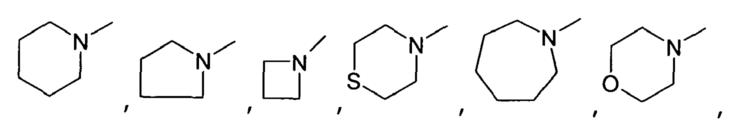 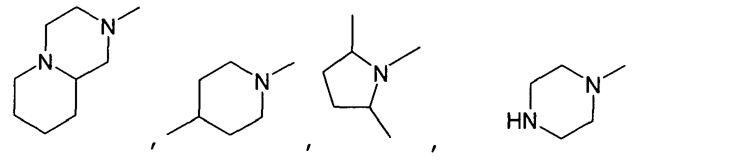 oder 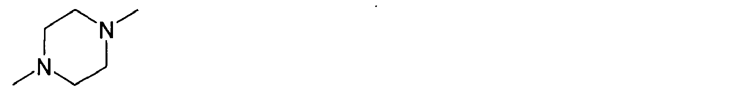 sind;
Z in jeder Position unabhängig voneinander Cl, F, Methoxy oder Methyl ist; und
n 0, 1, 2, 3 oder 4 ist; und
wobei das Verfahren umfasst:
a) Reduzieren einer Verbindung der Formel XII, wie oben definiert, zu einer Verbindung der Formel XVI 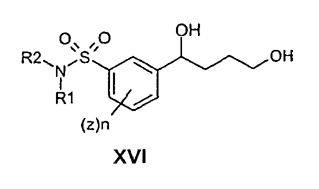
b) Reduzieren der Verbindung der Formel XVI zur Bereitstellung einer Verbindung der Formel XVII 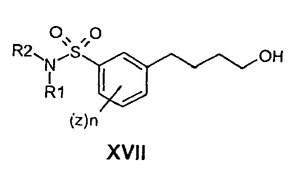
c) Oxidieren der Verbindung der Formel XVII zu einer Verbindung der Formel XVIII 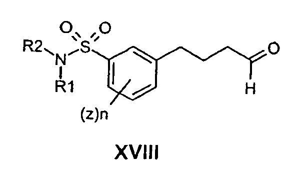
d) Alkylieren der Verbindung der Formel XVIII zur Bereitstellung einer Verbindung der Formel XIX 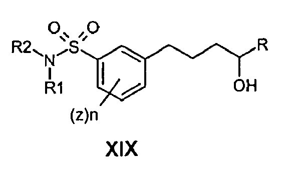 und
e) Oxidieren der Verbindung der Formel XIX zur Bereitstellung einer Verbindung der Formel XX.

## Revendications

1. Composé de formule I, sel de qualité pharmaceutique dudit composé, diastéréisomères, énantiomères ou mélanges de ceux-ci : 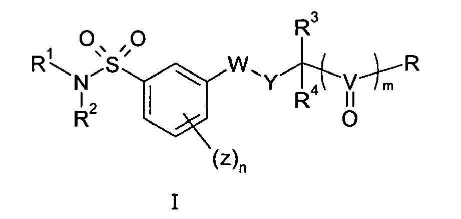 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 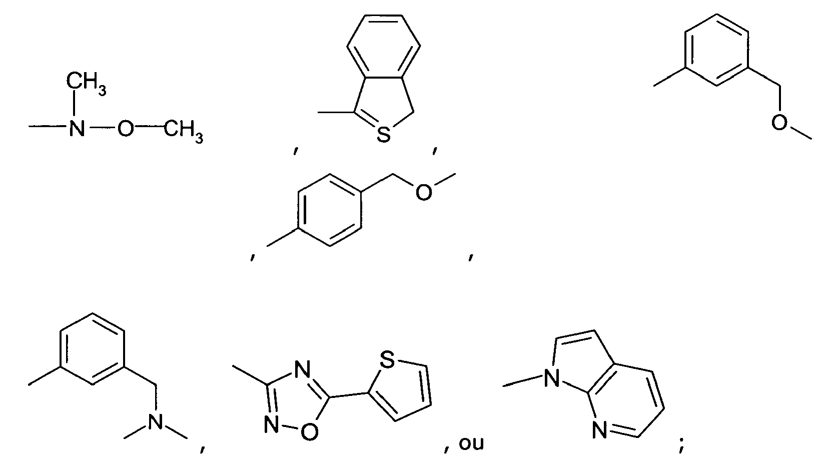
R¹ et R² sont chacun, indépendamment, un groupe alkyle en C₁₋₆, ou R¹, R² et le N auquel ils sont liés, par combinaison, sont 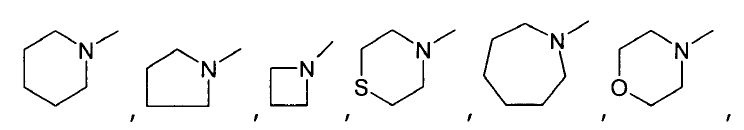 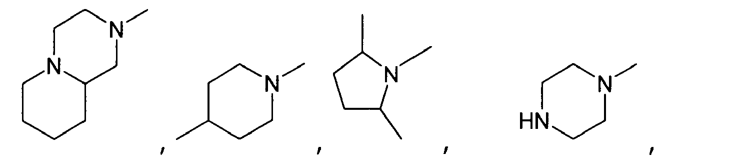 ou 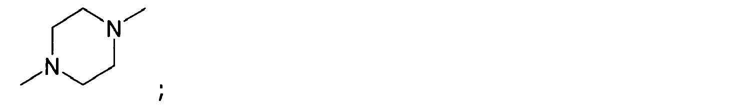
W est CH₂ ou C=O ;
Y est CH₂, NH, NCH₃ ou O ;
V est C ;
m est égal à 1 ;
n est égal à 0, 1, 2, 3 ou 4 ;
Z est, en chaque position, indépendamment, CI, F, méthoxy ou méthyle ; et
R³ et R⁴ sont chacun, indépendamment, H, méthyle ou éthyle ;
sous réserve que le composé ne soit pas
ester 2-oxo-2-phényléthylique d'acide 2-méthyl-5-(4-morpholinylsulfonyl)benzoïque ;
ester 2-oxo-2-phényléthylique d'acide 4-fluoro-3-(4-morpholinylsulfonyl)benzoïque ;
ester 2-oxo-2-phényléthylique d'acide 4-méthoxy-3-(4-morpholinylsulfonyl)benzoïque ;
ester 2-oxo-2-phényléthylique d'acide 4-méthyl-3-(4-morpholinylsulfonyl)benzoïque ;
ester 2-oxo-2-phényléthylique d'acide 2-chloro-5-(1-pyrrolidinylsulfonyl)benzoïque ;
ester 2-oxo-2-phényléthylique d'acide 4-chloro-3-(1-pipéridinylsulfonyl)benzoïque ;
ester 2-oxo-2-phényléthylique d'acide 4-chloro-3-[(hexahydro-1H-azépin-1-yl)sulfonyl]benzoïque ;
ester 2-oxo-2-phényléthylique d'acide 2,4-dichloro-5-(1-pipéridinylsulfonyl)benzoïque ;
ester 2-oxo-2-phényléthylique d'acide 4-méthyl-3-(1-pipéridinylsulfonyl)benzoïque ;
ester 2-oxo-2-phényléthylique d'acide 2-chloro-5-(1-pipéridinylsulfonyl)benzoïque ;
ester 2-oxo-2-phényléthylique d'acide 2,4-dichloro-5-(4-morpholinylsulfonyl)benzoïque ;
ester 2-oxo-2-phényléthylique d'acide 4-chloro-3-(4-morpholinylsulfonyl)benzoïque ;
ester 2-oxo-2-phényléthylique d'acide 2-chloro-5-(4-morpholinylsulfonyl)benzoïque ;
et
ester 2-oxo-2-phényléthylique d'acide 3-(4-morpholinylsulfonyl)benzoïque.

2. Composé de la revendication 1, dans lequel
R¹, R² et le N auquel ils sont liés, par combinaison, sont

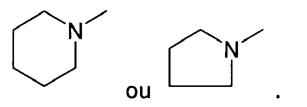

3. Composé de la revendication 1, dans lequel Z est 2-chloro.

4. Composé selon l'une quelconque des revendications 1 à 3, ledit composé étant choisi parmi :
ester 2-oxo-2-phényléthylique d'acide 2,4-dichloro-5-(pipéridine-1-sulfonyl)benzoïque ;
ester 2-oxo-2-phényléthylique d'acide 2,4-dichloro-5-(pyrrolidine-1-sulfonyl)benzoïque ;
ester 2-oxo-2-phényléthylique d'acide 5-(azétidine-1-sulfonyl)-2,4-dichloro-benzoïque ;
ester 2-oxo-2-phényléthylique d'acide 2,4-dichloro-5-(thiomorpholine-4-sulfonyl)benzoïque ;
ester 2-oxo-2-phényléthylique d'acide 2,4-dichloro-5-diéthylsulfamoylbenzoïque ;
ester 2-oxo-2-phényléthylique d'acide 5-(azépane-1-sulfonyl)-2,4-dichlorobenzoïque ;
ester 2-oxo-2-phényléthylique d'acide 2-méthoxy-5-(pipéridine-1-sulfonyl)benzoïque ;
ester 2-oxo-2-phényléthylique d'acide 2-chloro-5-(pipéridine-1-sulfonyl)benzoïque ;
ester 2-oxo-phényléthylique d'acide 2-chloro-4-fluoro-5-(pipéridine-1-sulfonyl)benzoïque ;
ester 2-(2,5-diméthoxyphényl)-2-oxoéthylique d'acide 2-chloro-5-(pipéridine-1-sulfonyl)benzoïque ;
ester 2-(3-méthoxyphényl)-2-oxoéthylique d'acide 2-chloro-5-(pipéridine-1-sulfonyl)benzoïque ;
ester 2-(3-chlorophényl)-2-oxoéthylique d'acide 2-chloro-5-(pipéridine-1-sulfonyl)benzoïque ;
ester 2-oxo-2-phényléthylique d'acide 2-méthyl-5-(pipéridine-1-sulfonyl)benzoïque ;
ester 2-oxo-2-phényléthylique d'acide 2-fluoro-5-(pipéridine-1-sulfonyl)benzoïque ;
ester 2-oxo-2-pyridin-2-yléthylique d'acide 2-chloro-5-(pipéridine-1-sulfonyl)benzoïque ;
ester 1-méthyl-2-oxo-2-phényléthylique d'acide 2-chloro-5-(pipéridine-1-sulfonyl)benzoïque ;
ester 2-(4-fluorophényl)-2-oxoéthylique d'acide 2-chloro-5-(pipéridine-1-sulfonyl)benzoïque ;
ester 2-oxo-2-p-tolyléthylique d'acide 2-chloro-5-(pipéridine-1-sulfonyl)benzoïque ;
ester 2-oxobutylique d'acide 2-chloro-5-(pipéridine-1-sulfonyl)benzoïque ;
ester 2-naphthalén-2-yl-2-oxoéthylique d'acide 2-chloro-5-(pipéridine-1-sulfonyl)benzoïque ;
ester 2-benzo[b]thiophén-3-yl-2-oxoéthylique d'acide 2-chloro-5-(pipéridine-1-sulfonyl)benzoïque ;
ester 3,3-diméthyl-2-oxobutylique d'acide 2-chloro-5-(pipéridine-1-sulfonyl)benzoïque ;
ester 2-oxo-2-phényléthylique d'acide 2-chloro-5-(morpholine-4-sulfonyl)benzoïque ;
ester 2-oxo-2-phényléthylique d'acide 5-(butyléthylsulfamoyl)-2-chlorobenzoïque ;
ester 2-oxo-2-phényléthylique d'acide 2-chloro-5-diméthylsulfamoylbenzoïque ;
ester 2-oxo-2-phényléthylique d'acide 2-chloro-5-(octahydropyrido[1,2-a]pyrazine-2-sulfonyl)benzoïque ;
ester 2-oxo-2-phényléthylique d'acide 2-chloro-5-(4-méthylpipéridine-1-sulfonyl)benzoïque ;
ester 2-oxo-2-phényléthylique d'acide 2-chloro-5-(2,5-diméthylpyrrolidine-1-sulfonyl)benzoïque ;
ester 2-oxo-2-phényléthylique d'acide 3-(pipéridine-1-sulfonyl)benzoïque ;
N-2-oxo-2-phénéthyl-2,4-dichloro-5-[pipéridinosulfonyl]benzamide ;
2-chloro-4-fluoro-N-(2-oxo-2-phényléthyl)-5-(pipéridine-1-sulfonyl)benzamide ;
chlorhydrate de 2-oxo-2-phénéthyl-2,4-dichloro-5-[pipérazinosulfonyl]benzoate ;
chlorhydrate de 2-oxo-2-phénéthyl-2,4-dichloro-5-[4-méthyl-pipérazinosulfonyl]benzoate ;
2,4-dichloro-5-[pipéridinosulfonyl]benzyloxyacétophénone;
ester 1,1-diméthyl-2-oxo-2-phényléthylique d'acide 2-chloro-5-(pipéridine-1-sulfonyl)benzoïque ;
ester tert-butylique d'acide [2-chloro-5-(pipéridine-1-sulfonyl)benzylamino]acétique ;
ester tert-butylique d'acide {[2-chloro-5-(pipéridine-l-sulfonyl)-benzyl]-méthyl-amino}acétique ;
ester tert-butylique d'acide (2-chloro-5-(pipéridine-1-sulfonyl)benzyloxy)acétique ;
ester tert-butylique d'acide 2-[2-chloro-5-(pipéridine-1-sulfonyl)benzyloxy]propionique ;
ester tert-butylique d'acide 2-[2-chloro-5-(pipéridine-1-sulfonyl)benzyloxy]butyrique ;
2-(2-chloro-5-(pipéridine-1-sulfonyl)benzyloxy)-1-pyrrolidin-1-yléthanone ;
2-(2-chloro-5-(pipéridine-1-sulfonyl)benzyloxy)-N-méthyl-N-phénylacétamide ;
N-tert-butyl-2-(2-chloro-5-(pipéridine-1-sulfonyl)benzyloxy)acétamide ;
ester tert-butylique d'acide (1-(2-(2-chloro-5-pipéridine-1-sulfonyl)benzyloxy)acétyl)pyrrolidin-3-yl)carbamique ;
ester tert-butylique d'acide (1-(2-(2-chloro-5-(pipéridine-1-sulfonyl)benzyloxy)acétyl)pipéridin-4-yl)carbamique ;
2-[2-chloro-5-(pipéridine-1-sulfonyl)-benzyloxy]-N-pyridin-2-yl-acétamide ;
2-[2-chloro-5-pipéridine-1-sulfonyl)benzyloxy]-1-pipéridin-1-yl-éthanone ;
2-[2-chloro-5-(pipéridine-1-sulfonyl)benzyloxy]-N-méthyl-N-pyridin-2-yl-acétamide ;
2-(2,4-dichloro-5-(pipéridine-1-sulfonyl)benzyloxy)-N-méthoxy-N-méthylacétamide ;
2-(2-chloro-5-(pipéridine-1-sulfonyl)benzyloxy)-N-méthoxy-N-méthylacétamide ;
N-méthoxy-2-[2-méthoxy-5-(pipéridine-1-sulfonyl)benzyloxy)-N-méthyl-acétamide ;
N-méthoxy-2-[2-méthoxy-5-(pipéridine-1-sulfonyl)benzyloxy)-N-méthyl-acétamide ;
2-[2-bromo-5-(pipéridine-1-sulfonyl)benzyloxy)-N-méthoxy-N-méthyl-acétamide ;
N-méthoxy-N-méthyl-2-[5-(pipéridine-1-sulfonyl)-2-trifluorométhoxy-benzyloxy]acétamide ;
2-(1-(2-chloro-5-(pipéridine-1-sulfonyl)phényl)éthoxy)-N-méthoxy-N-méthyl-acétamide ;
2-[2-chloro-5-(thiomorpholine-4-sulfonyl)benzyloxy]-N-méthoxy-N-méthyl-acétamide ;
2-[2-chloro-5-(pipéridine-1-sulfonyl)benzyloxy]-1-(3-méthoxyphényl)éthanone ;
2-[2-chloro-5-(pipéridine-1-sulfonyl)benzyloxy]-1-cyclohexyléthanone ;
2-[2-chloro-5-(pipéridine-1-sulfonyl)benzyloxy]-1-(2,5-diméthoxyphényl)éthanone ;
2-[2,4-dichloro-5-(pipéridine-1-sulfonyl)benzyloxy]-1-(3-méthoxyphényl)éthanone ;
1-(3-chlorophényl)-2-[2-chloro-5-(pipéridine-1-sulfonyl)benzyloxy]éthanone ;
1-(3-chlorophényl)-2-[2,4-dichloro-5-(pipéridine-1-sulfonyl)benzyloxy]éthanone ;
2-[2,4-dichloro-5-(pipéridine-1-sulfonyl)benzyloxy]-1-naphtalén-2-yléthanone ;
2-[2-chloro-5-(pipéridine-1-sulfonyl)benzyloxy]-1-naphtalén-2-yléthanone;
4-chloro-N,N-diméthyl-5-(2-oxo-2-phényléthoxyméthyl)benzènesulfonamide ;
4-chloro-N,N-diéthyl-3-(2-oxo-2-phényléthoxyméthyl)benzènesulfonamide ;
4-chloro-N-éthyl-N-méthyl-3-(2-oxo-2-phényléthoxyméthyl)benzènesulfonamide ;
4-chloro-N-méthyl-3-(2-oxo-2-phényléthoxyméthyl)-N-propyl-benzènesulfonamide ;
2-[2-bromo-5(pipéridine-1-sulfonyl)benzyloxy]-1-phényl-éthanone ;
2-[2-chloro-5-(thiomorpholine-4-sulfonyl)benzyloxy]-1-phényl-éthanone;
2-(2-chloro-5-(pipéridine-1-sulfonyl)benzyloxyl)-1-(1H-pyrrolo(2,3-b)-pyridin-3-yl)-éthanone ;
2-[2-chloro-5-(4-méthylpipérazine-1-sulfonyl)benzyloxy)-1-pyridin-2-yl-éthanone ;
2-[2-chloro-5-(pipéridine-1-sulfonyl)benzyloxy]-1-pyridin-3-yléthanone ;
2-[2-chloro-5-(pipéridine-1-sulfonyl)benzyloxy]-1-pyridin-4-yléthanone ;
2-[2-chloro-5-(pipéridine-1-sulfonyl)benzyloxy]-1-pyridin-2-yléthanone ;
4-chloro-N,N-diméthyl-3-(2-oxo-2-pyridin-3-yléthoxyméthyl)benzènesulfonamide ;
2-[2-chloro-5-(pipéridine-1-sulfonyl)benzyloxy]-1-quinoléin-8-yléthanone ;
4-chloro-N,N-diméthyl-3-(2-oxo-2-pyridin-2-yléthoxyméthyl)benzènesulfonamide ;
4-chloro-N-éthyl-N-méthyl-3-(2-oxo-2-pyridin-2-yléthoxyméthyl)benzènesulfonamide ;
4-chloro-N,N-diéthyl-3-(2-oxo-2-pyridin-2-yléthoxyméthyl)benzènesulfonamide ;
4-chloro-N-méthyl-3-(2-oxo-2-pyridin-2-yléthoxyméthyl)-N-propyl-benzènesulfonamide ;
4-chloro-N-éthyl-N-méthyl-3-(2-oxo-2-pyridin-3-yléthoxyméthyl)benzènesulfonamide ;
4-chloro-N,N-diéthyl-3-(2-oxo-2-pyridin-3-yléthoxyméthyl)benzènesulfonamide ;
2-[2-chloro-5-(pipéridine-1-sulfonyl)benzyloxy]-1-(4-méthyl-pyridin-2-yl)éthanone ;
2-[2-chloro-5-(pipéridine-1-sulfonyl)benzyloxy]-1-(6-méthyl-pyridin-2-yl)éthanone ;
2-[2-chloro-5-(pipéridine-1-sulfonyl)benzyloxy]-1-(5-méthyl-pyridin-2-yl)éthanone ;
4-chloro-N-éthyl-N-méthyl-3-[2-(4-méthyl-pyridin-2-yl)-2-oxo-éthoxyméthyl]benzènesulfonamide ;
4-chloro-N-éthyl-N-méthyl-3-[2-(6-méthyl-pyridin-2-yl)-2-oxo-éthoxyméthyl]benzènesulfonamide ;
4-chloro-N-éthyl-N-méthyl-3-[2-(5-méthyl-pyridin-2-yl)-2-oxo-éthoxyméthyl]benzènesulfonamide ;
2-[2-chloro-5-(pipéridine-1-sulfonyl)benzyloxy]-1-(3-méthyl-pyridin-2-yl)éthanone ;
4-chloro-N-éthyl-N-méthyl-3-[2-(3-méthyl-pyridin-2-yl)-2-oxo-éthoxyméthyl]benzènesulfonamide ;
1-(2-bromo-5-diméthylamino-pyridin-4-yl)-2-[2-chloro-5-(pipéridine-1-sulfonyl)benzyloxy]éthanone ;
2-[2-méthoxy-5-(pipéridine-1-sulfonyl)benzyloxy]-1-pyridin-2-yl-éthanone ;
2-[2-méthyl-5-(pipéridine-1-sulfonyl)benzyloxy]-1-pyridin-2-yl-éthanone ;
2-5-(pipéridine-1-sulfonyl)-2-trifluorométhoxy-benzyloxy]-1-pyridin-2-yl-éthanone ;
2-(1-(2-chloro-5-(pipéridine-1-sulfonyl)phényl)éthoxy)-1-pyridin-2-yl-éthanone ;
2-(1-(2-chloro-5-(pipéridine-1-sulfonyl)phényl)éthoxy)-1-pyridin-3-yl-éthanone ;
2-(2-chloro-5-(pipéridine-1-sulfonyl)benzyloxy)-1-pyrazin-2-yl-éthanone ;
2-(2-chloro-5-(pipéridine-1-sulfonyl)benzyloxy)-1-(1-méthyl-1H-imidazol-2-yl)éthanone ;
2-(2-chloro-5-(pipéridine-1-sulfonyl)benzyloxy)-1-(1-pyridin-2-yl-1H-imidazol-2-yl)éthanone ;
2-(2-chloro-5-(pipéridine-1-sulfonyl)benzyloxy)-1-(1-méthyl-1H-indol-5-yl)éthanone ;
2-[2-chloro-5-(pipéridine-1-sulfonyl)benzyloxy]-1-pyridin-2-yl-propan-1-one ;
2-[2,4-dichloro-5-(pipéridine-1-sulfonyl)benzyloxy]-1-(3-méthoxyméthylphényl)éthanone ;
2-[2,4-dichloro-5-(pipéridine-1-sulfonyl)benzyloxy]-1-(4-méthoxyméthylphényl)éthanone ;
2-[2,4-dichloro-5-(pipéridine-1-sulfonyl)benzyloxy]-1-(3-diméthylaminométhylphényl)éthanone ;
2-[2-chloro-5-(pipéridine-1-sulfonyl)benzyloxy]-1-(3-diméthylaminométhylphényl)éthanone ;
2-(2-oxo-2-phényl-éthoxyméthyl)-4-(pipéridine-1-sulfonyl)benzonitrile ;
4-[2-chloro-5-(pipéridine-1-sulfonyl)phényl]-4-oxobutyraldéhyde ;
4-[2-méthyl-5-(pipéridine-1-sulfonyl)phényl]-4-oxobutyraldéhyde ;
4-[2-chloro-5-(pipéridine-1-sulfonyl)phényl]butyraldéhyde ;
1-[2-chloro-5-(pipéridine-1-sulfonyl)phényl]-4-naphtalén-2-ylbutane-1,4-dione ;
1-[2-chloro-5-(pipéridine-1-sulfonyl)phényl]-4-naphtalén-1-ylbutane-1,4-dione ;
1-[2-chloro-5-(pipéridine-1-sulfonyl)phényl]-4-phénylbutane-1,4-dione ;
1-[2-chloro-5-(pipéridine-1-sulfonyl)phényl]-4-pyridin-3-ylbutane-1,4-dione ;
1-[2-chloro-5-(pipéridine-1-sulfonyl)phényl]-4-(3-diméthylaminométhylphényl)butane-1,4-dione ;
4-[2-chloro-5-(pipéridine-1-sulfonyl)phényl]-1-phényl-butan-1-one ;
4-[2-chloro-5-(pipéridine-1-sulfonyl)phényl]-1-pyridin-3-yl-butan-1-one ;
1-[2-chloro-5-(pipéridine-1-sulfonyl)phényl]-4-pyridin-2-ylbutane-1,4-dione ;
1-[2-méthyl-5-(pipéridine-1-sulfonyl)phényl]-4-pyridin-2-ylbutane-1,4-dione ;
4-[2-chloro-5-(pipéridine-1-sulfonyl)phényl]-1-pyridin-2-yl-butan-1-one ;
1-(4-chloro-3-(5-thiophén-2-yl-(1,2,4)oxadiazol-3-yl-méthoxyméthyl)benzènesulfonyl)pipéridine ;
(3-(3-benzènesulfinyl-propényl)-4-chloro-benzènesulfonyl)pipéridine ;
2-[2-chloro-5-(pipéridine-1-sulfonyl)benzyloxy]-1-pyrrolo[2,3-b]pyridin-1-yléthanone ; et
2-[2-chloro-5-(pipéridine-1-sulfonyl)benzylamino]-1-pipéridin-1-yl-éthanone.

5. Composé selon l'une quelconque des revendications 1 à 4 destiné à être utilisé comme médicament.

6. Utilisation d'un composé selon l'une quelconque des revendications 1 à 4 dans la fabrication d'un médicament destiné au traitement des troubles neurologiques et psychiatriques associés à des anomalies du métabolisme du glutamate.

7. Procédé de préparation d'un composé de formule IV 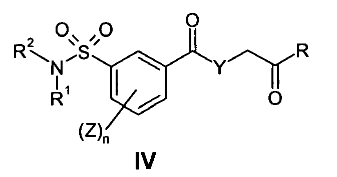 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 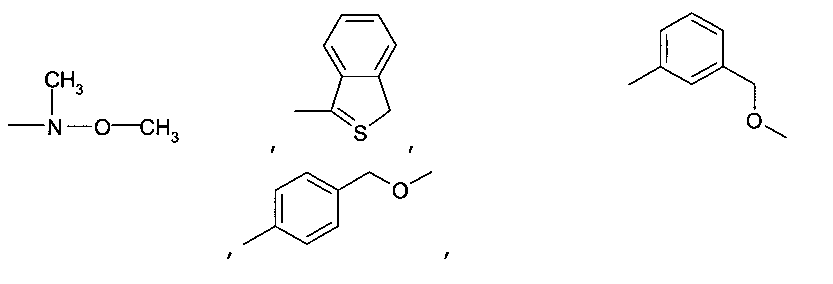 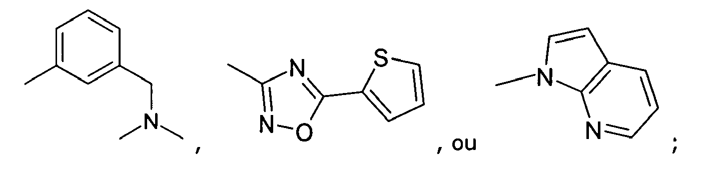
R¹ et R² sont chacun, indépendamment, un groupe alkyle en C₁₋₆, ou R¹, R² et le N auquel ils sont liés, par combinaison, sont 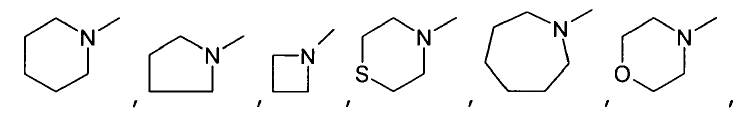 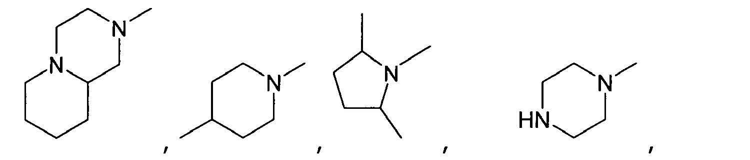 ou 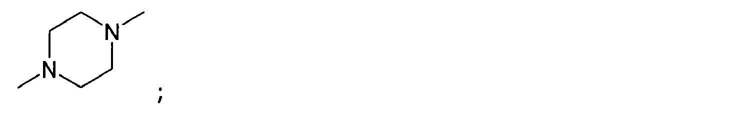
Y est CH₂, NH, NCH₃ ou O ;
Z est, en chaque position, indépendamment, CI, F, méthoxy ou méthyle ; et
n est égal à 0, 1, 2, 3 ou 4 ;
ledit procédé comprenant les étapes suivantes :
a) réaction d'un composé de formule 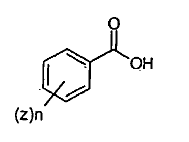 avec de l'acide chlorosulfonique pour obtenir un composé de formule II 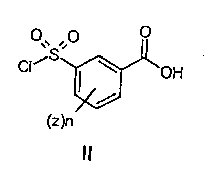
b) réaction du composé de formule II avec une amine pour obtenir un composé de formule III 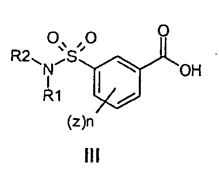 et
c) alkylation du composé de formule III pour obtenir un composé de formule IV.

8. Procédé de préparation d'un composé de formule VI 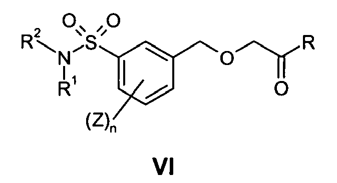 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 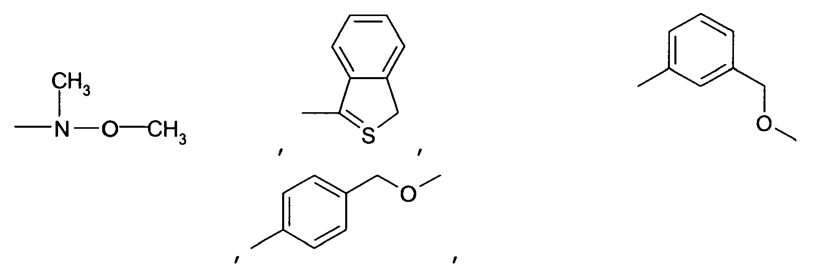 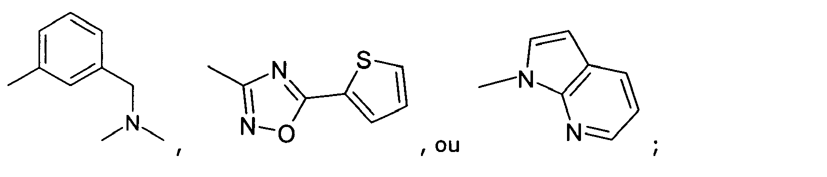
R¹ et R² sont chacun, indépendamment, un groupe alkyle en C₁₋₆, ou R¹, R² et le N auquel ils sont liés, par combinaison, sont 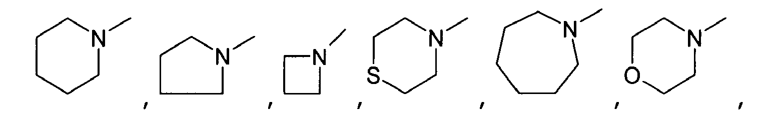 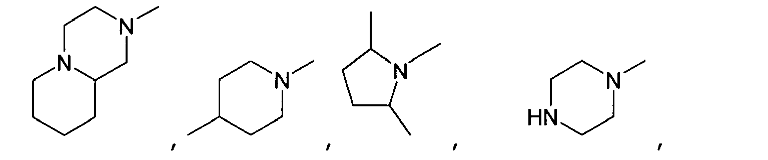 ou 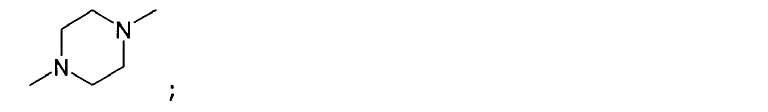
Z est, en chaque position, indépendamment, CI, F, méthoxy ou méthyle ; et
n est égal à 0, 1, 2, 3 ou 4 ;
ledit procédé comprenant les étapes suivantes :
a) réduction d'un composé de formule III tel que défini ci-dessus en un composé de formule V 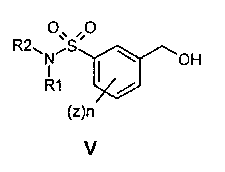 ; et
b) alkylation du composé de formule V pour obtenir un composé de formule VI.

9. Procédé de préparation d'un composé de formule IX 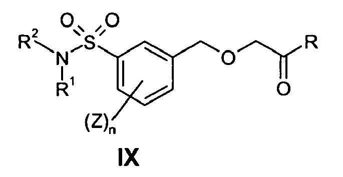 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 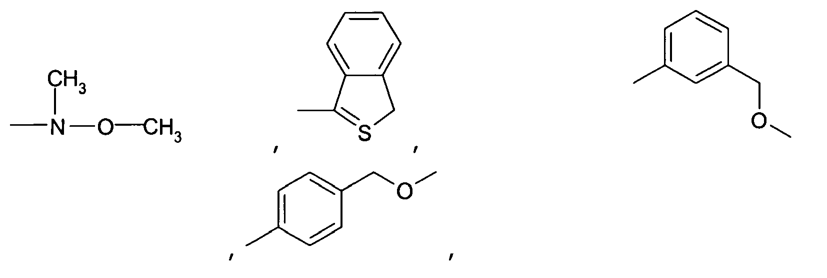 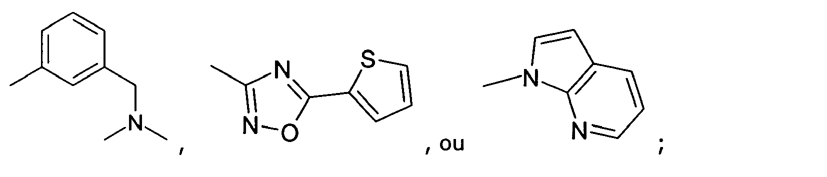
R¹ et R² sont chacun, indépendamment, un groupe alkyle en C₁₋₆, ou R¹, R² et le N auquel ils sont liés, par combinaison, sont 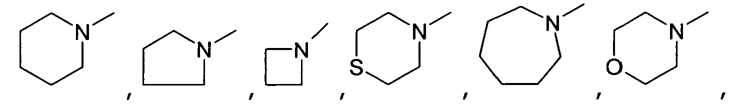 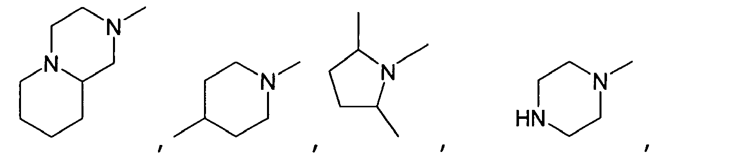 ou 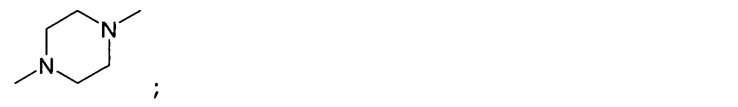
Z est, en chaque position, indépendamment, CI, F, méthoxy ou méthyle ; et
n est égal à 0, 1, 2, 3 ou 4 ;
ledit procédé comprenant les étapes suivantes :
a) alkylation d'un composé de formule V tel que défini ci-dessus pour obtenir un composé de formule VII 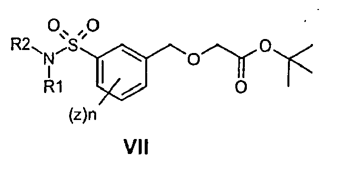
b) déprotection du composé de formule VII par l'acide formique pour obtenir un composé de formule VIII 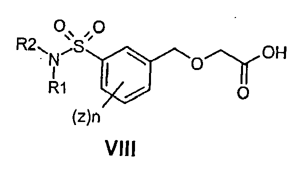 et
c) réaction du composé de formule VIII pour obtenir un composé de formule IX.

10. Procédé de préparation d'un composé de formule XI 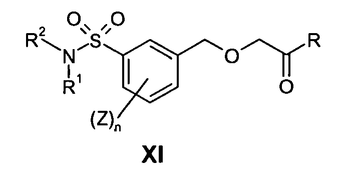 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 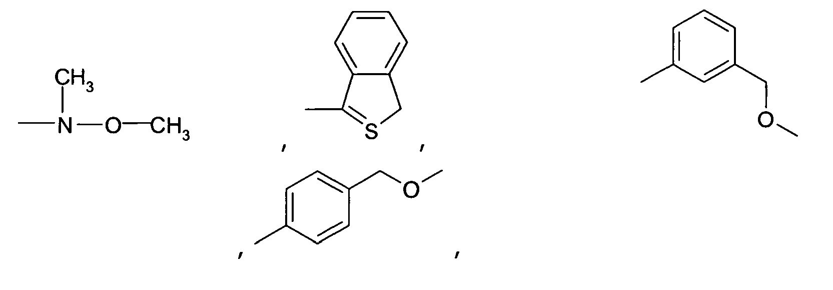 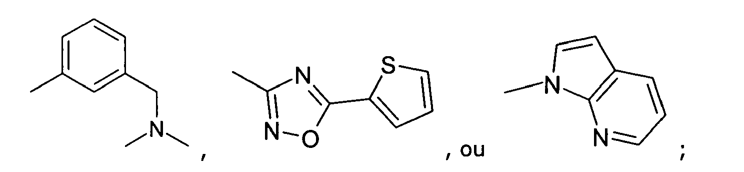
R¹ et R² sont chacun, indépendamment, un groupe alkyle en C₁₋₆, ou R¹, R² et le N auquel ils sont liés, par combinaison, sont 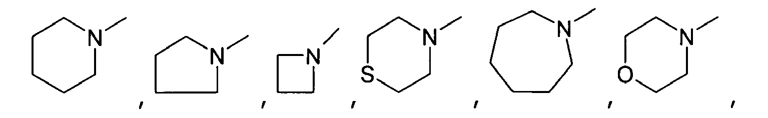 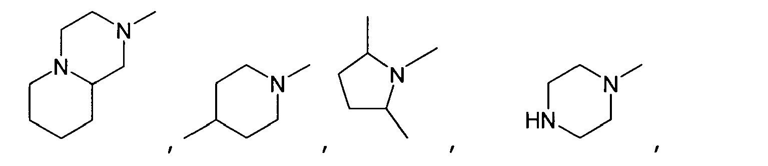 ou 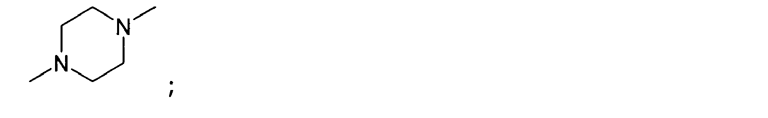
Z est, en chaque position, indépendamment, Cl, F, méthoxy ou méthyle ; et
n est égal à 0, 1, 2, 3 ou 4 ;
ledit procédé comprenant les étapes suivantes :
a) alkylation d'un composé de formule V tel que défini ci-dessus par du 2-chloro-N-méthoxy-N-méthylacétamide pour obtenir un composé de formule X 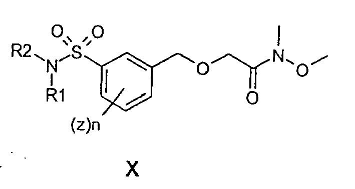 et
b) réaction du composé de formule X avec un réactif organométallique pour obtenir un composé de formule XI.

11. Procédé de la revendication 10, dans lequel le réactif organométallique est choisi parmi un réactif Grignard et un réactif organolithium.

12. Procédé de préparation d'un composé de formule XV 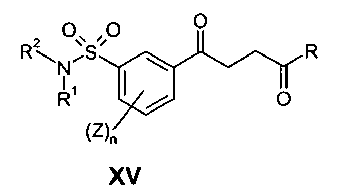 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 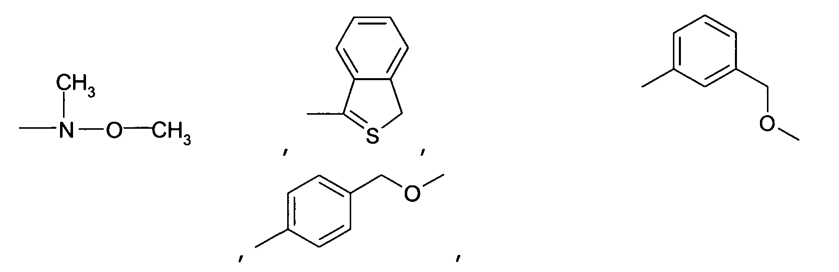 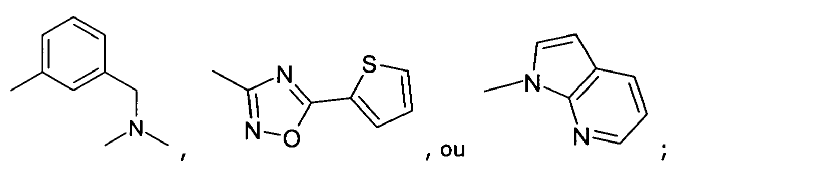
R¹ et R² sont chacun, indépendamment, un groupe alkyle en C₁₋₆, ou R¹, R² et le N auquel ils sont liés, par combinaison, sont 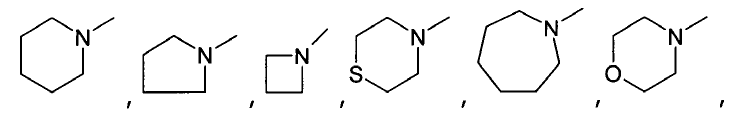 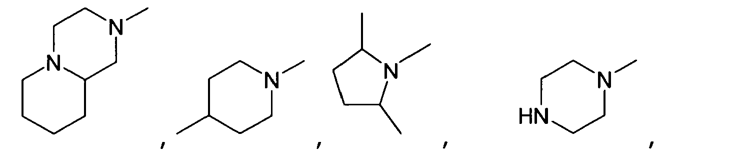 ou 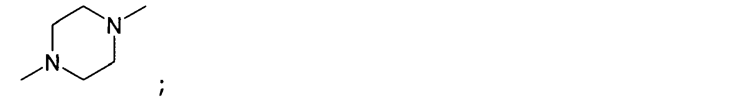
Z est, en chaque position, indépendamment, Cl, F, méthoxy ou méthyle ; et
n est égal à 0, 1, 2, 3 ou 4 ;
ledit procédé comprenant les étapes suivantes :
a) conversion d'un composé de formule III tel que défini ci-dessus en un chlorure d'acide correspondant, puis réaction avec un réactif Grignard dérivé du 1-bromo-3,3-diméthoxypropane pour obtenir un composé de formule XII 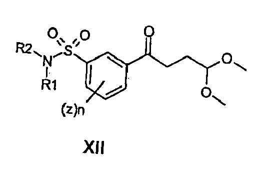
b) conversion du composé de formule XII en un composé de formule XIII 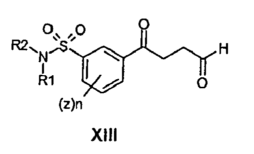
c) réaction du composé de formule XIII avec un réactif organométallique pour obtenir un composé de formule XIV 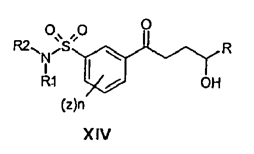 et
d) oxydation du composé de formule XIV pour obtenir un composé de formule XV.

13. Procédé de la revendication 12, dans lequel l'étape de conversion b) a lieu dans des conditions acides.

14. Procédé de la revendication 12, dans lequel le réactif organométallique de l'étape c) est choisi parmi un réactif Grignard et un réactif organolithium.

15. Procédé de préparation d'un composé de formule XX 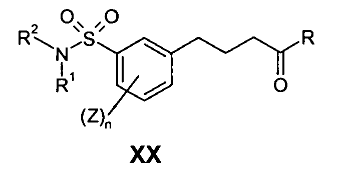 dans laquelle
R est un groupe phényle, diméthoxyphényle, méthoxyphényle, chlorophényle, pyridyle, fluorophényle, éthyle, naphtyle, tert-butyle, pyrrolidinyle éventuellement substitué, N-méthylphénylamino, pipéridinyle éventuellement substitué, cyclohexyle, quinolinyle, 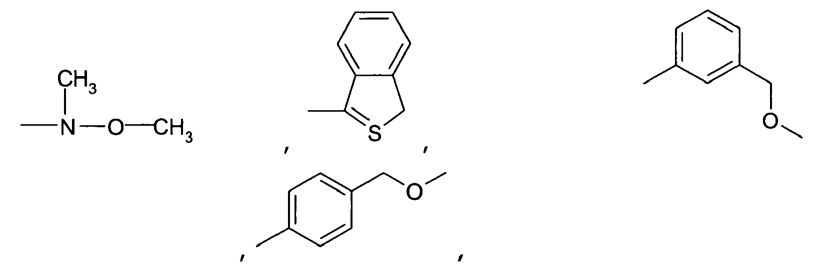 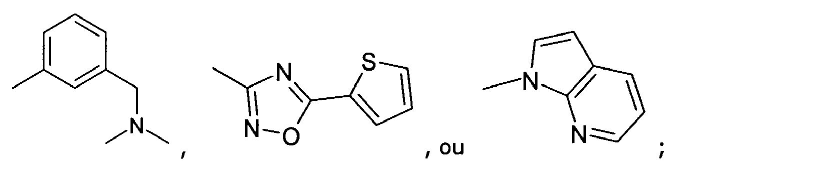
R¹ et R² sont chacun, indépendamment, un groupe alkyle en C₁₋₆, ou R¹, R² et le N auquel ils sont liés, par combinaison, sont 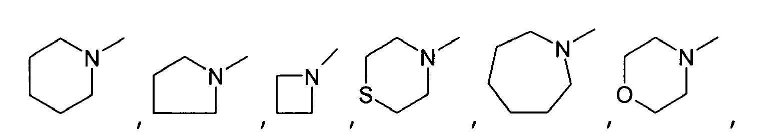 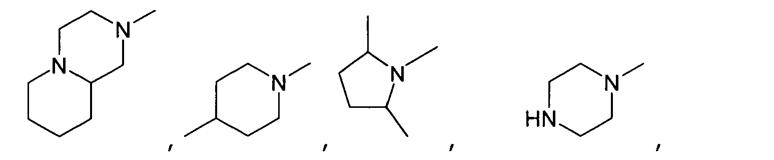 ou 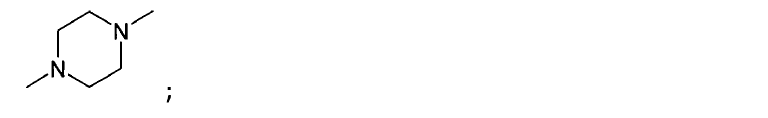
Z est, en chaque position, indépendamment, Cl, F, méthoxy ou méthyle ; et n est égal à 0, 1, 2, 3 ou 4 ;
ledit procédé comprenant les étapes suivantes :
a) réduction d'un composé de formule XII tel que défini ci-dessus en un composé de formule XVI 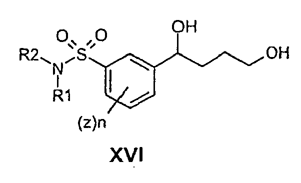
b) réduction du composé de formule XVI pour obtenir un composé de formule XVII 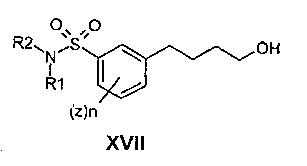
c) oxydation du composé de formule XVII en un composé de formule XVIII 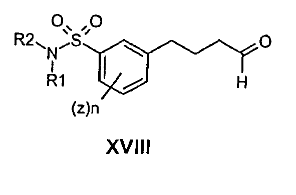
d) alkylation du composé de formule XVIII pour obtenir un composé de formule XIX 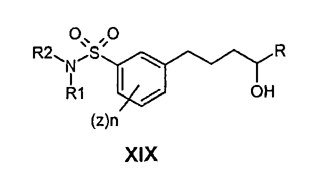 et
e) oxydation du composé de formule XIX pour obtenir un composé de formule XX.
